# EUROPEAN PATENT APPLICATION

(11) **EP 2 420 502 A1**
(43) Date of publication of application: **22.02.2012**
(21) Application number: 10764459.3
(22) Date of filing: 13.04.2010
(51) Int. Cl.: C07D 471/14, A61K 31/437, A61K 31/439, A61K 31/4545, A61K 31/497, A61K 31/541, A61K 31/55, A61P 1/04, A61P 1/16, A61P 3/10, A61P 7/00, A61P 9/00, A61P 9/10, A61P 11/00

(54) **FUSED PYRROLOPYRIDINE DERIVATIVE**

(30) Priority: 14.04.2009 JP 2009097974
(71) Applicant: Astellas Pharma Inc., Tokyo 103-8411 (JP)
(72) Inventor: SHIRAKAMI, Shohei, Tokyo 103-8411 (JP); NAKAJIMA, Yutaka, Tokyo 103-8411 (JP); MAEDA, Jun, Tokyo 103-8411 (JP); TOMINAGA, Hiroaki, Tokyo 103-8411 (JP); YAMAGISHI, Hiroaki, Tokyo 103-8411 (JP); HONDO, Takeshi, Tokyo 103-8411 (JP); INAMI, Masamichi, Tokyo 103-8411 (JP); MORIO, Hiroki, Tokyo 103-8411 (JP); INOUE, Takayuki, Tokyo 103-8411 (JP); MIZUTANI, Tsuyoshi, Tokyo 103-8411 (JP); ISHIOKA, Hiroki, Tokyo 103-8411 (JP)
(74) Representative: Gille Hrabal
(86) International application number: PCT/JP2010/056619
(87) International publication number: WO 2010/119875

(57) **Abstract**

[Problem]

The present invention provides a condensed pyrrolopyridine derivative which is useful as an active ingredient for a pharmaceutical composition, in particular, a pharmaceutical composition for preventing or treating diseases caused by undesirable cytokine signal transduction or diseases caused by abnormal cytokine signal transduction.

[Means for Solution]

The present inventors have extensively studied a compound having a JAK inhibitory action, and as a result, they have found that a condensed pyrrolopyridine derivative which is the compound of the present invention has an excellent JAK inhibitory action, and is therefore useful as an agent for preventing or treating diseases caused by undesirable cytokine signal transduction or diseases caused by abnormal cytokine signal transduction, thereby completing the present invention.

## Description

### Technical Field

The present invention relates to a fused pyrrolopyridine derivative which is useful as an active ingredient of a pharmaceutical composition, in particular, a pharmaceutical composition for preventing or treating diseases caused by undesirable cytokine signal transduction or diseases caused by abnormal cytokine signal transduction.

### Background Art

Janus kinase 3 (hereafter referred to as JAK) is a member of Janus family of protein kinases. Although kinases in this family, other than JAK3, are expressed in a wide range of tissues, JAK3 is expressed locally in hematopoietic cells. This does not contradict with the fact that JAK3 plays an important role in signal transduction via various receptors of interleukin (hereafter referred to as IL)-2, IL-4, IL-7, IL-9, IL-15, IL-21, and the like, by a noncovalent binding with the common Υ chain (Non-Patent Documents 1 and 2).
XSCID (X-linked Severe Combined Immuno Deficiency) patient groups have been identified with a reduced level of JAK3 protein or with a genetic defect in the common γ chain, suggesting that immunosupression is caused by blocking of the signal pathway through JAK3 (Non-Patent Documents 3 and 4). Animal experiments have suggested that JAK3 not only plays an important role in maturation of B- and T-lymphocytes but also in maintaining the function of T-cells. Hence, it is expected that the diseases in which abnormal proliferation of T-cells is participated, such as rejection upon organ transplantation and autoimmune diseases, can be treated by controlling an immune response through this mechanism.
With regard to JAK1, it has been clarifyed that JAK1 is participted in signal transduction via various receptors of interferon (hereinafter referred to as IFN)α, IFNβ, IFNγ, IL-2, IL-4, IL-6, IL-7, IL-15, and the like, based on analyses of JAK1 KO mice and JAK1-deficient cells (Non-Patent Document 5). It is considered that it is deeply participated in signal transduction via the IL-6 receptor by activation of Stat3. Therefore, by controlling the inflammatory response through this mechanism, it is expected that diseases in which activation of macrophages or lymphocytes are involved, such as autoimmune diseases and acute and chronic rejection upon organ transplantation, will be treated.
With regard to JAK2, it has been clarified that JAK2 is participated in signal transduction via various receptors of erythropoietin (hereinafter referred to as EPO)α, thrombopoietin (hereinafter referred to as TPO), IFNγ, IL-3, GM-CSF, and the like, based on the analyses of JAK2 KO mice and JAK2-deficient cells (Non-Patent Documents 6, 7, and 8). It is considered that these are participated in differentiation of progenitor cells such as erythrocytes, platelets, and the like in the bone marrow, through Stat3. Further, there is a case where phenylalanine of the 617^{th} base of JAK2 is replaced by valine, and participation in myeloproliferative disorders has been suggested (Non-Patent Document 6). Thus, it is expected that by controlling the differentiation of myeloid progenitor cells through such a mechanism, myeloproliferative disorders will be treated.

The Applicant has found that a fused pyridine compound represented by the formula (A) has a JAK3 inhibitory action, and is therefore useful for diseases such as organ transplantation, autoimmune diseases, asthma, atopic dermatitis, Alzheimer's disease, atherosclerosis, tumour, myeloma, leukemia, and the like, and filed a Patent Application (Patent Document 1).

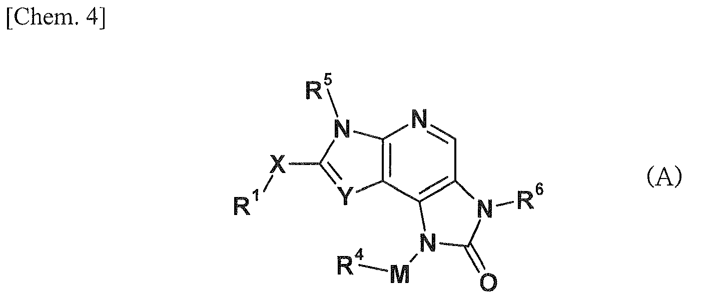

(for the symbols, refer to the patent publication).
This compound is **chracterized in that** an imidazolidin-2-one ring is fused with a pyrrolopyridine or imidazopyridine ring.

Furthermore, the Applicant has reported that a fused pyridine compound represented by the formula (B) has a JAK3 inhibitory action, and is therefore useful for diseases such as organ transplantation, autoimmune diseases, asthma, atopic dermatitis, tumour, myeloma, leukemia, allergic diseases, and the like (Patent Document 2).

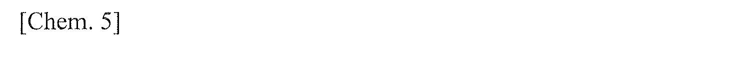

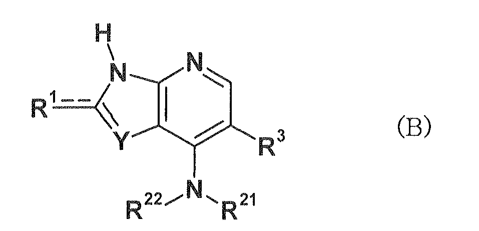

(wherein R²¹ represents -H; or may be combined with R³ through a certain functional group to form a divalent group with a group selected from the (IA), (IB), (IC), and (ID) shown below: 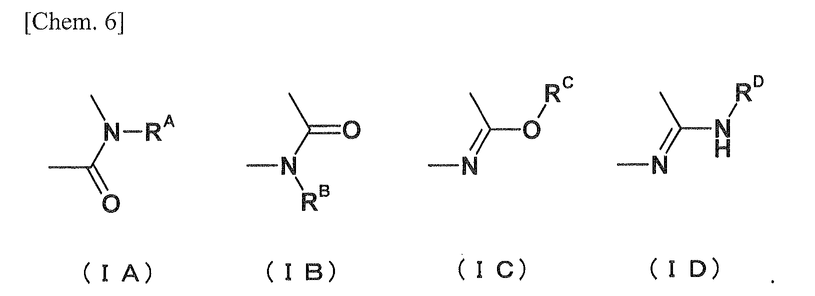 for the other symbols, refer to the patent publication).
This compound is **characterized in that** R²¹ is combined with R³ through a certain functional group to form a specific hetero ring.

Furthermore, in Pamphlet of International Publication WO 2007/022268, it is described that a compound having a dihydrodipyrrolopyridine skeleton with a carboxy group or a sulfonyl group as an essential structure, and the like are effective as various types of kinase inhibitors (Patent Document 3).

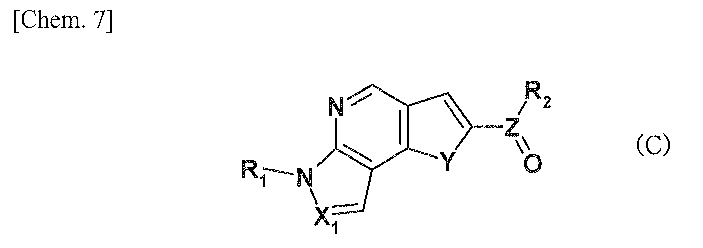

(wherein X₁ represents CH or N, Y represents S or NR₅, and R₅ represents H or C₁₋₆ alkyl; for the other symbols, refer to the patent publication).

Moreover, in Pamphlet of International Publication WO 2009/152133, which was published after the priority date of the present application, it is described that a group of compounds having dihydrodipyrrolopyridine skeletons are effective as various types of kinase inhibitors, but a specific compound having the skeleton is not disclosed in the specfication (Patent Document 4).

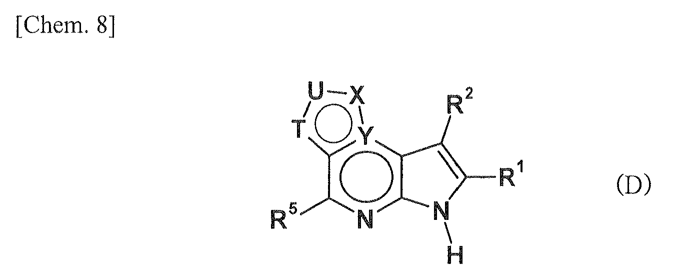

(wherein T represents CR⁶, U represents CR⁴, X represents NR³, and Y represents C; for the other symbols, refer to the patent publication).

In any of these Documents, there is no specific disclosure of the compound of the present invention.

### Prior Art

Patent Document 1: Pamphlet of International Publication WO 2007/007919
Patent Document 2: Pamphlet of International Publication WO 2008/084861
Patent Document 3: Pamphlet of International Publication WO 2007/022268
Patent Document 4: Pamphlet of International Publication WO 2009/152133

### Non-Patent Document

Non-Patent Document 1: J. J. O'Shea, et al., Cell, Vol. 109 (suppl.), S121, 2002
Non-Patent Document 2: K. Ozaki, et al., Science, Vol. 298, p. 1630, 2002
Non-Patent Document 3: P. Macchi, et al., Nature, Vol. 377, p. 65, 1995
Non-Patent Document 4: S. M. Russell, et al., Science, Vol. 270, p. 797, 1995
Non-Patent Document 5: Peter J. Murray, J Immunol., Vol. 178, pp. 2623-2629, 2007
Non-Patent Document 6: Staerk J, et al., Pathol Biol., Vol. 55, pp. 88-91, 2007
Non-Patent Document 7: Yoo JH, et al., Cancer Genet Cytogenet., Vol. 189, pp. 43-47,2009
Non-Patent Document 8: Vainchenker W, et al., Semin Cell Dev Biol., Vol. 19, pp. 385-393,2008

### Disclosure of Invention

### Technical Problem

### Problems to Be Solved by the Invention

The present invention provides a compound which is useful as an active ingredient of a pharmaceutical composition, in particular, a pharmaceutical composition for preventing or treating diseases caused by undesirable cytokine signal transduction or diseases caused by abnormal cytokine signal transduction.

### Means for Solving the Problems

The present inventors have extensively studied a compound having a JAK inhibitory action, and as a result, they have found that a fused pyrrolopyridine derivative which is the compound of the present invention has an excellent JAK inhibitory action, and is therefore useful as an agent for preventing or treating diseases caused by undesirable cytokine signal transduction or diseases caused by abnormal cytokine signal transduction, thereby completing the present invention.

The present invention relates to a compound of the formula (I) or a salt thereof: 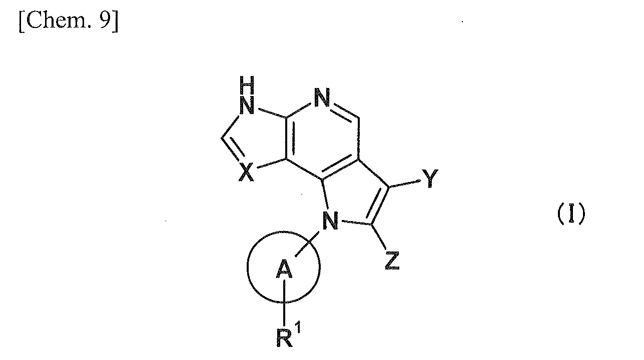 (wherein
A represents cycloalkyl which may be substituted, cycloalkenyl which may be substituted, or a nitrogen-containing hetero ring group which may be substituted,
X represents CR^{X} or N,
R^{X} represents H, OR^{XY1}, NR^{XY2}RX^{XY3}, SR^{XY4}, halogen, cyano, or a lower alkyl, aryl, or hetero ring group,
Y represents H, OR^{XY1}, NR^{XY2}R^{XY3}, SR^{XY4}, halogen, cyano, lower alkyl which may be substituted, aryl which may be substituted, or a hetero ring group which may be substituted,
Z represents H or lower alkyl,
R^{XY1}, R^{XY2}, R^{XY3}, and R^{XY4} are the same as or different from each other and represent H or lower alkyl,
R¹ represents H, OH, -(CR¹¹R¹²)ₘ-R¹³, -SO₂-R¹⁴, or a hetero ring group which may be substituted,
R¹¹ and R¹² are the same as or different from each other and represent H, halogen, OH, lower alkyl which may be substituted, aryl which may be substituted, or a hetero ring group which may be substituted,
R¹¹ and R¹² are combined with each other to form oxo (=O), or
R¹¹ and R¹² may be combined with a carbon atom to which they are bonded to form cycloalkyl which may be substituted,
R¹³ represents H, halogen, cyano, -NR^{N1}R^{N2}, aryl which may be substituted, cycloalkyl which may be substituted, or a hetero ring group which may be substituted,
R^{N1} and R^{N2} are the same as or different from each other and represent H, lower alkyl which may be substituted, or aryl which may be substituted,
R¹⁴ represents NR^{N3}R^{N4}, or lower alkyl which may be substituted, or a hetero ring group which may be substituted,
R^{N3} and R^{N4} are the same as or different from each other and represent H, lower alkyl which may be substituted, or aryl which may be substituted, and
m represents 1, 2, 3, or 4).

Further, unless specifically described otherwise, in the case where the symbols in any of the formulae in the present specification are also used in other formulae, the same symbols denote the same meanings. In addition, when m of _(CR¹¹R¹²)ₘ-R¹³ in R¹ is 2, 3, or 4, CR¹¹R¹²'s may be the same as or different from each other, and for example, in the case of m=2, they may be -C(=O)-CH₂-R¹³.

Furthermore, the present invention relates to a pharmaceutical composition, including the compound of the formula (I) or a salt thereof, and an excipient.
Moreover, the present invention relates to a pharmaceutical composition for preventing or treating diseases caused by undesirable cytokine signal transduction or diseases caused by abnormal cytokine signal transduction, including the compound of the formula (I) or a salt thereof, that is, an agent for preventing or treating diseases caused by undesirable cytokine signal transduction or diseases caused by abnormal cytokine signal transduction, including the compound of the formula (I) or a salt thereof.
Furthermore, the present invention relates to use of the compound of the formula (I) or a salt thereof for preparation of a pharmaceutical composition for preventing or treating diseases caused by undesirable cytokine signal transduction or diseases caused by abnormal cytokine signal transduction, the compound of the formula (I) or a salt thereof for preventing or treating diseases caused by undesirable cytokine signal transduction or diseases caused by abnormal cytokine signal transduction, and a method for preventing or treating diseases caused by undesirable cytokine signal transduction or diseases caused by abnormal cytokine signal transduction, including administering to a patient an effective amount of the compound of the formula (I) or a salt thereof.

### Effects of the Invention

The compound of the formula (I) or a salt thereof has a JAK inhibitory action, and therefore can be used as an agent for preventing or treating diseases caused by undesirable cytokine signal transduction or diseases caused by abnormal cytokine signal transduction.

### Best Mode for Carrying Out the Invention

The present invention will be explained in more detail herein below. Further, "the compound of the formula (I) or a salt thereof" may be denoted as "the compound (I) of the present invention" or "the compound (I)" below in some cases.

In the present specification, the "lower alkyl" is straight or branched alkyl having 1 to 6 carbon atoms (hereinafter simply referred to as C₁₋₆), for example, methyl, ethyl, n-propyl, isopropyl, n-butyl, isobutyl, sec-butyl, tert-butyl, n-pentyl, n-hexyl, and the like. In another embodiment, it is C₁₋₄ alkyl, and in a further embodiment, C₁₋₃ alkyl.

The "lower alkylene" is straight or branched C₁₋₆ alkylene, for example, methylene, ethylene, trimethylene, tetramethylene, pentamethylene, hexamethylene, propylene, methylmethylene, ethylethylene, 1,2-dimethylethylene, 1,1,2,2-tetramethylethylene, or the like. In another embodiment, it is C₁₋₄ alkylene, and in a further other embodiment, C₁₋₃ alkylene.

The "cycloalkyl" is a C₃₋₁₀ saturated hydrocarbon ring group, which may have a bridge. It is, for example, cyclopropyl, cyclobutyl, cyclopentyl, cyclohexyl, cycloheptyl, cyclooctyl, adamantyl, or the like, in another embodiment, C₃₋₈ cycloalkyl, and in a further embodiment, C₃₋₆ cycloalkyl.

The "cycloalkenyl" is a C₄₋₁₅ hydrocarbon ring group having at least one double bond in the ring (provided that an aromatic hydrocarbon ring group is excluded), which may have a bridge, and includes a ring group condensed with a benzene ring at a double bond site. It is, for example, cyclopentenyl, cyclopentadienyl, cyclohexenyl, cyclohexadienyl, 1-tetrahydronaphthyl, 1-indenyl, 9-fluorenyl, or the like. In another embodiment, it is C₅₋₁₀ cycloalkenyl, in a further embodiment, C₅₋₈ cycloalkenyl, and in a further embodiment, C₅₋₇ cycloalkenyl.

The "aryl" is a C₆₋₁₄ monocyclic to tricyclic aromatic hydrocarbon ring group, and includes a ring group condensed with C₅₋₈ cycloalkene at a double bond site. It is, for example, phenyl, naphthyl, 5-tetrahydronaphthyl, 4-indenyl, 1-fluorenyl, or the like.

The "hetero ring" group means a ring group containing i) a monocyclic 3- to 8-membered, and in another embodiment, 5- to 7-membered hetero ring, containing 1 to 4 hetero atoms selected from oxygen, sulfur, and nitrogen, and ii) a bicyclic to tricyclic hetero ring (in which the bicyclic to tricyclic hetero ring includes a spiro ring) containing 1 to 5 hetero atoms selected from oxygen, sulfur, and nitrogen, formed by condensation of the monocyclic hetero ring with one or two rings is selected from the group consisting of a monocyclic hetero ring, a benzene ring, C₅₋₈ cycloalkane, and C₅₋₈ cycloalkene. The ring atom, sulfur or nitrogen, may be oxidized to form an oxide or a dioxide.

In the present specification, other embodiments of the "hetero ring" include the following embodiments:
(1) Monocyclic Saturated Hetero Ring Groups
   (a) those containing 1 to 4 nitrogen atoms, for example, azepanyl, diazepanyl, aziridinyl, azetidinyl, pyrrolidinyl, imidazolidinyl, piperidyl, piperazolidinyl, piperazinyl, azocanyl, hexamethyleneimino, homopiperazinyl, and the like;
   (b) those containing 1 to 3 nitrogen atoms and 1 to 2 sulfur atoms and/or 1 to 2 oxygen atoms, for example, thiomorpholinyl, thiazolidinyl, isothiazolidinyl, oxazolidinyl, morpholinyl, and the like;
   (c) those containing 1 to 2 sulfur atoms, for example, tetrahydrothiopyranyl and the like;
   (d) those containing 1 to 2 sulfur atoms and 1 to 2 oxygen atoms, for example, oxathiolanyl and the like; and
   (e) those containing 1 to 2 oxygen atoms, for example, oxiranyl, oxetanyl, dioxolanyl, tetrahydrofuranyl, tetrahydropyranyl, 1,4-dioxanyl, and the like;

(2) Monocyclic Unsaturated Hetero Ring Groups
   (a) those containing 1 to 4 nitrogen atoms, for example, pyrrolyl, 2-pyrrolinyl, imidazolyl, 2-imidazolinyl, pyrazolyl, 2-pyrazolinyl, pyridyl, dihydropyridyl, tetrahydropyridinyl, pyrimidinyl, pyrazineyl, pyridazinyl, triazolyl, tetrazolyl, triazinyl, dihydrotriazinyl, azepinyl, and the like;
   (b) those containing 1 to 3 nitrogen atoms and 1 to 2 sulfur atoms and/or 1 to 2 oxygen atoms, for example, thiazolyl, isothiazolyl, thiadiazolyl, dihydrothiazinyl, oxazolyl, isoxazolyl, oxadiazolyl, oxazinyl, and the like;
   (c) those containing 1 to 2 sulfur atoms, for example, thienyl, thiepinyl, dihydrodithiopyranyl, dihydrodithionyl, 2H-thiopyranyl, and the like;
   (d) those containing 1 to 2 sulfur atoms and 1 to 2 oxygen atoms, specifically, dihydroxythiopyranyl and the like; and
   (e) those containing 1 to 2 oxygen atoms, for example, furyl, dihydrofuryl, pyranyl, 2H-pyranyl, oxepinyl, dioxolyl, and the like;

(3) Fused Polycyclic Saturated Hetero Ring Groups
   (a) those containing 1 to 5 nitrogen atoms, for example, quinuclidinyl, 7-azabicyclo[2.2.1]heptyl, 3-azabicyclo[3.2.2]nonanyl, 2,8-diazaspiro[4.5]deca-8-yl, 2,3,6,8-tetraazaspiro[4.5]decan-8-yl, and the like;
   (b) those containing 1 to 4 nitrogen atoms and 1 to 3 sulfur atoms, and/or 1 to 3 oxygen atoms, for example, trithiadiazaindenyl, dioxoloimidazolidinyl, 6-oxa-2,8-diazaspiro[4.5]decan-8-yl, 6-thia-2,8-diazaspiro[4.5]decan-8-yl, and the like; and
   (c) those containing 1 to 3 sulfur atoms and/or 1 to 3 oxygen atoms, for example, 2,6-dioxabicyclo[3.2.2]octo-7-yl, 2-oxa-6-thiaspiro[4.5]decan-8-yl, and the like;

(4) Fused Polycyclic Unsaturated Hetero Ring Groups
   (a) those containing 1 to 5 nitrogen atoms, for example, indolyl, isoindolyl, indolinyl, indolidinyl, benzoimidazolyl, dihydrobenzoimidazolyl, tetrahydrobenzoimidazolyl, quinolyl, tetrahydroquinolyl, isoquinolyl, tetrahydroisoquinolyl, indazolyl, imidazopyridyl, benzotriazolyl, tetrazolopyridazinyl, carbazolyl, acridinyl, quinoxalinyl, dihydroquinoxalinyl, tetrahydroquinoxalinyl, phthalazinyl, dihydroindazolyl, benzopyrimidinyl, naphthyridinyl, quinazolinyl, cinnolinyl, pyridopyrrolidinyl, triazolopiperidinyl, 9,10-dihydroacridine, 2,8-diazaspiro[4.5]deca-3-en-8-yl, 2,3,6,8-tetraazaspiro[4.5]deca-1-en-8-yl, and the like;
   (b) those containing 1 to 4 nitrogen atoms, and 1 to 3 sulfur atoms and/or 1 to 3 oxygen atoms, for example, benzothiazolyl, dihydrobenzothiazolyl, benzothiadiazolyl, imidazothiazolyl, imidazothiadiazolyl, benzoxazolyl, dihydrobenzoxazolyl, dihydrobenzoxazinyl, benzoxadiazolyl, benzoisothiazolyl, benzoisoxazolyl, thiazolopiperidinyl, 10H-phenothiazine, 6-oxa-2,8-diazaspiro[4.5]deca-3-en-8-yl, 6-thia-2,8-diazaspiro[4.5]deca-3-en-8-yl, and the like;
   (c) those containing 1 to 3 sulfur atoms, for example, benzothienyl, benzodithiopyranyl, chromanyl, dibenzo[b,d]thienyl, and the like;
   (d) those containing 1 to 3 sulfur atoms and 1 to 3 oxygen atoms, for example, benzoxathiopyranyl, phenoxazinyl, 2-oxa-6-thiaspiro[4.5]deca-3-en-8-yl, and the like;
   (e) those containing 1 to 3 oxygen atoms, for example, benzodioxolyl, benzofuranyl, dihydrobenzofuranyl, isobenzofuranyl, chromanyl, chromenyl, isochromenyl, dibenzo[b,d]furanyl, methylenedioxyphenyl, ethylenedioxyphenyl, xanthenyl, and the like;
   etc.

Further, the "aryl", "cycloalkyl", and "hetero ring" groups as described above are meant to be monovalent groups, but these may divalent or higher groups in some cases.

The "nitrogen-containing hetero ring" group refers to one containing 1 to 5 nitrogen atoms, as in (1)(a), (1)(b), (2)(a), (2)(b), (3)(a), (3)(b), (4)(a), (4)(b), and the like, among the "hetero ring" groups above.

The "nitrogen-containing monocyclic saturated hetero ring" group refers to one containing 1 to 5 nitrogen atoms, as in (1)(a), (1)(b), and the like, among the "monocyclic saturated hetero ring" groups above.

The "nitrogen-containing monocyclic unsaturated hetero ring" group refers to one containing 1 to 5 nitrogen atoms, as in (2)(a), (2)(b), and the like, among the "hetero ring" groups above.

The "fused nitrogen-containing polycyclic saturated hetero ring" group refers to one containing 1 to 5 nitrogen atoms, as in (3)(a), (3)(b), and the like, among the "hetero ring" groups above.

The "fused nitrogen-containing polycyclic unsaturated hetero ring" group refers to one containing 1 to 5 nitrogen atoms, as in (4)(a), (4)(b), and the like, among the "hetero ring" groups above.

The "halogen" means F, Cl, Br, or I, and preferably Br.

In the present specification, the expression "which may be substituted" represents being not substituted or being substituted with 1 to 5 substituents. Further, if a plurality of substituents are included, the substituents may be the same as or different from one other. For example, -N(lower alkyl)₂ includes an ethylmethylamino group.

In the present specification, examples of the "diseases caused by undesirable cytokine signal transduction or diseases caused by abnormal cytokine signal transduction" include "diseases in which each or a combination of JAK1, JAK2 and JAK3 is involved". Among the diseases, examples of the diseases in which JAK3 is participated include manifestations of inflammatory or hyperproliferative skin diseases, or immunologically-mediated skin diseases, such as psoriasis, atopic dermatitis, contact dermatitis, eczematoid dermatitis, seborrheic dermatitis, lichen planus, pemphigus, bullous penphigoid, epidermolysis bullosa, urticaria, angioedema, vasculitides, erythema, dermal eosinophilia, lupus erythematosus, acne, alopecia, and the like, reversible obstructive airway diseases, and mucosal or vascular inflammation. Further, among these diseases, examples of the diseases in which JAK3 and JAK1 are involved include autoimmune diseases, asthma, atopic dermatitis, Alzheimer's disease, atherosclerosis, cancer, leukemia, rejection by transplantation of organs or tissues (heart, kidney, liver, bone marrow, skin, cornea, lung, pancreas, islet, small intestine, limb, muscle, nerve, intervertebral disc, trachea, myoblasts, cartilage, and the like), graft-versus-host reactions following bone marrow transplantation, and autoimmune diseases such as rheumatism, systemic lupus erythematosus (SLE), Hashimoto's thyroiditis, multiple sclerosis, myasthenia gravis, type I diabetes mellitus, complications from diabetes, and the like. Further, among these diseases, examples of the diseases in which JAK2 is involved include myeloproliferative diseases.
In an embodiment, examples of the "diseases in which each of or a combination of JAK1, JAK2, and JAK3 is involved" include psoriasis, rejections by transplantation of organs or tissues (heart, kidney, liver, bone marrow, skin, cornea, lung, pancreas, islet, small intestine, limb, muscle, nerve, intervertebral disc, trachea, myoblast, cartilage, and the like), graft-versus-host reactions following bone marrow transplantation, a rheumatism, systemic lupus erythematosus (SLE), myeloproliferative disease, asthma, type I diabetes, complications from diabetes, etc., and multiple sclerosis, and in another embodiment, psoriasis, rejections by transplantation of organs or tissues (heart, kidney, liver, bone marrow, skin, cornea, lung, pancreas, islet, small intestine, limb, muscle, nerve, intervertebral disc, trachea, myoblast, cartilage, and the like), and rheumatism.

Examples of the "expression of immunologically-mediated skin diseases" include autoimmune diseases of the eye, such as keratoconjunctivitis, vernal conjunctivitis, uveitis associated with Behcet's disease, keratitis, herpetic keratitis, conical keratitis, corneal epithelial dystrophy, keratoleukoma, ocular premphigus, Mooren's ulcer, scleritis, Grave's opthalmopathy, Vogt-Koyanagi-Harada syndrome, keratoconjunctivitis sicca (dry eye), phlyctenule, iridocyclitis, sarcoidosis, endocrine opthalmopathy, and the like.

Examples of the "reversible obstructive airways diseases" include asthma, in particular, chronic or inveterate asthma, and bronchitis.

Examples of the "asthma" include bronchial asthma, allergic asthma, intrinsic asthma, extrinsic asthma, dust asthma, and the like.

Examples of the "chronic or inveterate asthma" include late-onset asthma, airway hyper-responsiveness, and the like.

Examples of the "mucosal or vascular inflammations" include gastric ulcer, ischemic or thrombotic vascular injury, ischemic bowel diseases, enteritis, necrotizing enterocolitis, intestinal damage associated with thermal burns, leukotriene B4-mediated diseases, and the like, proctitis, eosinophilic gastroenteritis, mastocytosis, Crohn's disease, ulcerative colitis, and the like, food-related allergic diseases with symptomatic manifestation remote from the gastrointestinal tract, migraine, rhinitis, eczema, and the like, autoimmune diseases and inflammatory conditions such as primary mucosal edema, autoimmune atrophic gastritis, premature menopause, juvenile diabetes mellitus, pemphigus vulgaris, pemphigoid, sympathetic ophthalmitis, lens-induced uveitis, idiopathic leukopenia, active chronic hepatitis, idiopathic cirrhosis, discoid lupus erythematosus, autoimmune orchitis, arthritis (for example, arthritis deformans and the like), polychondritis, and the like, and allergic conjunctivitis.

Examples of the "myeloproliferative diseases" include polycythemia vera, secondary erythrocytosis, myelofibrosis, primary thrombocythemia, chronic myeloid leukemia, chronic myelomonocytic leukemia, hypereosinophilic syndrome, and systemic mastocytosis.

Examples of the "immunogenic diseases" include chronic autoimmune liver diseases such as autoimmune hepatic diseases, primary biliary cirrhosis, sclerosing cholangitis, and the like.

Examples of the "acute liver necrosis" include necrosis caused by toxins, viral hepatitis, shock, anoxia, necrosis, and the like.

Examples of the "hepatic failure" include fulminant hepatitis, late-onset hepatitis, acute liver failure, or chronic liver diseases

The "rheumatism" means the generic name of the diseases with pain and stiffness in bone, cartilage, joints, or around them, and examples thereof include Rheumatoid Arthritis (RA).

Examples of the embodiment of the substituent acceptable in the "aryl which may be substituted", the "cycloalkyl which may be substituted", and the "hetero ring group which may be substituted" in R¹³ include the groups shown in (a) to (i) below.
(a) Halogen.
(b) -OH or -O-lower alkyl (in which the lower alkyl may be substituted with 1 to 3 halogen atoms)
(c) Amino which may be substituted with 1 or 2 lower alkyl groups; or nitro,.
(d) -SH or -S-lower alkyl (in which the lower alkyl may be substituted with 1 to 3 halogen atoms)
(e) -SO₂-lowe alkyl, -SO₂-cycloalkyl, -SO₂-hetero ring group, -SO₂-aryl, or sulfamoyl which may be substituted with 1 or 2 lower alkyl groups
(f) -CHO, -CO-lower alkyl, -CO-cycloalkyl (in which the cycloalkyl may be substituted with at least one -O-lower alkyl group), saturated -CO-monocyclic hetero ring group, or cyano
(g) Aryl or cycloalkyl; this group may be substituted with halogen or -O-lower alkyl
(h) Hetero ring group; this hetero ring group may be substituted with halogen or lower alkyl (in which this lower alkyl may be substituted with halogen)
(i) Lower alkyl which may be substituted with at least one group selected from the substituents shown in (a) to (h) above

Examples of the embodiment of the substituent acceptable in the groups of "cycloalkyl which may be substituted", "cycloalkenyl which may be substituted", and "nitrogen-containing hetero ring which may be substituted" in A include the groups shown in (a) to (i) above.

Examples of the embodiment of the substituent acceptable in the "lower alkyl which may be substituted" in Y include the groups shown in (a) to (i) above.

Examples of the embodiment of the substituent acceptable in the "aryl which may be substituted" and "hetero ring group which may be substituted" in Y include the groups shown in (a) to (i) above.

Examples of the embodiment of the substituent acceptable in the "-SO₂-low alkyl which may be substituted" in R¹ include the groups shown in (g) and (h) above.

Examples of the embodiment of the substituent acceptable in the group of "hetero ring which may be substituted" in R¹ include the groups shown in (f) above.

Examples of the embodiment of the substituent acceptable in the groups of "lower alkyl which may be substituted", "aryl which may be substituted", "hetero ring group which may be substituted", and "cycloalkyl which may be substituted, formed by combination of R¹¹ and R¹² with a carbon atom to which they are bonded" in R¹¹ and R¹² include the groups shown in (a) above.

Examples of the embodiment of the substituent acceptable in the "lower alkyl which may be substituted" in R¹⁴ include the groups shown in (g) and (h) above.

Examples of the embodiment of the substituent acceptable in the "hetero ring group which may be substituted" in R¹⁴ include the groups shown in (g), (h), and (i) above.

Examples of the embodiment of the substituent acceptable in the "lower alkyl which may be substituted" and the "aryl which may be substituted" in R^{N1} and R^{N2} include the groups shown in (a), (f), (g), and (h) above.

Examples of the embodiment of the substituent acceptable in the "lower alkyl which may be substituted" and the "aryl which may be substituted" in R^{N3} and R^{N4} include the groups shown in (a), (f), (g), and (h) above.

Examples of the embodiment of the compound (I) of the present invention include a compound of the formula (I') or a salt thereof. 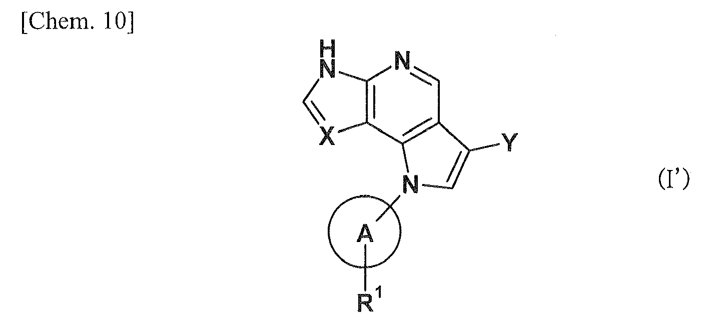 (wherein
A represents cycloalkyl which may be substituted, or a nitrogen-containing hetero ring group which may be substituted,
X represents CR^{X} or N,
R^{X} represents H, OR^{XY1}, NR^{XY2}R^{XY3}, SR^{XY4}, halogen, cyano, or a lower alkyl, aryl, or hetero ring group,
Y represents H, OR^{XY1}, NR^{XY2}R^{XY3}, SR^{XY4}, halogen, cyano, or a lower alkyl, aryl, or hetero ring group,
R^{XY1}, R^{XY2}, R^{XY3} and R^{XY4} are the same as or different from each other and represent H or lower alkyl,
R¹ represents H, OH, -(CR¹¹R¹²)ₘ-R¹³, -SO₂-(lower alkyl with which may be substituted), or a hetero ring group which may be substituted,
R¹¹ and R¹² are the same as or different from each other and represent H, halogen, OH, lower alkyl which may be substituted, aryl which may be substituted, or a hetero ring group which may be substituted,
R¹¹ and R¹² are combined with each other to form oxo (=O), or
R¹¹ and R¹² may be combined with a carbon atom to which they are bonded to form cycloalkyl,
R¹³ represents H, halogen, cyano, -NR^{N1}R^{N2}, aryl which may be substituted, cycloalkyl which may be substituted, or a hetero ring group which may be substituted,
R^{N1} and R^{N2} are the same as or different from each other and represent H, lower alkyl which may be substituted, or aryl which may be substituted, and
m represents 1, 2, 3, or 4).

Furthermore, other embodiments of the compounds (I) and (I') of the present invention are shown below.
(1) The compound, wherein A is cycloalkyl or a nitrogen-containing hetero ring group, each of which which may be substituted with lower alkyl or halogen
(2) The compound, wherein A is 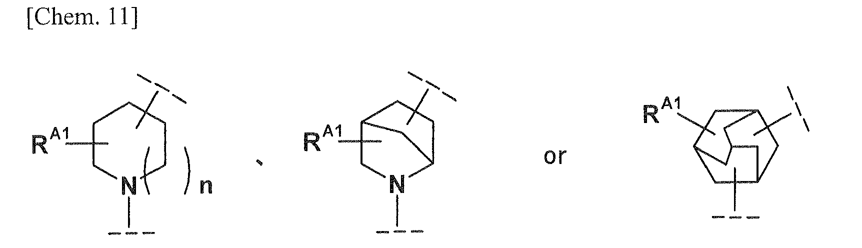 (wherein R^{A1} represents H or lower alkyl, and n represents 0, 1, or 2)
(3) The compound, wherein A is 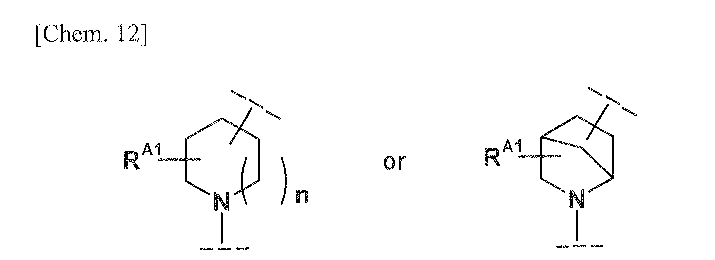 (wherein R^{A1} represents H or lower alkyl, and n represents 0, 1, or 2)
(4) The compound, wherein A is 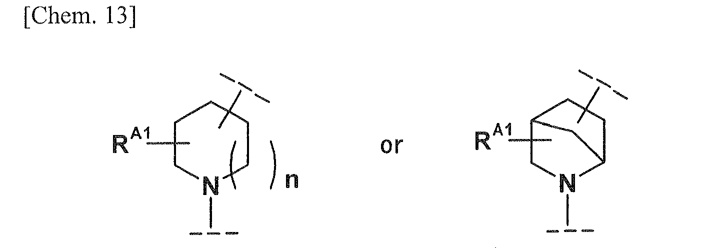 (wherein R^{A1} represents H or methyl, and n represents 0, 1, or 2)
(5) The compound, wherein A is 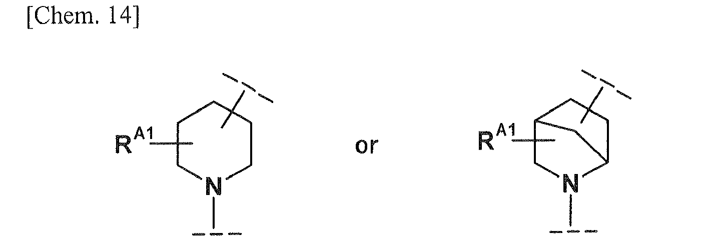 (wherein R^{A1} represents H or lower alkyl)
(6) The compound, wherein A is pyrrolidin-3-yl or azepan-4-yl, each of which is bonded to R¹ at an N atom
(7) The compound, wherein A is 4-methylpiperidin-3-yl or 2-azabicyclo[2.2.1]heptan-6-yl, each of which is bonded to R¹ at an N atom
(8) The compound, wherein A is 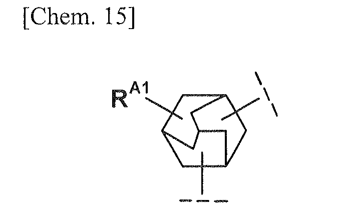
(9) The compound, wherein A is adamantan-2-yl which is bonded to R¹ at the 5-position
(10) The compound, wherein X is CR^{X}, R^{X} is H, bromo, or cyano
(11) The compound, wherein Y is H, halogen, cyano, or a hetero ring group
(12) The compound, wherein Y is H, bromo, cyano, or pyridin-4-yl
(13) The compound, wherein R¹ is OH
(14) The compound, wherein R¹ is -(CR¹¹R¹²)ₘ-R¹³, and R¹³ is cyano, -NR^{N1}R^{N2}, or a hetero ring group which may be substituted with lower alkyl
(15) The compound, wherein R¹ is -(CR¹¹R¹²)ₘ-R¹³,R¹³ is cyano, -NR^{N1}R², or a hetero ring group which may be substituted with lower alkyl, R^{N1} and R^{N2} are the same as or different from each other, and H or lower alkyl which may be substituted with cyano, and m is 1, or 2
(16) The compound, wherein R¹ is -C(=O)-R¹³ or -C(=O)-CH₂-R¹³, R¹³ is cyano, -NR^{N1}R^{N2}, or 1H-tetrazol-1-yl which may be substituted with lower alkyl, R^{N1} and R^{N2} are the same as or different from each other and represent H, or lower alkyl which may be substituted with cyano
(17) The compound, wherein R¹ is -C(=O)-CH₂-R¹³, R¹³ is cyano, or 1H-tetrazol-1-yl which may be substituted with methyl
(18) The compound, wherein R¹ is -C(=O)-R¹³, R¹³ is -NR^{N1}R^{N2}, and R^{N1} and R^{N2} are the same as or different from each other and represent H, or methyl which may be substituted with cyano
(19) The compound, wherein R¹ is pyridin-2-yl or pyrazin-2-yl, each of which may be substituted with cyano
(20) The compound, wherein R¹ is -C(=O)-CH₂-R¹³, and R¹³ is cyano, 1H-tetrazol-1-yl, or 5-methyl-1H-tetrazol-1-yl
(21) The compound, wherein R¹ is -C(=O)-R¹³, R¹³ is -NR^{N1}R^{N2} and R^{N1} and R^{N2} are the same as or different from each other and represent H, methyl, or cyanomethyl
(22) The compound, wherein R¹ is 5-cyanopyridin-2-yl or 5-cyanopyrazin-2-yl.
   Furthermore, still other embodiments of the compounds (I) and (I') of the present invention include the compounds including a combination of two or more of the groups described in (1) to (22) above, and specifically include the following compounds
(23) The compound as described in (1), (2), (8), or (9), wherein R¹ is OH
(24) The compound as described in any one of(1) to (7), wherein R¹ is -(CR¹¹R¹²)ₘ-R¹³, and R¹³ is cyano, -NR^{N1}R^{N2}, or a hetero ring group which may be substituted with lower alkyl
(25) The compound as described in any one of (1) to (7), wherein R¹ is -(CR11R¹²)ₘ-R¹³, R¹³ is cyano, -NR^{N1}R^{N2}, or a hetero ring group which may be substituted with lower alkyl, R^{N1} and R^{N2} are the same as or different from each other and represent H or lower alkyl which may be substituted with cyano, and m is 1 or 2
(26) The compound, as described in any one of(1) to (7), wherein R¹ is -C(=O)-R¹³ or -C(=O)-CH₂-R¹³, R¹³ is cyano, -NR^{N1}R^{N2}, or 1H-tetrazol-1-yl which may be substituted with lower alkyl, and R^{N1} and R^{N2} are the same as or different from each other and represent H or lower alkyl which may be substituted with cyano
(27) The compound as described in any one of (1) to (7), wherein R¹ is -C(=O)-CH₂-R¹³, and R¹³ is cyano, or 1H-tetrazol-1-yl which may be substituted with methyl
(28) The compound as described in any one of (1) to (7), wherein R¹ is -C(=O)-R¹³, R¹³ is -NR^{N1}R^{N2}, and R^{N1} and R^{N2} are the same as or different from each other and represent H, or methyl which may be substituted with cyano
(29) The compound as described in any one of (1) to (7), wherein R¹ is pyridin-2-yl or pyrazin-2-yl, each of which may be substituted with cyano
(30) The compound as described in any one of (1) to (7), wherein R¹ is -C(=O)-CH₂-R¹³, and R¹³. is cyano, 1H-tetrazol-1-yl, or 5-methyl-1H-tetrazol-1-yl
(31) The compound as described in any one of (1) to (7), wherein R¹ is -C(=O)-R¹³, and R¹³ is -NR^{N1}R^{N2}, and R^{N1} and R^{N2} are the same as or different from each other and represent H, methyl, or cyanomethyl
(32) The compound as described in any one of (1) to (7), wherein R¹ is 5-cyanopyridin-2-yl or 5-cyanopyrazin-2-yl
(33) The compound as described in any one of (1) to (7), wherein X is CR^{X}, and R^{X} is H, bromo, or cyano
(34) The compound as described in any one of (1) to (7), wherein Y is H, halogen, cyano, or a hetero ring group
(35) The compound as described in any one of (1) to (7), wherein Y is H, bromo, cyano, or pyridin-4-yl
(36) The compound as described in any one of (23) to (32), wherein X is CR^{X}, R^{X} is H, bromo, or cyano
(37) The compound as described in any one of (23) to (32), wherein Y is H, halogen, cyano, or a hetero ring group
(38) The compound as described in any one of (23) to (32), wherein Y is H, bromo, cyano, or pyridin-4-yl
(39) The compound as described in any one of (23) to (32), wherein X is CR^{X}, R^{X} is H, bromo, or cyano, and Y is H, halogen, cyano, or a hetero ring group Furthermore, still other embodiments of the compounds (I) and (I') of the present invention include the following compounds
(40) The compound, wherein X is CR^{X}, R^{X} is H, and Y is H
(41) The compound, wherein A is 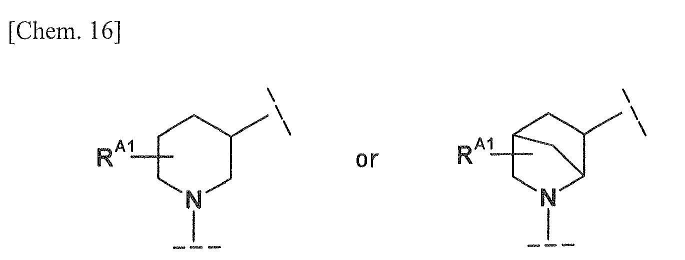 (wherein R^{A1} represents H or lower alkyl)
(42) The compound, wherein A is 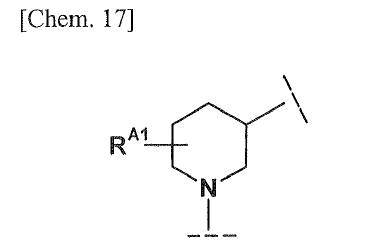 (wherein R^{A1} represents H or lower alkyl)
(43) The compound, wherein A is 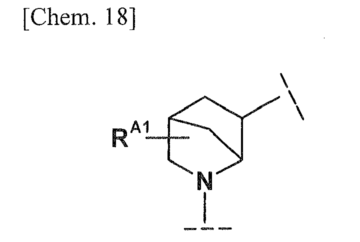 (wherein R^{A1} represents H or lower alkyl)
   In addition, still other embodiments of the compounds (I) and (I') of the present invention include the compounds including a combination of two or more of the groups described in (1) to (22) and (40) to (43) above, and specifically include the following compounds
(44) The compound as described in any one of (41) to (43), wherein R¹ is -(CR¹¹R¹²)ₘ-R¹³, and R¹³ is cyano, -NR^{N1}R^{N2}, or a hetero ring group which may be substituted with lower alkyl
(45) The compound as described in any one of (41) to (43), wherein R¹ is -(CR¹¹R¹²)ₘ-R¹³, R¹³ is cyano, -NR^{N1}R^{N2}, or a hetero ring group which may be substituted with lower alkyl, R^{N1} and R^{N2} are the same as or different from each other and represent H or lower alkyl which may be substituted with cyano, and m is 1 or 2
(46) The compound as described in any one of (41) to (43), wherein R¹ is -C(=O)-R¹³ or -C(=O)-CH₂-R¹³, R¹³ is cyano, -NR^{N1}R^{N2}, or 1H-tetrazol-1-yl which may be substituted with lower alkyl, and R^{N1} and R^{N2} are the same as or different from each other and represent H or lower alkyl which may be substituted with cyano
(47) The compound as described in any one of (41) to (43), wherein R¹ is -C(=O)-CH₂-R¹³, R¹³ is cyano, or 1H-tetrazol-1-yl which may be substituted with methyl
(48) The compound as described in any one of (41) to (43), wherein R¹ is -C(=O)-R¹³, R¹³ is -NR^{N1}R^{N2}, and R^{N1} and R^{N2} are the same as or different from each other and represent H, or methyl which may be substituted with cyano
(49) The compound as described in any one of (41) to (43), wherein R¹ is pyridin-2-yl or pyrazin-2-yl, each of which may be substituted with cyano
(50) The compound as described in any one of (41) to (43), wherein R¹ is -C(=O)-CH₂-R¹³, and R¹³ is cyano, 1H-tetrazol-1-yl, or 5-methyl-1H-tetrazol-1-yl
(51) The compound as described in any one of (41) to (43), wherein R¹ is -C(=O)-R¹³, R¹³ is -NR^{N1}R^{N2}, and R^{N1} and R^{N2} are the same as or different from each other and represent H, methyl, or cyanomethyl
(52) The compound as described in any one of (41) to (43), wherein R¹ is 5-cyanopyridin-2-yl or 5-cyanopyrazin-2-yl
(53) The compound as described in (41) to (52), wherein X is CR^{X}, and R^{X} is H, bromo, or cyano
(54) The compound as described in (41) to (52), wherein Y is H, halogen, cyano, or a hetero ring group
(55) The compound as described in (41) to (52), wherein Y is H, bromo, cyano, or pyridin-4-yl
(56) The compound as described in (54) and (55), wherein X is CR^{X}, and R^{X} is H, bromo, or cyano
(57) The compound as described in (56), wherein Y is H, halogen, cyano, or a hetero ring group
(58) The compound as described in (56), wherein Y is H, bromo, cyano, or pyridin-4-yl
(59) The compound as described in (1) to (39), or (41) to (52), wherein X is CR^{X}, R^{X} is H,and Y is H

Examples of the specific compounds encompassed by the present invention include the following compounds. Further, the "rac-" means a racemate of the compound denoted and enantiomers thereof:
(1) rac-3-[(3R,4R)-3-(dipyrrolo[2,3-b:2',3'-d]pyridin-1(6H)-yl)-4-methylpiperidin-1-yl]-3-oxopropanenitrile,
(2) rac-(1S,3R,4R,5S)-4-(dipyrrolo[2,3-b:2',3'-d]pyridin-1(6H)-yl)-adamantan-1-ol,
(3) rac-3-[(3R,4R)-3-(3-bromodipyrrolo[2,3-b:2',3'-d]pyridin-1(6H)-yl)-4-methylpiperidin-1-yl]-3-oxopropanenitrile,
(4) rac-1-[(3R,4R)-4-nlethyl-1-(1H-tetrazol-1-ylacetyl)piperidin-3-yl]-1,6-dihydrodipyrrolo[2,3-b:2',3'-d]pyridine,
(5) rac-3-[(3R,4R)-3-(8-bromodipyrrolo[2,3-b:2',3'-d]pyridin-1(6H)-yl)-4-methylpiperidin-1-yl]-3-oxopropanenitrile,
(6) rac-1-[(3R,4R)-1-(cyanoacetyl)-4-methylpiperidin-3-yl]-1,6-dihydrodipyrrolo[2,3-b:2',3'-d]pyridine-8-carbonitrile,
(7) rac-1-[(3R,4R)-1-(cyanoacetyl)-4-methylpiperidin-3-yl]-1,6-dihydrodipyrrolo[2,3-b:2',3'-d]pyridine-3-carbonitrile,
(8) rac-1-{(3R,4R)-4-methyl-1-[(5-methyl-1H-tetrazol-1-yl)acetyl]piperidin-3-yl}-1,6-dihydrodipyrrolo[2,3-b:2',3'-d]pyridine,
(9) 3-[(3S,4S)-3-dipyrrolo[2,3-b:2',3'-d]pyridin-1(6H)-yl-4-methylpiperidin-1-yl]-3-oxopropanenitrile,
(10) 3-[(3R,4R)-3-dipyrrolo[2,3-b:2',3'-d]pyridin-1(6H)-yl-4-methylpiperidin-1-yl]-3-oxopropanenitrile,
(11) (1S,3R,4R,5S)-4-(dipyrrolo[2,3-b: 2',3'-d]pyridin-1(6H)-yl)-adamantan-1-ol,
(12) (1R,3S,4S,5R)-4-(dipyrrolo[2,3-b:2',3'-d]pyridin-1(6H)-yl)-adamantan-1-ol,
(13) 3-[(3S,4S)-3-(3-bromodipyrrolo[2,3-b:2',3'-d]pyridin-1(6H)-yl)-4-methylpiperidin-1-yl]-3-oxopropanenitrile,
(14) 3-[(3R,4R)-3-(3-bromodipyrrolo[2,3-b:2',3'-d]pyridin-1(6H)-yl)-4-methylpiperidin-1-yl]-3-oxopropanenitrile,
(15) 1-[(3S4S)-4-methyl-1-(1H-tetrazol-1-ylacetyl)piperidin-3-yl]-1,6-dihydrodipyrrolo[2,3-b:2',3' -d]pyridine,
(16) 1-[(3R,4R)-4-methyl-1-(1H-tetrazol-1-ylacetyl)piperidin-3-yl]-1,6-dihydrodipyrrolo[2,3-b:2',3'-d]pyridine,
(17) 3-[(3S,4S)-3-(8-bromodipyrrolo[2,3-b:2',3'-d]pyridin-1(6H)-yl)-4-methylpiperidin-1-yl]-3-oxopropanenitrile,
(18) 3-[(3R,4R)-3-(8-bromodipyrrolo[2,3-b:2',3'-d]pyridin-1(6H)-yl)-4-methylpiperidin-1-yl]-3-oxopropanenitrile,
(19) 1-[(3S,4S)-1-(cyanoacetyl)-4-methylpiperidin-3-yl]-1,6-dihydrodipyrrolo[2,3-b:2',3'-d]pyridine-8-carbonitrile,
(20) 1-[(3R,4R)-1-(cyanoacetyl)-4-methylpiperidin-3-yl]-1,6-dihydrodipyrrolo[2,3-b:2',3'-d]pyridine-8-carbonitrile,
(21) 1-[(3S,4S)-1-(cyanoacetyl)-4-methylpiperidin-3-yl]-1,6-dihydrodipyrrolo[2,3-b:2',3'-d]pyridine-3-carbonitrile,
(22) 1-[(3R,4R)-1-(cyanoacetyl)-4-methylpiperidin-3-yl]-1,6-dihydrodipyrrolo[2,3-b:2',3'-d]pyridine-3-carbonitrile,
(23) 1-{(3S,4S)-4-methyl-1-[(5-methyl-1H-tetrazol-1-yl)acetyl]piperidin-3-yl}-1,6-dihydrodipyrrolo[2,3-b:2',3'-d]pyridine, or
(24) 1-{(3R,4R)-4-methyl-1-[(5-methyl-1H-tetrazol-1-yl)acetyl]piperidin-3-yl}-1,6-dihydrodipyrrolo[2,3-b:2',3'-d]pyridine.

The compound of the formula (I) may exist in the form of tautomers or geometrical isomers depending on the kind of substituents. In the present specification, the compound of the formula (I) shall be described in only one form of isomer, yet the present invention includes such an isomer, isolated forms of the isomers, or a mixture thereof.
In addition, the compound of the formula (I) may have asymmetric carbon atoms or axial asymmetry in some cases, and correspondingly, it may exist in the form of optical isomers. The present invention includes both an isolated form of the optical isomers of the compound of the formula (I) or a mixture thereof.

Moreover, the present invention also includes a pharmaceutically acceptable prodrug of the compound of the formula (I). The pharmaceutically acceptable prodrug is a compound having a group that can be converted into an amino group, a hydroxyl group, a carboxyl group, or the like through solvolysis or under physiological conditions. Examples of the group forming the prodrug include the groups described in Prog. Med., 5, 2157-2161 (1985) and Pharmaceutical Research and Development, Drug Design, Hirokawa Publishing Company (1990), Vol. 7, 163-189.

Furthermore, the salt of the compound of the formula (I) is a pharmaceutically acceptable salt of the compound of the formula (I) and may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or organic bases such as methylamine, ethylamine, ethanolamine, lysine, ornithine, and the like, salts with various amino acids or amino acid derivatives such as acetylleucine and the like, ammonium salts, etc.

In addition, the present invention also includes various hydrates or solvates, and polymorphic crystal substances of the compound of the formula (I) and a salt thereof. In addition, the present invention also includes compounds labeled with various radioactive or non-radioactive isotopes.

### (Preparation Methods)

The compound of the formula (I) and a salt thereof can be prepared by using the characteristics based on the basic structure or the type of substituents thereof and by applying various known synthesis methods. During the preparation, replacing the relevant functional group with a suitable protective group (a group that can be easily converted into the functional group) at the stage from starting material to an intermediate may be effective depending on the type of the functional group in the production technology in some cases. The protective group for such a functional group may include for example, the protective groups described in "Greene's Protective Groups in Organic Synthesis (4^{th} Ed., 2006)", P. G. M. Wuts and T. W. Greene, and one of these may be selected and used as necessary depending on the reaction conditions. In this kind of method, a desired compound can be obtained by introducing the protective group, by carrying out the reaction and by eliminating the protective group as necessary.
In addition, the prodrug of the compound of the formula (I) can be produced by introducing a specific group at the stage from a starting material to an intermediate or by carrying out the reaction using the obtained compound of the formula (I), just as in the case of the above-mentioned protective group. The reaction can be carried out using methods known to those skilled in the art, such as ordinary esterification, amidation, dehydration, and the like.
Hereinbelow, the representative preparation methods for the compound of the formula (I) will be described. Each of the production processes may also be carried out with reference to the References appended in the present description. Further, the preparation methods of the compound of the formula (I) are not limited to the examples as shown below.

### (Production Process 1)

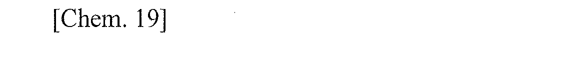

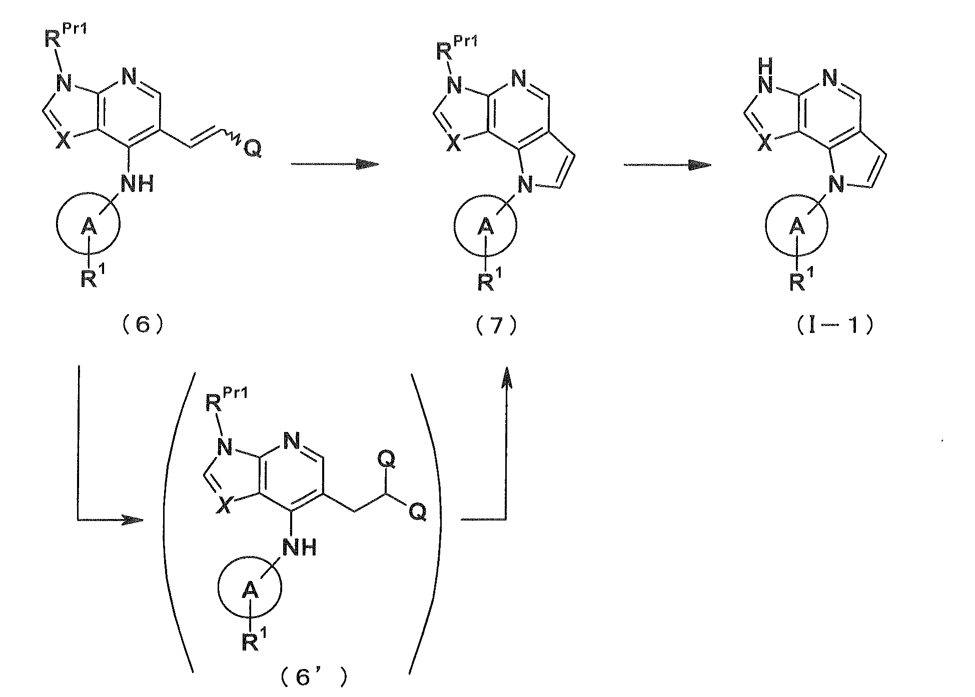

(wherein Q represents -OR^{Q}, -NHR^{Q}, -NR^{Q}₂, or -SR^{Q}, R^{Q} represents a hetero atom-protecting substituent such as lower alkyl, aryl, cycloalkyl, acyl, a hetero ring group, and the like, and R^{Pr1} represents a protecting group).
A compound (I-1) among the compounds of the present invention (I) can be obtained by converting a compound (6) to a compound (7) by a cyclization reaction, and then performing a deprotection reaction. Herein, examples of the substituent Q include a methoxy group, an ethoxy group, a tert-butoxy group, a dimethylamino group, a methylthio group, and the like, and examples of the protecting group R^{Pr1} include a tert-butoxycarbonyl group, a benzyloxycarbonyl group, a 2-(trimethylsilyl)ethoxymethyl group, and the like.
First, the compound (6) and an equivalent amount or an excess amount of Q-H are stirred under any temperature condition from cooling to heating and refluxing, and preferably at 0°C to 80°C, usually for 0.1 hours to 5 days, in a solvent which is inert to the reaction or without a solvent, to generate an acetal (6'). Examples of the solvent as used herein are not particularly limited, but include aromatic hydrocarbons such as benzene, toluene, xylene, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, ethyl acetate, acetonitrile, and a mixture thereof. It is in some cases advantageous in advancing the reaction smoothly to carry out the reaction under an acidic condition. Examples of the acid as used herein include hydrochloric acid, acetic acid, and the like, and a method in which acetyl chloride is used to generate Q-H and hydrochloric acid in a reaction system can also be employed. Further, examples of Q-H as used herein include alcohols, amines, and thiols, and although not being particularly limited, specifically methanol, ethanol, tert-butanol, methylamine, ethylamine, ethanethiol, and the like. In addition, the present reaction can also be advanced using Q-H, which has been used as a reagent, as a solvent.
Next, the acetal (6') generated is stirred under any temperature condition from cooling to heating and refluxing, preferably at 0°C to 80°C, usually for 0.1 hours to 5 days, in a solvent which is inert to the reaction or without a solvent, in the presence of an equivalent amount or an excess amount of water, to obtain a compound (7). The present reaction can be carried out with or without isolation of the acetal (6'), or it is in some cases preferable to carry out the reaction in the presence of an acid or a base. Examples of the acid as used herein are not particularly limited, but include organic acids such as p-toluenesulfonic acid, anhydrous acetic acid, and the like, and inorganic acids such as hydrochloric acid, sulfuric acid, and the like, and examples of the base are not particularly limited, but include organic bases such as triethylamine, N,N-diisopropylethylamine, N-methylmorpholine, and the like, and inorganic bases such as potassium carbonate, sodium carbonate, potassium hydroxide, sodium hydroxide, and the like.
Further, the compound (I-1) can be obtained by subjecting the compound (7) to a deprotection reaction.
Here, the present deprotection reaction can be carried out with reference to, for example, "Greene's Protective Groups in Organic Synthesis (4th Ed., 2006)", P. G. M. Wuts and T. W. Greene.

### (References)

### "Organic Functional Group Preparations", S. R. Sandler and W. Karo, 2^{nd} Ed., Vol. 1, Academic Press Inc., 1991

### (Production Process 2)

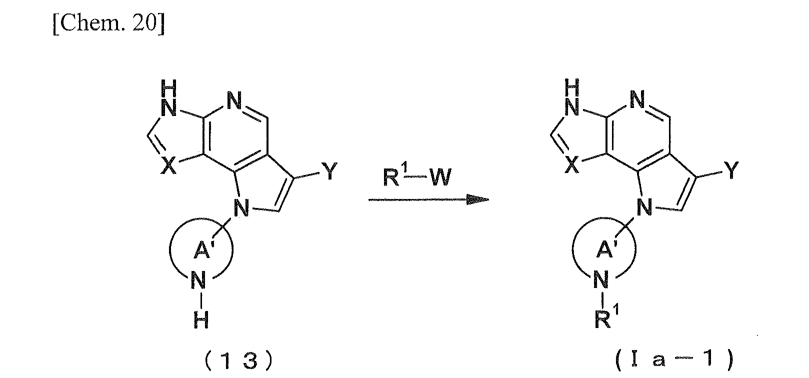

(wherein W represents a leaving group, and 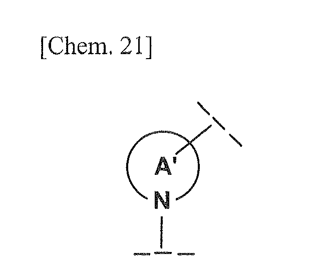 represents a nitrogen-containing hetero ring group).
A compound (Ia-1) among the compounds (I) of the present invention can be obtained by subjecting a compound (13) and R¹-W to a substitution reaction. Herein, examples of the leaving group W include halogen, a methanesulfonyloxy group, a p-toluenesulfonyloxy group, a methoxy group, an ethoxy group, and the like.
In this reaction, the compound (13) and an equivalent amount or an excess amount of R¹-W are used, and a mixture thereof is stirred under any temperature condition from cooling to heating and refluxing, preferably at 0°C to 200°C, and still more preferably at 20°C to 120°C, usually for 0.1 hours to 5 days, in a solvent which is inert to the reaction or without a solvent. It may be advantageous in some cases for the smooth progress of the reaction to carry out the reaction under microwave irradiation. Examples of the solvent as used herein are not particularly limited, but include alcohols such as methanol, ethanol, tert-butanol, and the like, aromatic hydrocarbons such as benzene, toluene, xylene, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, ethyl acetate, acetonitrile, and a mixture thereof. It may be advantageous in some cases for the smooth progress of the reaction to carry out the reaction in the presence of an organic base such as triethylamine, N,N-diisopropylethylamine, N-methylmorpholine, and the like, or an inorganic base such as sodium tert-butoxide, potassium carbonate, bis(methylsilyl)sodiumamide, sodium carbonate, potassium hydroxide, and the like.
Moreover, the reaction may be carried out using a catalyst which is not particularly limited, but includes catalysts used for an Ullmann reaction, a Buchwald-Hartwig reaction, or the like. The catalyst as used herein is not particularly limited, but a suitable combination of tris(dibenzylideneacetone)palladium, tetrakis(triphenylphosphine) palladium, or the like with 4,5-bis(diphenylphosphino)-9,9'-dimethylxanthene (Xantphos), 2-dicyclohexylphosphino-2',6'-dimethoxybipheny 1 (SPhos), 2-dicyclohexylphosphino-2',4',6'-triisopropylbiphenyl (XPhos), and the like can be used.
In addition, the reaction can also be carried out in the presence of a condensing agent. Examples of the condensing agent as used herein are not particularly limited, but include dicyclohexylcarbodiimide, diisopropylcarbodiimide, 1-ethyl-3-(3-dimethylaminopropyl)carbodiimide hydrochloride, and the like.

### (References)

"Organic Functional Group Preparations", S. R. Sandler and W. Karo, 2nd Ed., Vol. 1, Academic Press Inc., 1991
"Courses in Experimental Chemistry (5th edition)", edited by The Chemical Society of Japan, Vol. 14 (2005) (Maruzen)
Synthesis 2006, 4, 629-632

### (Production Process 3)

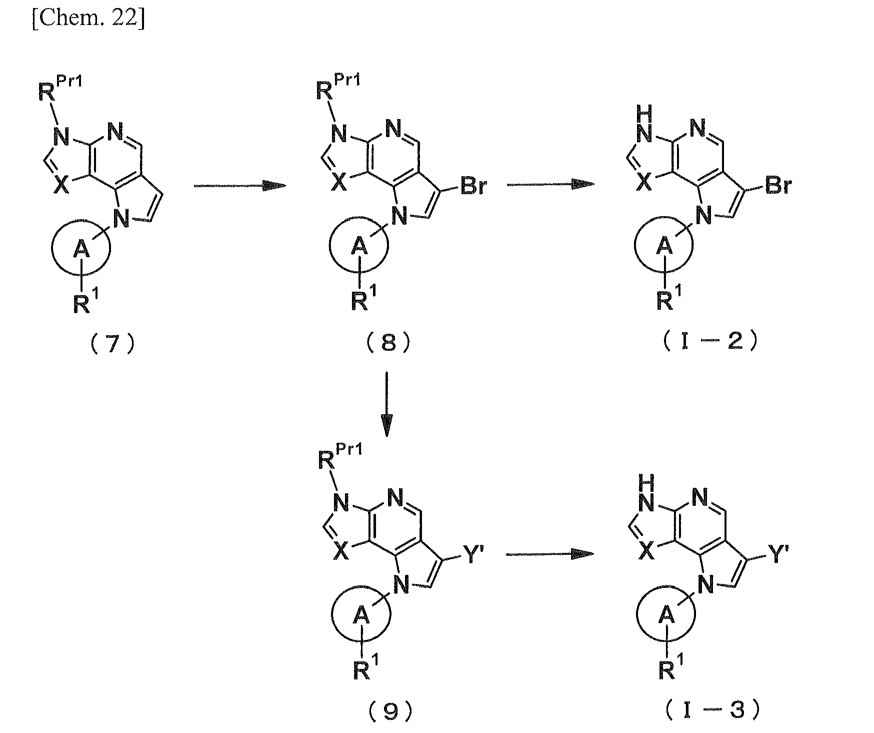

(wherein Y' represents H, or a substituent of Y other than bromine).
A compound (I-2) among the compounds (I) of the present invention can be obtained by subjecting a compound (7) to a bromination reaction and then to a deprotection reaction.
For the bromination reaction, the compound (7) and an equivalent amount or an excess amount of a brominating reagent are used, and a mixture thereof is stirred under any temperature condition from cooling to heating and refluxing, preferably at -20°C to 200°C, and still more preferably at -10°C to 150°C, usually for 0.1 hours to 5 days, in a solvent which is inert to the reaction or without a solvent. The solvent as used herein is not particularly limited, but examples thereof include alcohols such as methanol, ethanol, tert-butanol, and the like, ethers such as diethyl ether, tetrahydrofuran, dioxane, dimethoxyethane, and the like, halogenated hydrocarbons such as dichloromethane, 1,2-dichloroethane, chloroform, and the like, N,N-dimethylformamide, dimethylsulfoxide, and a mixture thereof. It may be advantageous in some cases for the smooth progress of the reaction to carry out the reaction in the presence of a Lewis acid such as aluminum chloride (AlCl₃), boron trifluoride (BF₃), and the like. Examples of the brominating reagent include in addition bromine (Br₂), N-bromosuccinimide and the like.
The deprotection reaction can be carried out using the same method as the aforementioned preparation method (Production Process 1).
On the other hand, the compound (I-3) can be obtained by subjecting a compound (8) to a substitution reaction to be coverted into the compound (9), and then performing a deprotection reaction. The substitution reaction of the compound (8) can be carried out using the same method as the aforementioned preparation method (Production Process 2), and the subsequent deprotection reaction can be carried out using the same method as the aforementioned preparation method (Production Process 1).

### (Production Process 4)

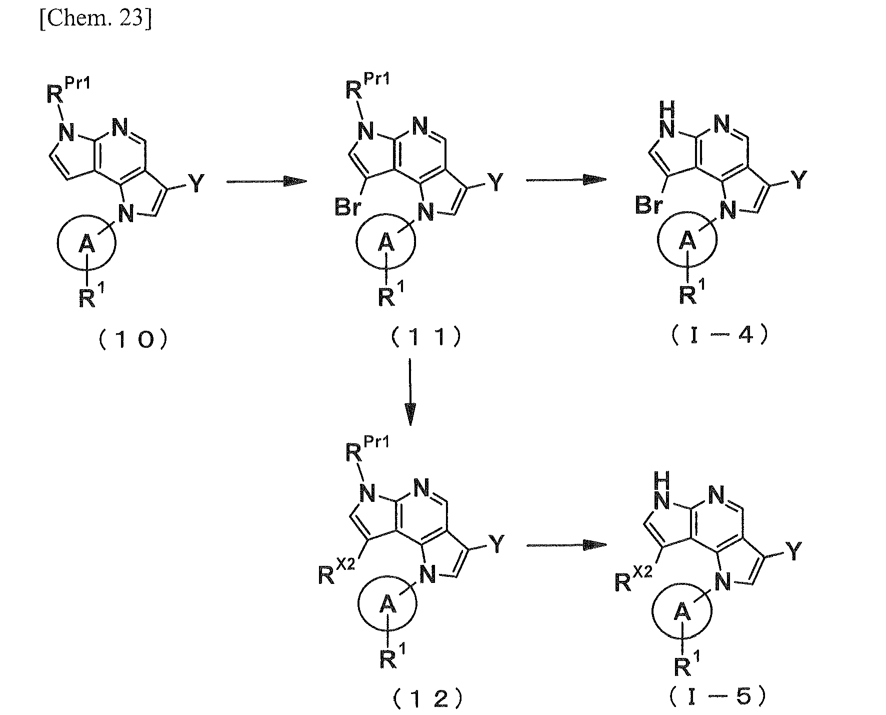

(wherein R^{X2} represents H, or the substituent of R^{X} other than bromine).
A compound (I-4) among the compounds (I) of the present invention can be obtained by subjecting a compound (10) to a bromination reaction and then to a deprotection reaction. Further, the compound (I-5) can be obtained by subjecting a compound (11) to a substitution reaction to be converted into a compound (12), and then performing a deprotection reaction.
The bromination reaction can be carried out using the same method as the aforementioned preparation method (Production Process 3), the substitution reaction can be carried out using the same method as the aforementioned preparation method (Production Process 2), and the deprotection reaction can be carried out using the same method as the aforementioned preparation method (Production Process 1).

### (Starting Material Synthesis 1)

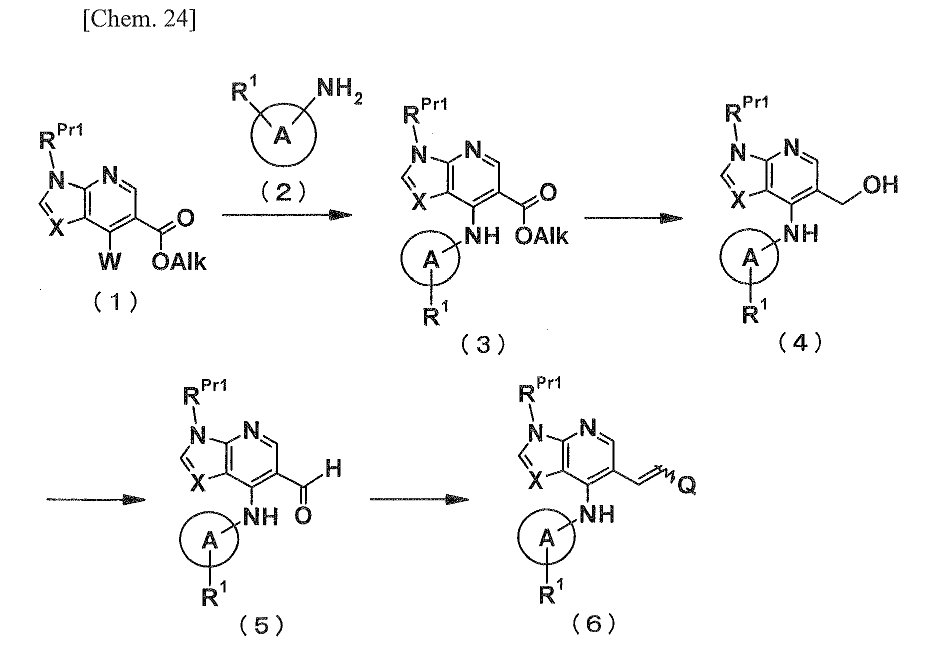

(wherein Alk represents lower alkyl).
A compound (6) can be synthesized from the compound (1) through four steps.
First, the compound (3) can be obtained by the reaction of the compound (1) and an amine (2). This reaction can be carried out using the same method as the aforementioned preparation method (Production Process 2).
Next, a compound (4) can be obtained by subjecting the compound (3) to a reduction reaction.
In this reaction, the compound (3) is treated with an equivalent amount or an excess amount of a reducing agent under any temperature condition from cooling to heating, and preferably at -20°C to 80°C, usually for 0.1 hours to 3 days. Examples of the solvent as used herein are not particularly limited, but include ethers such as diethyl ether, tetrahydrofuran, dioxane, dimethoxyethane, and the like, aromatic hydrocarbons such as benzene, toluene, xylene, and the like, and a mixture thereof. As the reducing agent, a hydrogenating reducing agent such as lithium aluminum halide, diisobutyl aluminum hydride, and the like, or a reducing agent in the references shown below is suitably used.
Furthermore, the compound (5) can be obtained by subjecting the compound (4) to an oxidation reaction.
In this reaction, the compound (4) is treated with an equivalent amount or an excess amount of an oxidant under any temperature condition from cooling to heating, and preferably at -20°C to 80°C, usually for 0.1 hours to 3 days, in a solvent which is inert to the reaction. Examples of the solvent as used herein are not particularly limited, but include ethers such as diethyl ether, tetrahydrofuran, dioxane, dimethoxyethane, and the like, halogenated hydrocarbons such as dichloromethane, 1,2-dichloroethane or chloroform, and the like, aromatic hydrocarbons such as benzene, toluene, xylene, and the like, N,N-dimethylformamide, dimethylsulfoxide, ethyl acetate, water, or a mixture thereof. As the oxidant, for example, activated manganese dioxide is suitably used.
In the present reaction, an oxidant using DMSO oxidation such as Swern oxidation and the like or using a Dess-Martin reagent is suitably used.
Finally, the compound (6) can be synthesized by subjecting the compound (5) to an addition/elimination reaction. The present reaction is not particularly limited, but can be carried out by, for example, a phosphorous compund that is used in a Wittig reaction.
In this reaction, the compound (5) is treated under any temperature condition from cooling to heating, and preferably -20°C to 80°C, usually for 0.1 hours to 3 days, in a solvent which is inert to the reaction, in the presence of an equivalent amount or an excess amount of a phosphorous compound. Examples of the solvent as used herein are not particularly limited, but include ethers such as diethyl ether, tetrahydrofuran, dioxane, dimethoxyethane, and the like, aromatic hydrocarbons such as benzene, toluene, xylene, and the like, N,N-dimethylformamide, dimethylsulfoxide, or a mixture thereof. As the phosphorous compound, for example, an alkyltriphenyl phosphonium salt is suitably used, and specific examples thereof include (methoxymethyl)triphenylphosphonium chloride, (methylthiomethyl)triphenylphosphonium chloride, and the like. It may be advantageous in some cases for the smooth progress of the reaction to carry out the reaction in the presence of a base such as sodium bis(trimethylsilyl)amide, n-butyllithium, tert-butoxypotassium, sodium ethoxide, sodium methoxide, and the like.

### (References)

"Organic Functional Group Preparations", S. R. Sandler and W. Karo, 2nd Ed., Vol. 1, Academic Press Inc., 1991
"Courses in Experimental Chemistry (5th edition)", edited by The Chemical Society of Japan, Vol. 14 (2005) (Maruzen)
Synthesis 2006, 4, 629-632
"Reductions in Organic Chemistry, 2nd ed. (ACS Monograph: 188)", M. Hudlicky, ACS, 1996
"Comprehensive Organic Transformations", 2nd ed., R.C.Larock. VCH Publishers, Inc., 1999
"Oxidation and Reduction in Organic Synthesis (Oxford Chemistry Primers 6)", T. J. Donohoe, Oxford Science Publications, 2000
"Comprehensive Organic Synthesis", B. M. Trost, Vol. 7, 1991
"Oxidation in Organic Chemistry (ACS Monograph: 186)", M. Hudlicky. ACS, 1990
"Courses in Experimental Chemistry (5th edition)", edited by The Chemical Society of Japan, Vol. 17 (2005) (Maruzen)

### (Starting Material Synthesis 2)

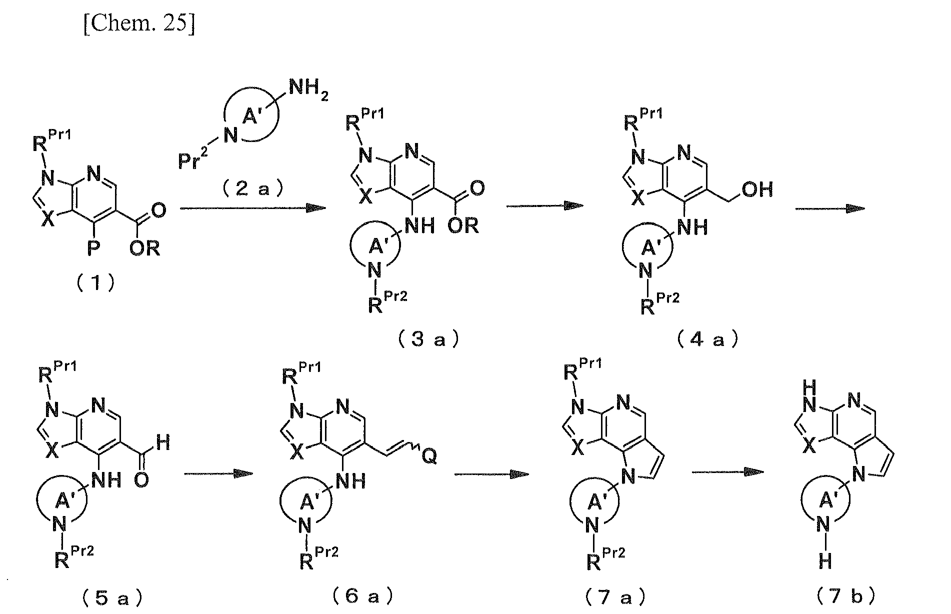

(wherein R^{Pr2} represents a protecting group).
The compound (6a) can be prepared from the compound (1) through four steps by performing the same method stepwise as in (Starting Material Synthesis 1). Herein, examples of the protecting group R^{Pr2} include a tert-butoxycarbonyl group, a benzyloxycarbonyl group, and the like.
Furthermore, the compound (7b) can be synthesized from the compound (6a) through two steps, using the same method as the cyclization reaction and the deprotection reaction described in the aforementioned preparation method (Production Process 1).

### (Starting Material Synthesis 3)

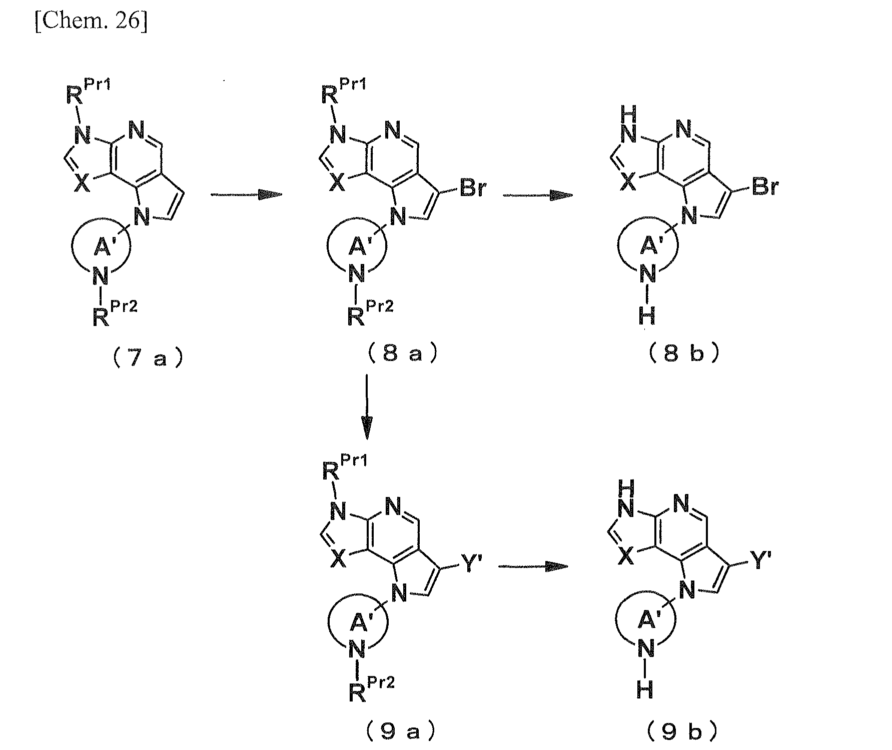

The compound (8b) can be obtained by subjecting the compound (7a) to the bromination reaction described in the aforementioned preparation method (Production Process 3) and the deprotection reaction described in the preparation method (Production Process 1).
Furthermore, the compound (9b) can be obtained by subjecting the compound (8a) to the substitution reaction described in the preparation method (Production Process 3) and the deprotection reaction described in the preparation method (Production Process 1).

### (Starting Material Synthesis 4)

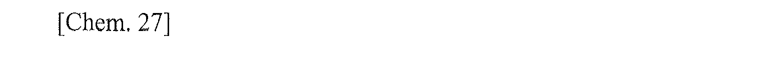

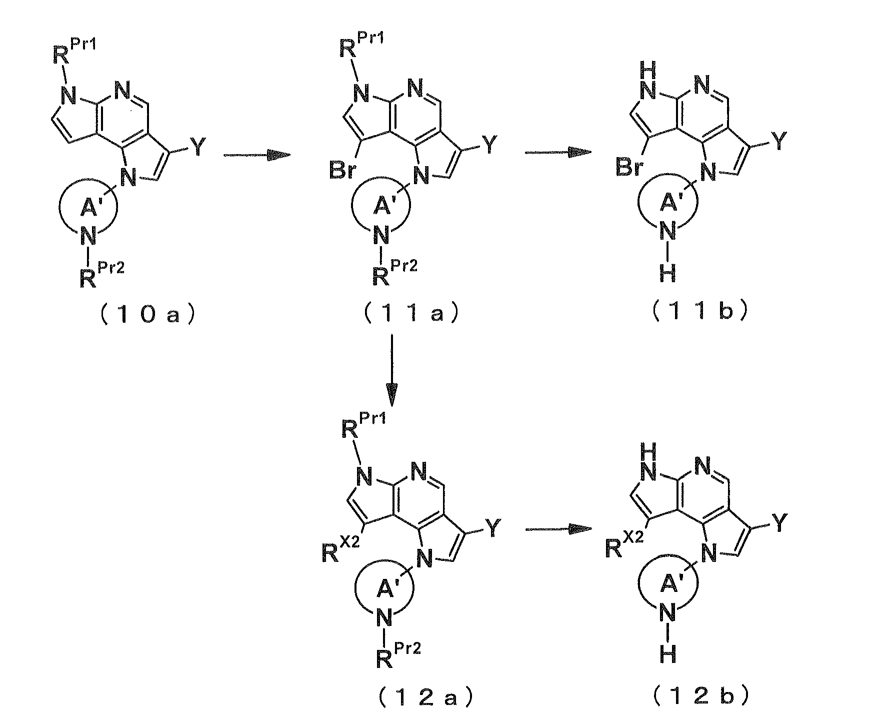

The compound (11b) can be obtained by subjecting the compound (l0a) to the bromination reaction described in the preparation method (Production Process 3) and the deprotection reaction described in the preparation method (Production Process 1).
Furthermore, the compound (12b) can be obtained by subjecting the compound (11 a) to the substitution reaction described in the preparation method (Production Process 3) and the deprotection reaction described in the preparation method (Production Process 1).

### (Starting Material Synthesis 5)

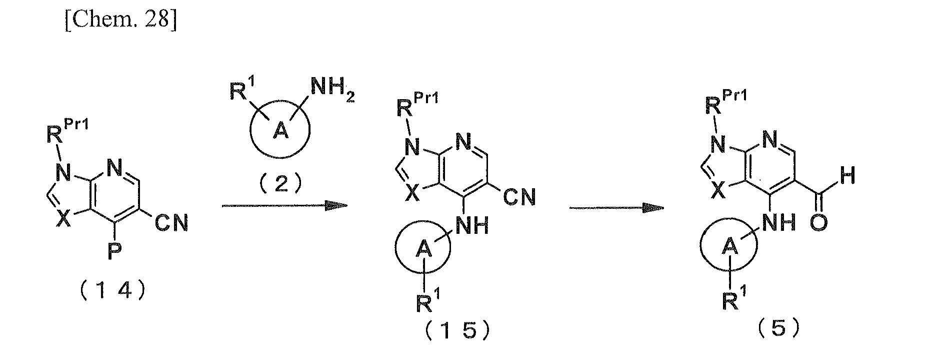

Furthermore, the compound (5) can be prepared from the compound (14) through two steps.
First, the compound (15) can be obtained by the reaction of the compound (14) and an amine (2). This reaction can be carried out, using the same method as the aforementioned preparation method (Production Process 2).
Next, the compound (5) can be obtained by subjecting the compound (15) to a reduction reaction. This reaction can be carried out, using the same method as the reduction reaction described in the aforementioned (Starting Material Synthesis 1).

### (Starting Material Synthesis 6)

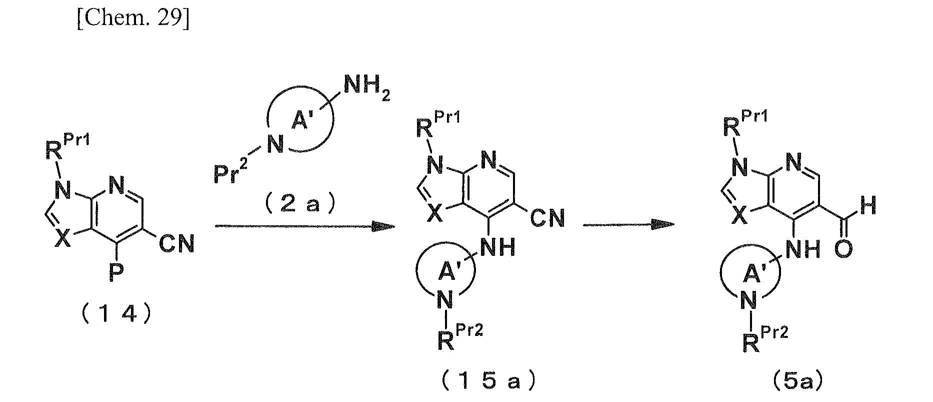

The compound (5a) can be prepared from the compound (14) through two steps, by carrying out the method described in the aforementioned (Starting Material Synthesis 5) stepwise.

The compounds of the formula (I) can be isolated and purified as their free compounds, salts, hydrates, solvates, or polymorphic crystal substances thereof. The salts of the compound of the formula (I) can be prepared by carrying out the treatment of a conventional salt forming reaction.
Isolation and purification are carried out by employing ordinary chemical operations such as extraction, fractional crystallization, various types of fractional chromatography, and the like.
Various isomers can be prepared by selecting an appropriate starting compound or separated by using the difference in the physicochemical properties between the isomers. For example, the optical isomers can be obtained by means of a general method for designing optical resolution of racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the formula (I) was confirmed by the tests shown below.
Test Example 1: JAK Inhibition Test

### (1) Preparation of Human JAK1

A purified human JAK1 kinase domain was purchased from Carna Biosciences, Inc. (Kobe, Japan). This was obtained as follows. A GST tag (62 kDa) was attached to the N-terminal of the 850 to 1154 (C-terminal) fragment of the human JAK1 protein (accession number #BAD92294.1), expressed using a baculovirus expression system, and then purified using Glutathion Sepharose chromatography.

### (2) Measurement of JAK1 Activity

As substrates, Biotin-Lyn-Substrate-2 (Biotin-XEQED EPEGF YFEWL EPE, X=ε-Acp (Peptide Institute, Inc., Osaka, Japan) and ATP were used. As an assay buffer, 15 mM Tris-HCl pH 7.5 containing 0.01 % Tween 20 and 2 mM DTT was used. Normally, 20 µL of a substrate solution (an assay buffer containing 627 nM Biotin-Lyn-Substrate-2, 500 µM ATP, and 25 mM MgCl₂), an assay buffer containing 10 µL of a test compound, and 20 µL of an enzyme solution were added to a microplate, and stirred sufficiently.
After incubation at room temperature for 1 hour, the plate was washed with a cleaning buffer (50 mM Tris-HCl pH 7.5, 150 mM NaCl, 0.02% Tween 20), and a blocking buffer (a cleaning buffer containing 0.1 % bovine serum albumin) was added to the plate. After incubation at room temperature for 30 minutes, the blocking buffer was removed, and an HRP-PY-20 solution (obtained by diluting HRP-PY-20 solution with the blocking buffer 500 times) was added. After incubation at room temperature for 30 minutes, the plate was washed four times, and a TMB substrate solution (Sigma) was added to the plate. After incubation at room temperature for 4 minutes, 1 M sulfuric acid was added to stop the reaction. Enzyme activity was measured with an absorbance at 450 nm. The JAK1 inhibitory activity of the test compound was calculated by taking the concentration of the test compound which inhibits the JAK1 activity by 50% as an IC₅₀ value.

### (3) Preparation of Human JAK2

A purified human JAK2 kinase domain was purchased from Carna Biosciences, Inc. (Kobe, Japan). This was obtained as follows. A His tag (39 kDa) was attached to the N-terminal of the 826 to 1132 (C-terminal) fragment of the human JAK2 protein (accession number #NP_004963.1), expressed using a baculovirus expression system, and then purified using Ni-NTA affinity column chromatography.

### (4) Measurement of JAK2 Activity

As substrates, Biotin-Lyn-Substrate-2 (Biotin-XEQED EPEGF YFEWL EPE, X=ε-Acp (Peptide Institute, Inc., Osaka, Japan) and ATP were used. As an assay buffer, 15 mM Tris-HCl pH 7.5 containing 0.01 % Tween 20 and 2 mM DTT was used. Normally, 20 µL of a substrate solution (an assay buffer containing 627 nM Biotin-Lyn-Substrate-2, 25 µM ATP, and 25 mM MgCl₂), an assay buffer containing 10 µL of a test compound, and 20 µL of an enzyme solution were added to a microplate, and stirred sufficiently.
After incubation at room temperature for 1 hour, the plate was washed with a cleaning buffer (50 mM Tris-HCl pH 7.5, 150 mM NaCl, 0.02% Tween 20), and a blocking buffer (a cleaning buffer containing 0.1 % bovine serum albumin) was added to the plate. After incubation at room temperature for 30 minutes, the blocking buffer was removed, and an HRP-PY-20 solution (obtained by diluting HRP-PY-20 solution with the blocking buffer 500 times) was added. After incubation at room temperature for 30 minutes, the plate was washed four times, and a TMB substrate solution (Sigma) was added to the plate. After incubation at room temperature for 4 minutes, 1 M sulfuric acid was added to stop the reaction. Enzyme activity was measured with an absorbance at 450 nm. The JAK2 inhibitory activity of the test compound was calculated by taking the concentration of the test compound which inhibits the JAK2 activity by 50% as an IC₅₀ value.

### (5) Preparation of Human JAK3

A purified human JAK3 kinase domain was purchased from Carna Biosciences, Inc. (Kobe, Japan). This was obtained as follows. A His tag (41 kDa) was attached to the N-terminal of the 796 to 1124 (C-terminal) fragment of the human JAK3 protein (accession number #NM_000215), expressed using a baculovirus expression system, and then purified using Ni-NTA affinity column chromatography.

### (6) Measurement of JAK3 Activity

As substrates, Biotin-Lyn-Substrate-2 (Biotin-XEQED EPEGF YFEWL EPE, X=ε-Acp (Peptide Institute, Inc., Osaka, Japan) and ATP were used. As an assay buffer, 15 mM Tris-HCl pH 7.5 containing 0.01 % Tween 20 and 2 mM DTT was used. Normally, 20 µL of a substrate solution (an assay buffer containing 627 nM Biotin-Lyn-Substrate-2, 20 µM ATP, and 25 mM MgCl₂), an assay buffer containing 10 µL of a test compound, and 20 µL of an enzyme solution
After incubation at room temperature for 1 hour, the plate was washed with a cleaning buffer (50 mM Tris-HCl pH 7.5, 150 mM NaCl, 0.02% Tween 20), and a blocking buffer (a cleaning buffer containing 0.1 % bovine serum albumin) was added to the plate. After incubation at room temperature for 30 minutes, the blocking buffer was removed, and an HRP-PY-20 solution (obtained by diluting HRP-PY-20 solution with the blocking buffer 500 times) was added. After incubation at room temperature for 30 minutes, the plate was washed four times, and a TMB substrate solution (Sigma) was added to the plate. After incubation at room temperature for 4 minutes, 1 M sulfuric acid was added to stop the reaction. Enzyme activity was measured with an absorbance at 450 nm. The JAK3 inhibitory activity of the test compound was calculated by taking the concentration of the test compound which inhibits the JAK3 activity by 50% as an IC₅₀ value.
The results of the JAK1, JAK2 and JAK3 inhibitory activity measurement tests of the representative compounds of the present invention are shown in Table 1. Further, each "Ex" represents the Example Compounds as described later.

**[Table 1]**

| Ex | JAK1 IC₅₀ (nM) | JAK2 IC₅₀ (nM) | JAK3 IC₅₀ (nM) |
|---|---|---|---|
| 1 | 6.1 | 1.6 | 1 |
| 4 | 16 | 3.5 | 1.1 |
| 2-5 | 1.1 | 0.6 | 0.3 |
| 4-1 | 7.9 | 3 | 0.88 |
| 5-1 | 3 | 0.89 | 0.61 |
| 2-14 | 3.1 | 2.5 | 0.52 |
| 2-4 | 6.8 | 1 | 0.26 |
| 2-15 | 4.7 | 2.9 | 1.3 |
| 2-10 | 0.49 | 0.35 | 0.19 |
| 2-2 | 6.9 | 2.5 | 0.27 |
| 2-19 | 3.2 | 1.4 | 0.11 |
| 2-17 | 0.76 | 0.53 | 0.41 |

### Test Example 2: Measurement of PD activity

Measurement of PD activity was carried out by evaluating the degree of stat5 phosphorylation of CD3+lymphocytes in the blood.
Compounds at (20 mg/kg) were orally administered to normal rats, and 4 hours later, 200 µL of blood was collected therefrom. For phosphorylation of stat5, stimulating cytokines (IL,-2, 7, 15, etc., Peprotec) were added thereto, and CD3 antibodies fluorescently labeled (BD pharmingen) was added at a dose of 1/20, followed by incubation at 37°C. 5 mL of a lysing buffer (BD Pharmingen) was added to stop the reaction, and then washed by the addition of PBS (-) (Phosphate Buffered Saline (-), manufactured by Shin Yang Chemical Industries, Ltd.). 300 µL of a permeabilization buffer (BD Pharmingen) was added thereto, followed by immobilization on ice. The resultant was washed with a FACS buffer, and then fluorescently-labeled phosphorylation stat5 antibodies (BD Pharmingen) were added thereto, followed by further incubation at room temperature. After incubation, the cells were washed with a FACS buffer, insoluble materials were removed through a filter. Fluorescence measurement was carried out using a FACScalibur. For an individual of each rat, a sample with addition of cytokine and a sample without addition of cytokine were prepared, and a difference of the respective average fluorescence intensities (ΔMFI) was determined. As a control, the blood of rats to which the compound had not been administered was used, and an inhibitory rate was calculated from the average fluorescence intensity of the respective group of the samples to which the compound had been added, relative to the average fluorescence intensity of the control sample (Inhibitory Rate (%) = 100*(1-(ΔMFI of Sample)/(ΔMFI of Control)).
The results of the representative compounds are shown below.

**[Table 2]**

| Ex | Inhibition Ratio (%) |
|---|---|
| 2-5 | 86 |
| 2-7 | 95 |
| 2-74 | 98 |
| 9-3 | 62 |

It was confirmed that the compound of the present invention has a significantly high STAT phosphorylation inhibitory rate and inhibits cytokine signal transduction well. Therefore, the compound of the present invention is useful for diseases caused by undesirable cytokine signal transduction or diseases caused by abnormal cytokine signal transduction.

### Test Example 3: Rat Heart Transplantation

Heterotopic heart transplantation into the rat abdomen was performed in accordance with reference materials. With regard to the animals, ACI-based male rats were used as donors and Lewis-based male rats were used as recipients. In addition, Lewis rats were fasted the day before the transplant. The test drugs were orally administered at a dose of 20 mg/kg once daily for 14 days from the date of transplantation. For determining the heart transplantation rejection, the heart transplantation was observed daily by palpation for 29 days after transplantation, and stoppage of beating was taken as rejection. The survival period in days was counted until the day before rejection. From the survival period in days, a value of MST (Median Survival Time) was determined. The results of the representative compounds of the compound of the present invention are shown in Tables.

### Reference Materials

1. K. One, E. S Lindsey: Improved technique of heart transplantation in rats.
J. Thorac. Cardioras. Surg, 57: 225, 1969
2. Manual for Organ Transplantation Experiment, Masumi Nozawa, Kluwer Academic Publishers, 1999

**[Table 3]**

| Ex | MST (days) |
|---|---|
| 2-7 | >28 |
| 2-10 | 17 |
| No administration of agent | 6 |

The MST of the compound of the present invention having a high STAT phosphorylation inhibitory rate was significantly long, as compared with a case where a drug is not used.
As a result of the present test, with animal models in vivo, it was confirmed that the compound of the present invention inhibits rejection after organ transplantation when administered orally at low doses.

### Test Example 4: Rat Adjuvant Arthritis (AIA) Model Test

The present test was carried out using rats with adjuvant arthritis induced by injection of killed bacteria suspended in liquid paraffin under the cutaneae pectoris of a right hind paw. Before and after induction of arthritis, the volume in the left hind paw was measured using a rat paw volume measuring device (Muromachi Kikai Co., Ltd.) by a water displacement method. Paw swelling as an index of arthritis was expressed as the change in the paw volume from an adjuvant sensitization day. An agent obtained by dissolving propylene glycol, HCO40 (manufactured by NIKKOL Chemical Co., Ltd.), Tween 80 (manufactured by Hayashi Pure Pharmaceutical Industrial Co., Ltd.), and water was administered orally once a day for 25 days from the day of adjuvant sensitization to a day 24, and an anti-inflammation effect was confirmed.

### [References]

Br. J. Pharmacol. 2003 Jul; 139 (5): 927-34.

As described above, it was confirmed that the compound of the formula (I) has JAK1, JAK2, and JAK3 inhibitory activities, and has a significantly high STAT phosphorylation inhibitory action as well as an effect of inhibiting rejection after transplantation, and can be therefore used for treating diseases caused by undesirable cytokine signal transduction or diseases caused by abnormal cytokine signal transduction, or the like.

The pharmaceutical composition containing one or two or more kinds of the compound represented by the formula (I) or salts thereof as an active ingredient can be prepared using excipients that are usually used in the art, that is, excipients for pharmaceutical preparation, carriers for pharmaceutical preparation, and the like.
Administration can be accomplished either by oral administration via tablets, pills, capsules, granules, powders, solutions, and the like, or parenteral administration, such as intraarticular, intravenous, or intramuscular injections, and the like, suppositories, ophthalmic solutions, eye ointments, transdermal liquid preparations, ointments, transdermal patches, transmucosal liquid preparations, transmucosal patches, inhalers, and the like.

The solid composition for use in the oral administration according to the present invention is used in the form of tablets, powders, granules, or the like. In such a solid composition, one or more active ingredient(s) are mixed with at least one inactive excipient. According to a conventional method, the composition may contain inactive additives, such as a lubricant, a disintegrating agent and the like, a stabilizer, or a solubilization assisting agent. If necessary, tablets or pills may be coated with sugar or a film of a gastric or enteric coating substance.
The liquid composition for oral administration contains pharmaceutically acceptable emulsions, solutions, suspensions, syrups, elixirs, or the like, and also contains generally used inert diluents, for example, purified water or ethanol. In addition to the inert diluent, the liquid composition may also contain auxiliary agents, such as a solubilization assisting agent, a moistening agent, and a suspending agent, sweeteners, flavors, aromatics, and antiseptics.

The injections for parenteral administration include sterile aqueous or non-aqueous solution preparations, suspensions and emulsions. The aqueous solvent includes, for example, distilled water for injection and physiological saline. Examples of the non-aqueous solvent include alcohols such as ethanol. Such a composition may further contain a tonicity agent, an antiseptic, a moistening agent, an emulsifying agent, a dispersing agent, a stabilizing agent, or a solubilizing aid. These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, poultices, sprays, lotions, eye drops, eye ointments, and the like. The agents contain generally used ointment bases, lotion bases, aqueous or non-aqueous liquid preparations, suspensions, emulsions, and the like.

As the transmucosal agents such as an inhaler, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate ejection agent, for example, a suitable gas such as chlorofluoroalkane, hydrofluoroalkane, carbon dioxide, and the like, or other forms.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

The compound of the formula (I) can be used in combination with various therapeutic or prophylactic agents for the diseases, in which the compound of the formula (I) is considered effective, as described above. The combined preparation may be administered simultaneously or separately and continuously, or at a desired time interval. The preparations to be co-administered may be a blend or prepared individually.

Moreover, the compound of the present invention can be administered alone as a JAK inhibitor or in combination with at least one agent in the same or different dosages via the same or different routes of administration. Agents that can be used in combination may include, but are not limited to, cyclosporin A, tacrolimus, sirolimus, everolimus, micophenolate, azathioprine, brequinar, leflunomide, fingolimod, anti-IL-2 receptor antibodies (for example, daclizumab and the like), anti-CD3 antibodies (for example, OKT3 and the like), anti-T cell immunogloblin (for example, AtGam and the like), aspirin, acetaminophen, ibuprofen, naproxen, piroxicam, anti-inflammatory steroids (for example, prednisolone or dexamethasone), and the like.

### EXAMPLES

Hereinbelow, the preparation methods for the compound of the formula (I) will be described in more detail with reference to Examples. Further, the present invention is not limited to the preparation methods described in the specific Examples and Preparation Examples as described below, but the compound of the formula (I) can be prepared by any combination of the preparation methods or the methods that are apparent to a skilled person in the art.

Furthermore, the following symbols are used in the Examples, Preparation Examples, and Tables as described below.
Pr: Preparation Example No.,
Ex: Example No.,
No.: Compound No.,
Data: Physicochemical data,
ESI+: m/z value in ESI-MS (positive ion)
ESI-: m/z value in ESI-MS (negative ion)
APCI+: m/z value in APCI-MS (positive ion)
NMR-DMSO-d₆: δ (ppm) in ¹H-NMR in DMSO-d₆,
rac-: Racemate of the compound shown in the sentence or the structural formula and enantiomer(s) thereof),
Structure: Structural formula (the "diastereomeric mixture" in the structural formula means that two binding arms in the same ring are in a mixture of various isomers with a cis configuration),
DMSO: Dimethylsulfoxide,
THF: Tetrahydrofuran,
DIBOC: Di-tert-butyldicarbonate,
LAH: Lithium aluminum hydride,
EtOAc: Ethyl acetate,
Hx: n-Hexane,
MgSO₄: Anhydrous magnesium sulfate,
DMF: N,N-Dimethylformamide,
MsCl: Methanesulfonylchloride,
brine: Saturated brine,
Na₂SO₄: Anhydrous sodium sulfate,
MeOH: Methanol,
EtOH: Ethanol,
CHCl₃: Chloroform,
CH₂Cl₂: Dichloromethane,
Et₃N: Triethylamine,
TFA: Trifluoroacetic acid,
CDI: Carbonyldiimidazole,
iPrNH₂: Isopropylamine.

### Preparation Example 1

To (methoxymethyl)triphenylphosphonium chloride (1.00 g) was added THF (10.6 mL), and sodium bis(trimethylsilyl)amide (1.07 M solution in THF, 3.22 mL) dropwise thereto under ice-cooling, followed by stirring for 30 minutes. To the reaction mixture was added dropwise a solution of rac-4-{[(3R,4R)-1-benzyl-4-methylpiperidin-3-yl]amino}-1-{[2-(trimethylsilyl)ethoxy]methyl}-1H-pyrrolo[2,3-b]pyridine-5-carbaldehyde (1.27 g) in THF (10.0 mL), followed by stirring at room temperature for 4 hours. To the reaction mixture was added a saturated aqueous ammonium chloride solution, and then the mixture was extracted with EtOAc and washed with brine. The organic layer was dried over Na₂SO₄ and then filtered, and the filtrate was concentrated under reduced pressure. The residue was purified by silica gel column chromatography (EtOAc/Hx = 10/90 to 30/70) to obtain rac-N-[(3R,4R)-1-benzyl-4-methylpiperidin-3-yl]-5-(2-methoxyvinyl)-1-{[2-(trimethylsilyl)ethoxy]methyl}-1H-pyrrolo[2,3-b]pyridin-4-amine (1.34 g).

In the same manner as the method of Preparation Example 1, the compounds of Preparation Examples 1-1 to 1-8 shown in Tables below were prepared.

### Preparation Example 2

By continuously carrying out the same method as in Preparation Examples 1 and 3, the compounds of Preparation Examples 2 and 2-1 shown in Tables below were prepared.

### Preparation Example 3

To rac-N-[(3R,4R)-1-Benzyl-4-methylpiperidin-3-yl]-5-(2-methoxyvinyl)-1-{[2-(trimethylsilyl)ethoxy]methyl}-1H-pyrrolo[2,3-b]pyridin-4-amine (1.34 g) were added MeOH (9.0 mL) and acetyl chloride (0.56 mL), followed by stirring at 80°C for 1 hour. To the reaction mixture was added water (1.0 mL), followed by stirring at 80°C for 4 hours. After completion of the reaction, to the reaction mixture was added a saturated aqueous sodium hydrogen carbonate solution, and then the mixture was extracted with EtOAc and washed with brine. The organic layer was dried over Na₂SO₄ and then filtered, and the filtrate was concentrated under reduced pressure. The residue was purified by silica gel column chromatography (EtOAc/Hx = 10/90 to 30/70) to obtain rac-1-[(3R,4R)-1-benzyl-4-methylpiperidin-3-yl]-6-{[2-(trimethylsilyl)ethoxy]methyl}-1,6-dihydrodipyrrolo[2,3-b:2',3'-d]pyridine (720 mg).

In the same manner as the method of Preparation Example 3, the compounds of Preparation Examples 3-1 to 3-8 shown in Tables below were prepared.

### Preparation Example 4

To a mixed liquid of rac-ethyl 5-amino-4-{[(3R,4R)-1-benzyl-4-methylpiperidin-3-yl]amino}-6-[(3,4-dimethoxybenzyl)amino]nicotinate (1.4 g) in triethyl orthoformate (26 mL) was added dropwise concentrated hydrochloric acid (0.44 mL) under ice-cooling, followed by stirring at room temperature for 16 hours. To the reaction mixture was added a saturated aqueous sodium hydrogen carbonate solution, and then the mixture was extracted with CHCl₃ and washed with water. The organic layer was dried over Na₂SO₄ and then filtered, and the filtrate was concentrated under reduced pressure. The residue was purified by silica gel column chromatography (CHCl₃/MeOH = 100/0 to 95/5) to obtain rac-ethyl 7-{[(3R,4R)-1-benzyl-4-methylpiperidin-3-yl]amino-3-(3,4-dimethoxybenzyl)-3H-imidazo[4,5-b]pyridine-6-carboxylate (802 mg) as white amorphous.

### Preparation Example 5

To ethyl 4-chloro-1H-pyrrolo[2,3-b]pyridine-5-carboxylate (2.00 g) was added DMF (20 ml), and the mixture was ice-cooled. Sodium hydride (60% dispersed in mineral oil) (427 mg) was added thereto, followed by stirring for 1 hour under ice-cooling. Thereafter, [2-(chloromethoxy)ethyl](trimethyl)silane (1.71 mL) was added dropwise thereto, followed by warming to room temperature and stirring for 30 minutes. After completion of the reaction, to the reaction mixture was added a saturated aqueous sodium hydrogen carbonate solution, and the mixture was extracted with EtOAc and washed with brine. The organic layer was dried over Na₂SO₄ and then filtered, and the filtrate was concentrated under reduced pressure. The residue was purified by silica gel column chromatography (EtOAc/Hx =0/100 to 10/90) to obtain ethyl 4-chloro-1-{[2-(trimethylsilyl)ethoxy]methyl}-1H-pyrrolo[2,3-b]pyridine-5-carboxylate (2.50 g) as a colorless transparent oily material.

In the same manner as the method of Preparation Example 5, the compounds of Preparation Examples 5-1 to 5-3 shown in Tables below were prepared.

### Preparation Example 6

To rac-1-[(3R,4R)-1-benzyl-4-methylpiperidin-3-yl]-6-{[2-(trimethylsilyl)ethoxy]methyl}-1,6-dihydrodipyrrolo[2,3-b:2',3'-d]pyridine (720 mg) were added CH₂Cl₂ (3.0 mL) and TFA (3.0 mL), followed by stirring at room temperature for 1.5 hours. To the reaction mixture was added a saturated aqueous sodium hydrogen carbonate solution, followed by extraction with CHCl₃. The extract was concentrated under reduced pressure. To the residue were added CH₂Cl₂ (2.0 mL), MeOH (1.0 mL), ethylenediamine (2.0 mL), and a1 M aqueous sodium hydroxide solution (3.0 mL), followed by stirring at room temperature overnight. To the reaction mixture was added water, followed by extraction with CHCl₃. The extract was dried over Na₂SO₄ and then filtered, and the filtrate was concentrated under reduced pressure. The residue was purified by silica gel column chromatography (MeOH/CHCl₃ = 0/100 to 10/90) to obtain rac-1-[(3R,4R)-1-benzyl-4-methylpiperidin-3-yl]-1,6-dihydrodipyrrolo[2,3-b:2',3'-d]pyridine (522 mg).

In the same manner as the method of Preparation Example 6, the compounds of Preparation Examples 6-1 to 6-9 shown in Tables below were prepared.

Furthermore, the compound of Preparation Example 6 was subjected to optical resolution by the following method, and the compounds of Preparation Example 6a and Preparation Example 6b were isolated as optically active forms.
rac-1-[(3R,4R)-1-Benzyl-4-methylpiperidin-3-yl]-1,6-dihydrodipyrrolo[2,3-b:2,3'-d]pyridine (310 mg) (Preparation Example 6) was collected by optical resolution by means of HPLC (DAICEL CHIRALPAK IA (5 µm 20 mmφ×250 mm)) (mobile phase: Hx/EtOH = 95/5, flow rate 8 mL/min, and room temperature) to obtain 1-[(3R,4R)-1-benzyl-4-methylpiperidin-3-yl]-1,6-dihydrodipyrrolo[2,3-b:2',3'-d]pyridine (112 mg) (Preparation Example 6a) and 1-[(3S,4S)-1-benzyl-4-methylpiperidin-3-yl]-1,6-dihydrodipyrrolo[2,3-b:2',3'-d]pyridine (119 mg) (Preparation Example 6b) at the first peak and the second peak as white solids, respectively.

### Preparation Example 7

To a mixture of rac-1-[(3R,4R)-1-benzyl-4-methylpiperidin-3-yl]-6-{[2-(trimethylsilyl)ethoxy]methyl}-1,6-dihydrodipyrrolo[2,3-b:2',3'-d]pyridine (570 mg) and 20% palladium hydroxide on carbon powder (84 mg) was added MeOH (10 mL). Further, DIBOC (314 mg) was added thereto, and the reactor was purged with hydrogen, followed by stirring at room temperature for 4 hours and 30 minutes. After completion of the reaction, the reaction mixture was filtered through Celite and the filtrate was concentrated under reduced pressure. The residue was purified by silica gel column chromatography (EtOAc/Hx= 15/85 to 20/80) to obtain rac-tert-butyl (3R,4R)-4-methyl-3-[6-{[2-(trimethylsilyl)ethoxy]methylldipyrrolo[2,3-b:2',3'-d]pyridin-1(6H)-yl]piperidine-l-carboxylate (582 mg).

### Preparation Example 8

To a mixture of rac-1-[(3R,4R)-4-methylpiperidin-3-yl]-1,6-dihydrodipyrrolo[2,3-b:2',3'-d]pyridine (103 mg) and CH₂Cl₂ (2.0 mL) were added DIBOC (220 mg) and N,N-dimethylpyridin-4-amine (4.9 mg), followed by stirring at room temperature for 30 minutes. After completion of the reaction, to the reaction mixture was added a saturated aqueous ammonium chloride solution, and the mixture was extracted with CH₂Cl₂. The organic layer was dried over Na₂SO₄ and then filtered, the filtrate was concentrated under reduced pressure. The residue was purified by silica gel column chromatography (AcOEt/Hx = 30/70 to 50/50) to obtain tert-butyl rac-1-[(3R,4R)-1-(tert-butoxycarbonyl)-4-methylpiperidin-3-yl]dipyrrolo[2,3-b:2',3'-d]pyridine-6(1H)-carboxylate (170 mg).

In the same manner as the method of Preparation Example 8, the compound of Preparation Example 8-1 shown in Tables below was prepared.

### Preparation Example 9

To tert-butyl rac-(3R,4R)-3-[8-bromo-6-{[2-(trimethylsily)ethoxy]methyl}dipyrrolo[2,3-b:2,3'-d]pyridin-1 (6H)-yl]-4-methylpiperidine-1-carboxylate (60 mg) were added CH₂Cl₂ (1.0 ml) and TFA (1.0 ml), followed by stirring at room temperature for 2 hours. The reaction mixture was concentrated under reduced pressure, and to the residue were added CH₂Cl₂ (1.0 mL), MeOH (0.5 mL), ethylenediamine (7.0 mL), and a 1 M aqueous sodium hydroxide solution (0.1 mL), followed by stirring at room temperature for 1 hour. To the reaction mixture was added water and the mixture was extracted with CH₂Cl₂. The organic layer was dried over Na₂SO₄ and then filtered, and the filtrate was concentrated under reduced pressure. The residue was purified by basic silica gel column chromatography (MeOH/CHCl₃ = 0/100 to 5/95) to obtain rac-8-bromo-1-[(3R,4R)-4-methylpiperidin-3-yl]-1,6-dihydrodipyrrolo[2,3-b:2',3'-d]pyridine (33 mg).

In the same manner as the method of Preparation Example 9, the compound of Preparation Example 9-1 shown in Tables below was prepared.

Furthermore, the compound of Preparation Example 9-1 was subjected to optical resolution by means of HPLC (DAICEL CHIRALCEL OD, 0.46 cm I.D. × 25 cm L) (mobile phase: MeCN/MeOH/iPrNH₂ = 95/5/0.1) to obtain the compound of Preparation Example 9-1a at the first peak and the compound of Preparation Example 9-1b at the second peak, which were isolated, respectively, in an optically active form.

### Preparation Example 10

In the same manner as the method of Preparation Example 9, the compounds of Preparation Examples 10-1 to 10-2 shown in Tables below were prepared.

### Preparation Example 11

1-(1-Benzylpyrrolidin-3-yl)-1,6-dihydrodipyrrolo[2,3-b:2',3'-d]pyridine (126 mg) and 20% palladium hydroxide-type palladium on carbon powder (wetted product, manufactured by N.E. CHEMCAT Corporation) (30.0 mg) were added to MeOH (1.9 mL) and THF (1.9 mL), and ammonium formate (100 mg) was further added thereto, followed by stirring at 80°C for 2 hours and 30 minutes. To the reaction mixture was added ammonium formate (100 mg), followed by stirring at 80°C for 3 hours and 30 minutes, and ammonium formate (200 mg) was further added thereto, followed by stirring at 80°C for 1 hour. The reaction mixture was filtered through Celite and the filtrate was concentrated under reduced pressure. To the residue was added a saturated aqueous sodium hydrogen carbonate solution, followed by extraction with a mixed solvent (MeOH/CHCl₃) at 90/10. The organic layer was dried over Na₂SO₄ and then filtered, and the filtrate was concentrated under reduced pressure. The residue was purified by basic silica gel column chromatography (MeOH/CHCl₃ = 0/100 to 5/95) to obtain 1-pyrrolidin-3-yl-1,6-dihydrodipyrrolo[2,3-b:2',3'-d]pyridine (71.7 mg).

In the same manner as the method of Preparation Example 11, the compounds of Preparation Examples 11-1 to 11-11 shown in Tables below were prepared.

### Preparation Example 12

rac-8-[(3R,4R)-1-Benzyl-4-methylpiperidin-3-yl]-3-(3,4-dimethoxybenzyl)-3,8-dihydroimidazo[4,5-b]pyrrolo[2,3-d]pyridine (490 mg) was dissolved in TFA (10 mL), followed by stirring at room temperature for 2 days. The reaction mixture was concentrated under reduced pressure, diisopropyl ether was added to the residue, and the precipitate was collected by filtration to obtain rac-8-[(3R,4R)-1-benzyl-4-methylpiperidin-3-yl]-3,8-dihydroimidazo[4,5-b]pyrrolo[2,3-d]pyridine trifluoroacetic acid salt(454 mg) as a white solid.

### Preparation Example 13

To rac-2-[(1R,4S,6S)-2-benzyl-2-azabicyclo[2.2.1]hept-6-yl]-1H-isoindole-1,3(2H)-dione (700 mg) were added MeOH (11 mL) and THF (11 mL), and hydrazine monohydrate (0.409 mL) was further added thereto, followed by stirring for 2 hours under heating and refluxing. The precipitate was collected by filtration using THF, and the filtrate was concentrated under reduced pressure. The residue was alkalified by the addition of a 1 M aqueous sodium hydroxide solution, followed by extraction with CHCl₃. The organic layer was dried over MgSO₄ and then filtered, and the filtrate was concentrated under reduced pressure to obtain rac-(1R, 4R, 6S)-2-benzyl-2-azabicyclo[2.2.1]heptan-6-amine (436 mg).

### Preparation Example 14

To a mixture of ethyl 4-{([(1R,2R,3S,5S)-5-hydroxyadamantan-2-yl]amino}-1-{[2-(trimethylsilyl)ethoxy]methyl}-1H-pyrrolo[2,3-b]pyridine-5-carboxylate (2.07 g) and CH₂Cl₂ (8.0 ml) was added 2,6-dimethylpyridine (0.99 mL), followed by ice-cooling. tert-Butyl(dimethyl)silyl trifluoromethanesulfonate (1.46 mL) was added dropwise thereto under ice-cooling, followed by warming to room temperature and stirring for 20 minutes. After completion of the reaction, saturated sodium hydrogen carbonate was added to the reaction mixture, and the mixture was extracted with CH₂Cl₂. The organic layer was dried over Na₂SO₄ and then filtered, and the filtrate was concentrated under reduced pressure. The residue was purified by silica gel column chromatography (EtOAc/Hx = 10/90 to 20/80) to obtain ethyl 4-{[(1R,2R,3S,5s)-5-{[tert-butyl(dimethyl)silyl]oxy}adamantan-2-yl]amino}-1-{[2-(trimethylsilyl)ethoxy]methyl}-1H-pyrrolo[2,3-b]pyridine-5-carboxylate (2.55 g).

### Preparation Example 15

A mixture of 4-chloro-1-{[2-(trimethylsilyl)ethoxy]methyl}-1H-pyrrolo[2,3-b]pyridine-5-carbonitrile (400 mg), 1-benzylpyrrolidin-3-amine (344 mg), and N,N-diisopropylethylamine (0.679 mL) was stirred at 180°C for 1 hour under microwave irradiation. The reaction mixed liquid was diluted with EtOAc and then washed with brine twice. The organic layer was dried over MgSO₄ and then filtered, and the filtrate was concentrated under reduced pressure. The residue was purified by basic silica gel column chromatography (EtOAc/Hx = 0/100 to 20/80) to obtain 4-[(1-benzylpyrrolidin-3-yl)amino]-1-{[2-(trimethylsilyl)ethoxy]methyl}-1H-pyrrolo[2,3-b]pyridine-5-carbonitrile (552 mg).

In the same manner as the method of Preparation Example 15, the compounds of Preparation Examples 15-1 to 15-12 shown in Tables below were prepared.

### Preparation Example 16

To a mixed liquid of 4,6-dichloro-5-nitronicotinic acid ethyl ester (5.7 g) in DMF (40 mL) were added rac-(3R,4R)-1-benzyl-4-methylpiperidin-3-amine (4.39 g) and N,N-diisopropylethylamine (3.7 mL), followed by stirring at room temperature for 1 hour. To the reaction mixture were added 1-(3,4-dimethoxyphenyl)methaneamine (4.8 mL) and N,N-diisopropylethylamine (9.4 mL), followed by stirring at 110°C for 2 hours. The reaction mixture was quenched with water, and then the mixture was extracted with EtOAc and washed with water. The organic layer was dried over MgSO₄ and then filtered, and the filtrate was concentrated under reduced pressure. The residue was purified by silica gel column chromatography (EtOAc/Hx = 1/4 to 1/2) to obtain ethyl rac-4-{[(3R,4R)-1-benzyl-4-methylpiperidin-3-yl]amino}-6-[(3,4-dimethoxybenzyl)amino]-5-nitronicotinate (9.9 g) as a yellow amorphous substance.

### Preparation Example 17

tert-Butyl rac-(3R,4R)-4-methyl-3-[6-{[2-(trimethylsilyl)ethoxy]methyl}dipyrrolo[2,3-b:2',3'-d]pyridin-1(6H)-yl]piperidine-1-carboxylate (500 mg) was dissolved in CH₂Cl₂ (5.0 ml), followed by ice-cooling. N-Bromosuccinimide (183 mg) was added thereto under ice-cooling, and after 10 minutes, a saturated aqueous sodium hydrogen carbonate solution was further added thereto. The mixture was warmed to room temperature and extracted with CH₂Cl₂. The organic layer was dried over Na₂SO₄ and then filtered, and the filtrate was concentrated under reduced pressure. The residue was purified by silica gel column chromatography (EtOAc/Hx = 10/90 to 30/70) to obtain rac-tert-butyl (3R,4R)-4-methyl-3-[8-bromo-6-{[2-(trimethylsilyl)ethoxy]methyl}dipyrrolo[2,3-b:2',3'-d]pyridin-1(6H)-yl]piperidine-1-carboxylate (202 mg).

### Preparation Example 18

In the same manner as the method of Preparation Example 17, the compounds of Preparation Examples 18, and 18-1 to 18-2 shown in Tables below were prepared.

### Preparation Example 19

rac-tert-Butyl (3R,4R)-4-methyl-3-[8-bromo-6-{[2-(trimethylsilyl)ethoxy]methyl } dipyrrolo[2,3-b:2',3'-d]pyridin-1 (6H)-yl]piperidine-1-carboxylate (21.2 mg), dicyanozinc (6.6 mg), tetrakistriphenylphosphinepalladium (4.3 mg), and DMF (0.3 mL) were added and reacted in a microwave reactor at 100°C for 1 hour. After completion of the reaction, to the reaction mixture was added a saturated aqueous sodium hydrogen carbonate solution, and the mixture was extracted with EtOAc. The organic layer was dried over Na₂SO₄ and then filtered, and the filtrate was concentrated under reduced pressure. The residue was purified by silica gel column chromatography (EtOAc/Hx = 10/90 to 20/80) to obtain rac-tert-butyl (3R,4R)-3-[8-cyano-6-{[2-(trimethylsilyl)ethoxy]methyl} dipyrrolo[2,3-b:2',3'-d]pyridin-1 (6H)-yl]-4-methylpiperidine-1-carboxylate (15.4 mg).

### Preparation Example 20

To a mixture of (4-{[(1R,2R,3S,5s)-5-{[tert-butyl(dimethyl)silyl]oxyadamantan-2-yl]amino}1-{[2-(trimethylsilyl)ethoxy)methyl}-1H-pyrrolo[2,3-b]pyridin-5-yl)methanol (536 mg) and manganese dioxide (835 mg) was added CH₂Cl₂ (10 ml), followed by heating and refluxing for 2 hours. After completion of the reaction, the reaction mixture was filtered through Celite and the filtrate was concentrated under reduced pressure. The residue was purified by silica gel column chromatography (EtOAc/Hx = 10/90 to 20/80) to obtain 4-{[(1R,2R,3S,5s)-5-{[tert-butyl(dimethyl)silyl]oxy}adamantan-2-yl]amino}-1{[2-(trimethylsilyl)ethoxy]methyl}-1H-pyrrolo[2,3-b]pyridine-5-carbaldehyde (465 mg).

In the same manner as the method of Preparation Example 20, the compounds of Preparation Examples 20-1 to 20-6 shown in Tables below were prepared.

### Preparation Example 21

rac-Ethyl 4-{[(3R,4R)-1-benzyl-4-methylpiperidin-3-yl]amino }-6-[(3,4-dimethoxybenzyl)amino]-5-nitronicotinate (2.65 g) was dissolved in EtOH (88 mL) and water (11 mL), and then to the mixed liquid were added reduced iron (788 mg) and ammonium chloride (251 mg), followed by stirring at 120°C for 4 hours. The reaction mixture was cooled to room temperature, and a saturated aqueous sodium hydrogen carbonate solution was added thereto, followed by stirring for 30 minutes. The insoluble materials were filtered through Celite and the filtrate was concentrated under reduced pressure. The residue was extracted with CHCl₃ and washed with water. The organic layer was dried over MgSO₄ and then filtered, and the filtrate was concentrated under reduced pressure. The residue was purified by silica gel column chromatography (CHCl₃/MeOH = 100/0 to 95/5) to obtain rac-ethyl 5-amino-4-{[(3R,4R)-1-benzyl-4-methylpiperidin-3-yl]amino}-6-[(3,4-dimethoxybenzyl)amino]nicotinate (1.4 g) as a yellow amorphous substance.

In the same manner as the method of Preparation Example 21, the compound of Preparation Example 21-1 shown in Tables below was prepared.

### Preparation Example 22

A mixture of 4-[(1-benzylpyrrolidin-3-yl)amino]-1-{[2-(trimethylsilyl)ethoxy]methyl}-1H-pyrrolo[2,3-b]pyridine-5-carbonitrile (548 mg) and THF (5.5 mL) was cooled to -70°C, and diisobutylaluminum hydride (1.0 M Hx solution, 3.06 mL) was added thereto, followed by stirring for 1 hour under ice-cooling. To the reaction mixture was slowly added MeOH, and then a 0.5 M aqueous hydrochloric acid solution (8 mL) and EtOAc (4 mL) were added thereto, followed by stirring for 1 hour. The reaction mixture was neutralized by the addition of a 1 M aqueous sodium hydroxide solution, and the mixture was extracted with EtOAc. The organic layer was dried over MgSO₄ and then filtered, and the filtrate was concentrated under reduced pressure. The residue was purified by silica gel column chromatography (MeOH/CHCl₃ = 0/100 to 2/98) to obtain 4-[(1-benzylpyrrolidin-3-yl)amino]-1-{[2-(trimethylsilyl)ethoxy]methyl}-1H-pyrrolo[2,3-b]pyridine-5-carbaldehyde (236 mg).

In the same manner as the method of Preparation Example 22, the compounds of Preparation Examples 22-1 to 22-9 shown in Tables below were prepared.

### Preparation Example 23

In the same manner as the method of Preparation Example 22, the compounds of Preparation Examples 23, and 23-1 to 23-2 shown in Tables below were prepared.

### Preparation Example 24

THF (75 mL) was ice-cooled, and powder of aluminum halide (316 mg) were added thereto. Subsequently, a solution of ethyl 4-{[(1R,2R,3S,5s)-5-{[tert-butyl(dimethyl)silyl]oxy}adamantan-2-yl]amino}-1-{[2-(trimethylsilyl)ethoxy]methyl}-1H-pyrrolo[2,3-b]pyridine-5-carboxylate (2.50 g) in THF (75 mL) was added dropwise thereto over 30 minutes, followed by warming to room temperature and stirring for 4 hours. Thereafter, after leaving to stand at room temperature for 15 hours, the mixture was stirred at 35°C for 2 hours. After further ice-cooling, water (0.32 mL), a 15% aqueous sodium hydroxide solution (0.32 mL), and water (0.32 mL) were sequently added thereto, followed by returning to room temperature and stirring for 1 hour. The reaction mixture was filtered through Celite and the filtrate was concentrated under reduced pressure. The residue was purified by silica gel column chromatography (EtOAc/Hx = 10/90 to 30/70) to obtain (4-{[(1R,2R,3S,5s)-5-{[tert-butyl(dimethyl)silyl]oxy}adamantan-2-yl]amino}-1-{[2-(trimethylsilyl)ethoxy]methyl}-1H-pyrrolo[2,3-b]pyridin-5-yl)methanol (1.10 g).

### Preparation Example 25

To a mixture of rac-(1R,4S,6S)-2-benzyl-2-azabicyclo[2.2.1]heptan-6-ol (1.00 g), 1H-isoindole-1,3(2H)-dione (796 mg), tributylphosphine (1.46 mL), and toluene (30 mL) was added 1,1'-(azodicarbonyl)dipiperidine (1.49 g) under ice-cooling, followed by stirring at room temperature overnight. Next, to the reaction mixture were added 1H-isoindole-1,3(2H)-dione (362 mg), tributylphosphine (0.728 mL), and 1,1'-(azodicarbonyl)dipiperidine (745 mg), followed by stirring at room temperature for 4 hours. To the reaction mixture was added diisopropyl ether (30 mL), followed by stirring for 30 minutes, then the reaction mixture was filtered, and the filtrate was concentrated under reduced pressure. The residue was purified by silica gel column chromatography (EtOAc/Hx = 0/100 to 34/66) to obtain rac-2-[(1R,4S,6S)-2-benzyl-2-azabicyclo[2.2.1]hept-6-yl]-1H-isoindole-1,3(2H)-dione (1.21 g).

### Preparation Example 26

To a solution of tert-butyl (4-methylpyridin-3-yl)carbarnate (12 g) in acetone (480 mL) was added benzyl bromide (6.98 mL), followed by stirring at 75°C for 3 hours. After returning to room temperature, the resulting solid was collected by filtration and washed with acetone (120 mL) to obtain 1-benzyl-3-[(tert-butoxycarbonyl)amino]-4-methylpyridium bromide (20.96 g) as a pale yellow crystal.

### Preparation Example 27

To a solution of rac-1-benzyl-3-[(tert-butoxycarbonyl)amino]-4-methylpyridium bromide (1.2 g) in EtOH (48 mL) was added platinum oxide (Adam's Catalyst) (36 mg), followed by performing catalytic reduction at 40°C under3 atm. for 4 hours. The reaction mixture was separated by filtration through Celite and the filtrate was concentrated under reduced pressure. The obtained residue was neutralized with a saturated aqueous sodium hydrogen carbonate solution (50 mL), and then the mixture was extracted with CHCl₃ (200 mL). The organic layer was dried over MgSO₄ (20 g) and then filtered, and the filtrate was concentrated under reduced pressure. The obtained residue was purified by silica gel column chromatography (EtOAc/Hx = 10/90 to 13/87) to obtain tert-butyl rac-[(3R,4R)-1-benzyl-4-methylpiperidin-3-yl]carbamate (664 mg) as a colorless oily substance.

### Preparation Example 28

By the same manner as the method of Preparation Examples 1, 3 being conducted sequentially, and 6, the compounds of Preparation Examples 28 and 28-1 to 28-2 shown in Tables below were prepared.

### Preparation Example 29

rac-tert-Butyl (3R,4R)-3-[3-bromo-8-cyano-6-{[2-(trimethylsilyl)ethoxy]methyl}dipyrrolo[2,3-b:2',3'-d]pyridin-1(6H)-yl]-4-methylpiperidine-1-carboxylate (125 mg), pyridin-4-ylboronic acid (31.3 mg), tetrakistriphenylphosphinepalladium (24.5 mg), dioxane (1.6 mL), and a 2 M aqueous sodium carbonate solution (0.8 mL) were added and reacted at 100°C for I hour. After completion of the reaction, to the reaction mixture was added water, followed by extraction with CH₂C1₂. The obtained extract was washed with brine. The organic layer was dried over Na₂SO₄ and then filtered, and the filtrate was concentrated under reduced pressure. The residue was purified by silica gel column chromatography (EtOAc/Hx = 30/70 to 95/5), and then purified by silica gel column chromatography (MeOH/CHCl₃= 0/100 to 5/95) to obtain rac-tert-butyl (3R,4R)-3-[8-cyano-3-pyridin-4-yl-6-{[2-(trimethylsilyl)ethoxy]methyl}dipyrrolo[2,3-b:2',3'-d]pyridin-1 (6H)-yl]-4-methylpiperidine-1-carboxylate (120 mg).

In the same manner as the method of Preparation Example 29, the compound of Preparation Example 29-1 shown in Tables below was prepared.

### Preparation Example 30

To a mixture of rac-4-{[(3R,4R)-1-benzyl-4-methylpiperidin-3-yl]amino}-1-{[2-(trimethylsilyl)ethoxy]methyl}-1H-pyrrolo[2,3-b]pyridine-5-carbaldehyde (1.75 g), triphenylphosphine (5.75 g), and CH₂Cl₂ (17.5 mL) was added carbon tetrabromide (3.64 g) under ice-cooling, followed by stirring for 1 hour at room temperature. To the reaction mixture was added a saturated aqueous sodium hydrogen carbonate solution under ice-cooling, followed by extraction with CHCl₃, and the organic layer was washed with a saturated aqueous sodium chloride solution. The organic layer was dried over MgSO₄, filtered, and concentrated under reduced pressure. The obtained residue was purified by silica gel column chromatography (EtOAc/Hx = 0 to 10%) to obtain rac-N-[(3R,4R)-1-benzyl-4-methylpiperidin-3-yl]-5-(2,2-dibromovinyl)-1- {[2-(trimethylsilyl)ethoxy]methyl}-1H-pyrrolo[2,3-b]pyridin-4-amine (2.07 g).

### Preparation Example 31

Benzyl rac-(3R,4R)-3-[3-(1-acetyl-1,2,3,6-tetrahydropyridin-4-yl)dipyrrolo[2,3-b:2',3'-d]pyridin-1(6H)-yl]-4-methylpiperidine-1-carboxylate (27 mg), 10% palladium on carbon powder (wetted product, manufactured by N.E. CHEMCAT Corporation) (5.6 mg), MeOH (0.6 ml), and ammonium formate (33 mg) were added, and stirred at 80°C for 15 minutes. The reaction mixture was filtered through Celite and the filtrate was concentrated under reduced pressure. To the residue was added a saturated aqueous sodium hydrogen carbonate solution, followed by extraction with a mixed solution of MeOH/CHCl₃ (9/1), and the obtained organic layer was dried over Na₂SO₄, then filtered, and concentrated under reduced pressure. The obtained residue was washed with diisopropyl ether to obtain rac-1-(4- {1-[(3R,4R)-4-methylpiperidin-3-yl]-1,6-dihydrodipyrrolo[2,3-b:2',3'-d]pyridin-3-yl}piperidin-1-yl)ethanone (10 mg) as a white powder.

### Preparation Example 32

To a solution of rac-1-[(3R,4R)-1-benzyl-4-methylpiperidin-3-yl]-6-{[2-(trimethylsilyl)ethoxy]methyl}-1,6-dihydrodipyrrolo[2,3-b:2',3'-d]pyridine (160 mg) in DMF (3.2 mL) was added N,N-dimethyleneammonium iodide (81 mg), and the reaction mixture was stirred at 60°C for 1.5 hours. After returning to room temperature, to the reaction mixture was added a saturated aqueous sodium hydrogen carbonate solution, followed by stirring. The reaction mixture was extracted with CHCl₃ and washed with water. The organic layer was dried over MgSO₄ and then filtered, and the filtrate was concentrated under reduced pressure. The obtained residue was purified by silica gel column chromatography (eluent; CHCl₃: MeOH =100:0 to 92:8) to obtain rac-1-(1-[(3R,4R)-1-benzyl-4-methylpiperidin-3-yl]-6-{[2-(trimethylsilyl)ethoxy]methyl}-1,6-dihydrodipyrrolo[2,3-b:2',3'-d]pyridin-8-yl)-N,N-dimethylmethaneamine (172 mg) as a pale yellow oily substance.

### Preparation Example 33

1-(Piperidin-4-yl)-6-{[2-(trimethylsilyl)ethoxy]methyl}-1,6-dihydrodipyrrolo[2,3-b:2',3'-d]pyridine (200 mg), K₂CO₃ (224 mg) and KI (448 mg) were added to acetonitrile (4 mL), and bromoacetonitrile (324 mg) was added thereto at room temperature, followed by stirring for 4 hours. To the reaction mixture was added pure water, followed by extraction with EtOAc. The organic layer was washed with a saturated aqueous sodium chloride solution once, and the organic layer was separated and then dried over MgSO₄. The organic layer was filtered and the filtrate was concentrated to obtain a brown oily substance. This was purified by silica gel column chromatography (CHCl₃/MeOH (1:0 to 20:1) to obtain {4-[6-{[2-(trimethylsilyl)ethoxy]methyl}dipyrrolo[2,3-b:2',3'-d]pyridin-1-(6H)-yl]piperidin-1-y1}acetonitrile (205 mg) as a pale brown oily substance.

### Preparation Example 34

To a mixture of rac-N-[(3R,4R)-1-benzyl-4-methylpiperidin-3-yl]-5-(2,2-dibromovinyl)-1-{[2-(trimethylsilyl)ethoxy]methyl}-1H-pyrrolo[2,3-b]pyridin-4-amine (2.06 g) and DMSO (20 mL) was added 1,8-diazabicyclo[5.4.0]-7-undecene (1.46 mL) under water-cooling, followed by stirring at room temperature for 1 hour. After water-cooling, the reaction mixture was neutralized with the cooled 1 N hydrochloric acid and extracted with EtOAc. The organic layer was sequentially washed with water and a saturated aqueous sodium chloride solution, then the organic layer was separated, and the organic layer was dried over MgSO₄. This organic layer was filtred and concentrated under reduced pressure. The obtained residue was purified by silica gel column chromatography (EtOAc/Hx = 0 to 10%) to obtain rac-N-[(3R,4R)-1-benzyl-4-methylpiperidin-3-yl]-5-(bromoethynyl)-1-{[2-(trimethylsilyl)ethoxy]methyl}-1H-pyrrolo[2,3-b]pyridin-4-amine (1.80 g) as a brown oily substance.

### Preparation Example 35

To a mixture of rac-N-[(3R,4R)-1-benzyl-4-methylpiperidin-3-yl]-5-(bromoethynyl)-1-{[2-(trimethylsilyl)ethoxy]methyl}-1H-pyrrolo [2,3-b]pyridin-4-amine (710 mg) and THF (7.1 mL) was added dropwise n-butyllithium (1.65 M Hx solution, 1.71 mL) at -50°C, followed by stirring for 30 minutes. Thereafter, to the reaction mixture was added methyl iodide (0.958 mL), followed by stirring at room temperature for 1.75 hours. To the reaction mixture was added a saturated aqueous ammonium chloride solution, followed by extraction with EtOAc, and the organic layer was washed with a saturated aqueous sodium chloride solution. The organic layer was separated and then dried over MgSO₄. The organic layer was filtered and concentrated under reduced pressure. The obtained residue was purified by silica gel column chromatography (EtOAc/Hx = 0/100 to 7/93) to obtain rac-N-[(3R,4R)-1-benzyl-4-methylpiperidin-3-yl]-5-prop-1-yn-1-yl-1-{[2-(trimethylsilyl)ethoxy]methyl}-1H-pyrrolo[2,3-b]pyridin-4-amine (316 mg) as a yellow oily substance.

### Preparation Example 36

To a mixture of rac-N-[(3R,4R)-1-benzyl-4-methylpiperidin-3-yl]-5-prop-1-yn-1-yl-1-{[2-(trimethylsilyl)ethoxy]methyl}-1H-pyrrolo[2,3-b]pyridin-4-amine (440 mg) and THF (4.4 mL) was added potassium tert-butoxide (253 mg) at room temperature, followed by warming to 50°C and stirring for 1 hour. Thereafter, the mixture was returned to room temperature, and potassium t-butoxide (253 mg) was added thereto, followed by stirring at 50°C for additional 1.5 hours. To the reaction mixture was added a saturated aqueous ammonium chloride solution, followed by extraction with CHCl₃. The organic layer was washed with a saturated aqueous sodium chloride solution, and then the organic layer was separated. This organic layer was dried over MgSO₄, filtered, and concentrated under reduced pressure. The obtained residue was purified by silica gel column chromatography (EtOAc/Hx = 0 to 15%) to obtain rac-1-[(3R,4R)-1-benzyl-4-methylpiperidin-3-yl]-2-methyl-6-{ [2-(trimethylsilyl)ethoxy]methyl}-1,6-dihydrodipyrrolo[2,3-b:2',3'-d]pyridine (294 mg) as a yellow oily substance.

### Preparation Example 37

cis-4-[6-{[2-(Trimethylsilyl)ethoxy]methyl}ldipyrrolo[2,3-b:2',3'-d]pyridin-1(6H)-yl]cyclohexylbenzoate (214 mg) was added to MeOH (4.3 mL), and 1 M NaOH (0.9 mL) was added thereto at room temperature, followed by stirring at 60°C for 1 hour. Thereafter, MeOH (4.3 mL) and 1 M NaOH (0.9 mL) were sequentially added thereto at room temperature, followed by stirring at 60°C for 1 hour. To the reaction mixture was added pure water, followed by extraction with EtOAc once. The organic layer was washed once with a saturated aqueous sodium chloride solution, and the organic layer was separated and then dried over MgSO₄. The organic layer was filtered and concentrated to obtain cis-4-[6-{ [2-(trimethylsilyl)ethoxy]methyl}dipyrrolo[2,3-b:2',3'-d]pyridin-1 (6H)-yl]cyclohexanol (168 mg) as a colorless oily substance.

### Preparation Example 3 8

Under nitrogen air flow, a mixture of sulfuryl dichloride (1.80 g) and CH₂C1₂ (30 ml) was cooled to -60°C. To the mixture was added dropwise a mixture of (methylamino)acetonitrile (920 mg), 4-dimethylaminopyridine (1.48 g), and CH₂Cl₂ (20 ml) over 10 minutes, followed by warming to room temperature and stirring overnight. To the reaction mixture was added silica gel, and the solvent was evaporated under reduced pressure. The obtained residue was filtered through silica gel (hexane/EtOAc = 1/1) to obtain (cyanomethyl)methylsulfamyl chloride (1.09 g).

In the same manner as the method of Preparation Example 38, the compound of Preparation Example 38-1 shown in Tables below was prepared.

### Preparation Example 39

To 6-{[2-(trimethylsilyl)ethoxy]methyl}-1,6-dihydrodipyrrolo[2,3-b:2',3'-d]pyridine (200 mg) were added dioxane (2 ml), 3-iodopyridine (171 mg), potassium phosphate (310 mg), rac-(1R,2R)-cyclohexane-1,2-diamine (64 mg), and copper iodide (I) (53 mg), followed by stirring at 110°C overnight. To the reaction mixture was added EtOAc, and the insoluble materials were removed by filtration. The filtrate was concentrated under reduced pressure, and the obtained residue was purified by silica gel column chromatography (Hx/EtOAc = 100/0 to 50/50) to obtain 1-(pyridin-3-yl)-6-{[2-(trimethylsilyl)ethoxy]methyl}-1,6-dihydrodipyrrolo[2,3-b:2',3'-d]pyridine (192 mg) as a pale yellow oily substance.

In the same manner as the method of Preparation Example 38, the compounds of Preparation Examples 39-1 to 39-3 shown in Tables below were prepared.

### Preparation Example 40

To 4-[(4-methoxybenzyl)amino]-1-{[2-(trimethylsilyl)ethoxy]methyl}-1H-pyrrolo[2,3-b]pyridine-5-carbonitrile (120 mg) were added toluene (4 mL), water (1 mL), and 2,3-dichloro-5,6-dicyano-1,4-benzoquinone (200 mg), followed by stirring at 80°C for 2 hours. To the reaction mixture was added a saturated aqueous sodium hydrogen carbonate solution, followed by extraction with CHCl₃ The extract was dried over MgSO₄ and then filtered, and the filtrate was concentrated under reduced pressure. The obtained residue was purified by silica gel column chromatography (Hx/EtOAc = 100/0 to 70/30) to obtain 4-amino-1-{[2-(trimethylsilyl)ethoxy]methyl}-1H-pyrrolo[2,3-b]pyridine-5-carbonitrile (81 mg) as a white solid.

### Preparation Example 41

rac-tert-Butyl (3R,4R)-3-[3-bromo-6-{[2-(trimethylsilyl)ethoxy]methyl}dipyrrolo[2,3-b:2',3'-d]pyridin-1(6H)-yl]-4-methylpiperidine-1-carboxylate (370 mg) was dissolved in MeOH (3.0 ml). To the mixture was added a 4 M hydrogen chloride-dioxane solution (3.0 ml), followed by stirring at room temperature for 30 minutes. The reaction mixture was concentrated under reduced pressure, and a saturated aqueous sodium hydrogen carbonate solution was added thereto, followed by extraction with CH₂Cl₂. The organic layer was dried over Na₂SO₄, then filtered, and concentrated under reduced pressure. The obtained residue was purified by silica gel column chromatography (MeOH: CHCl₃=0: 100 to 10: 90) to obtain rac-3-bromo-1-[(3R,4R)-4-methylpiperidin-3-yl]-6-{[2-(trimethylsilyl)ethoxy]methyl}-1,6-dihydrodipyrrolo[2,3-b:2',3'-d]pyridine (283 mg).

In the same manner as the method of Preparation Example 41, the compound of Preparation Example 41-1 shown in Tables below was prepared.

### Preparation Example 42

In the same manner as the method of Example 7 above, the compound of Preparation Example 42 was prepared.

### Preparation Example 43

To a mixture of rac-3-bromo-1-[(3R,4R)-4-methylpiperidin-3-yl]-6-{[2-(trimethylsilyl)ethoxy]methyl}-1,6-dihydrodipyrrolo[2,3-b:2',3'-d]pyridine (280 mg), CH₂Cl₂ (3.0 ml), and a saturated aqueous sodium hydrogen carbonate solution (3.0 ml) was added benzyl chloroformate (104 µl), followed by stirring at room temperature for 15 minutes. After completion of the reaction, a saturated aqueous sodium hydrogen carbonate solution was added thereto, followed by extraction with CH₂Cl₂. The organic layer was dried over Na₂SO₄ and then filtered, and the filtrate was concentrated under reduced pressure. The residue was purified by silica gel column chromatography (EtOAc:Hx=10:90 to 30:70) to obtain benzyl rac-(3R,4R)-3-[3-bromo-6-{[2-(trimethylsilyl)ethoxy]methyl}dipyrrolo[2,3-b:2',3'-d]pyridin-1(6H)-yl]-4-methylpiperidine-1-carboxylate (328 mg).

### Preparation Example 44

In the same manner as in the method of Preparation Example 25, the compound of Preparation Example 44 and and the compound of Preparation Example 44-1, as shown in Tables below, were prepared, using trans-4-[6-{[2-(trimethylsilyl)ethoxy]methyl}dipyrrolo[2,3-b:2',3'-d]pyridin-1(6H)-yl]cyclohexanol as a starting material.

For the Preparation Example Compounds, the structures are shown in Tables 4 to 13 and Tables 33 to 40, and the physicochemical data are shown in Tables 14 to 15 and Tables 41 to 42.

**[Table 4]**

| Pr | Strucure | Pr | Strucure |
|---|---|---|---|
| 1 | 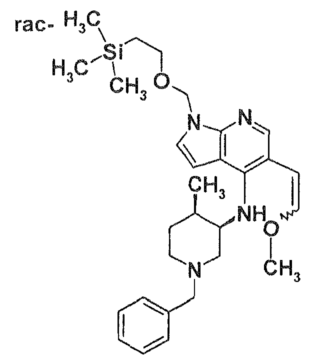 | 1-1 | 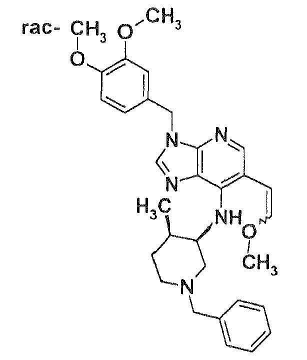 |
| 1-2 | 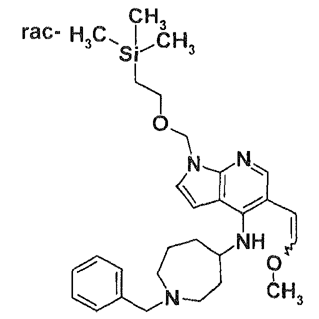 | 1-3 | 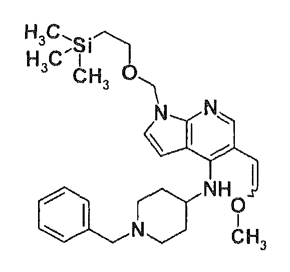 |
| 2 | 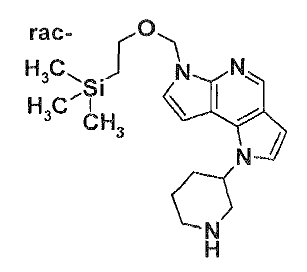 | 3 | 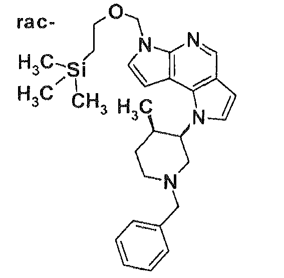 |

**[Table 5]**

| Pr | Strucure | Pr | Strucure |
|---|---|---|---|
| 3-1 | 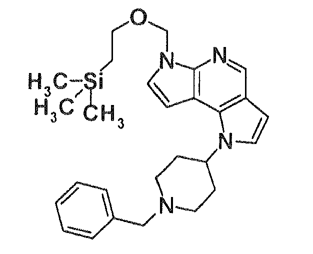 | 3-2 | 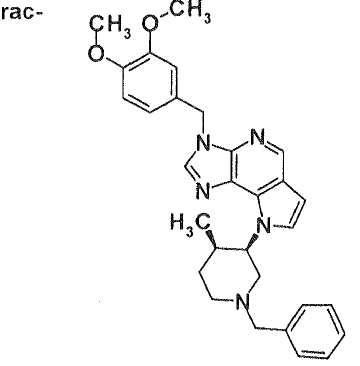 |
| 3-3 | 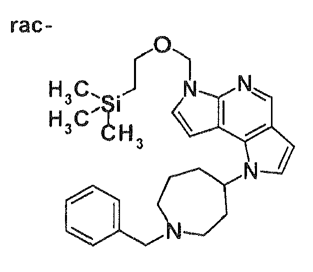 | 4 | 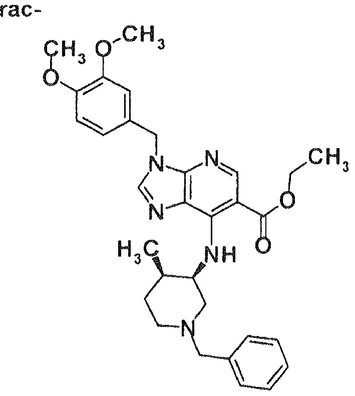 |
| 5 | 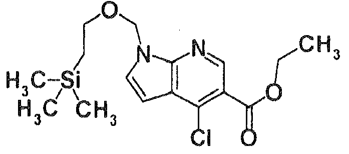 | 5-1 | 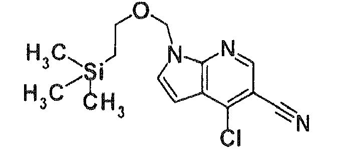 |

**[Table 6]**

| Pr | Strucure | Pr | Structure |
|---|---|---|---|
| 6 | 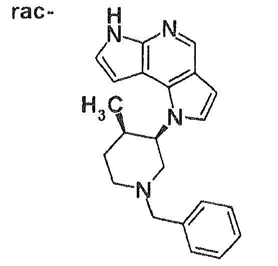 | 6a | 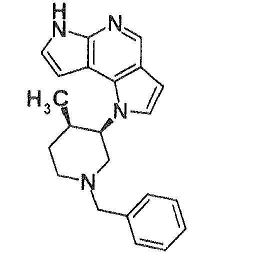 |
| 6b | 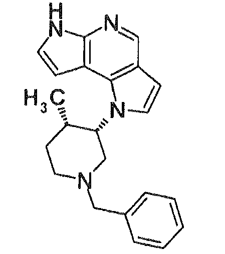 | 6-1 | 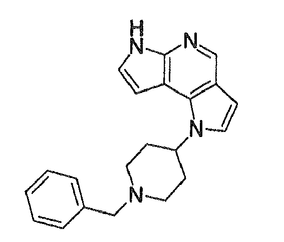 |
| 6-2 | 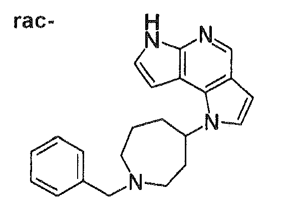 | 6-3 | 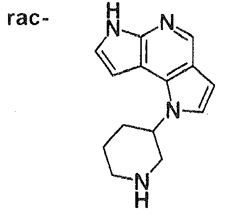 |
| 6-4 | 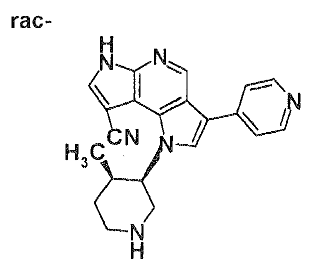 | 7 | 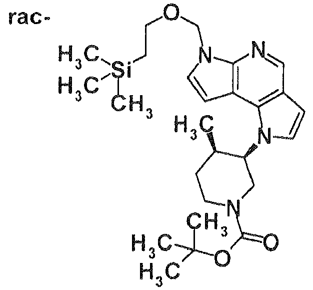 |

**[Table 7]**

| Pr | Strucure | Pr | Strucure |
|---|---|---|---|
| 8 | 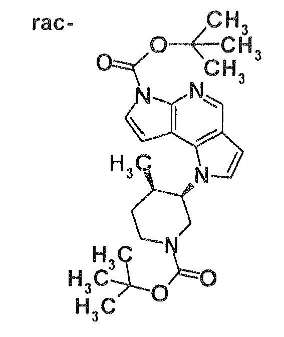 | 8-1 | 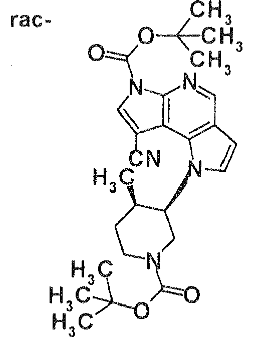 |
| 9 | 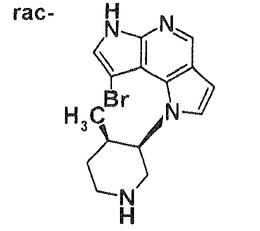 | 9-1 | 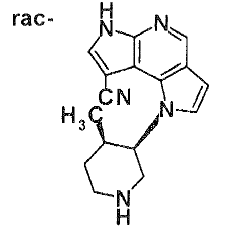 |
| 9-1a | 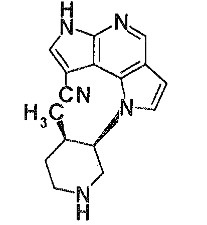 | 9-1b | 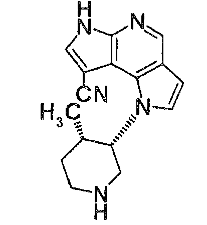 |
| 10 | 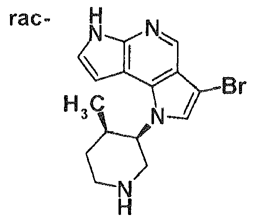 | 10-1 | 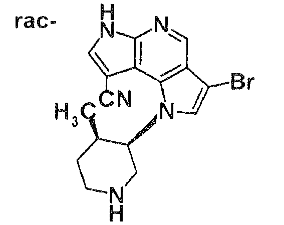 |

**[Table 8]**

| Pr | Strucure | Pr | Strucure |
|---|---|---|---|
| 11 | 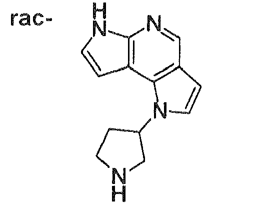 | 11-1 | 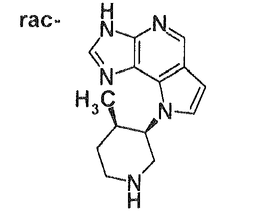 |
| 11-2 | 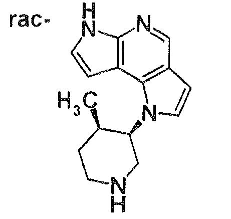 | 11-3 | 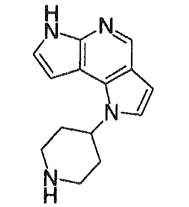 |
| 11-4 | 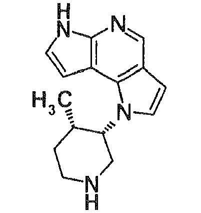 | 11-5 | 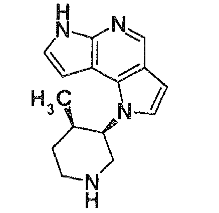 |
| 11-6 | 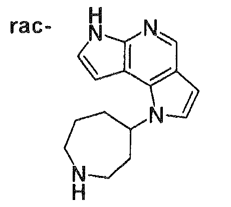 | 11-7 | 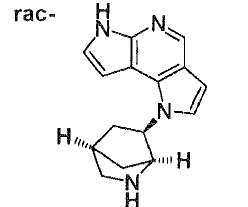 |
| 11-8 | 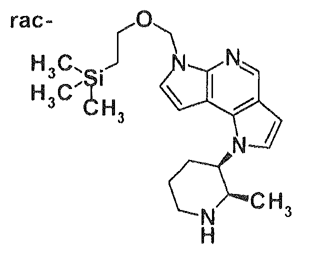 | 12 | 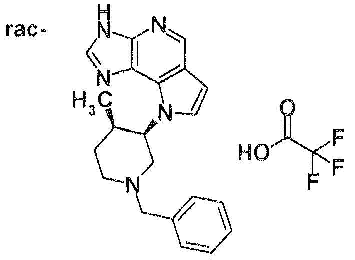 |

**[Table 9]**

| Pr | Strucure | Pr | Strucure |
|---|---|---|---|
| 13 | 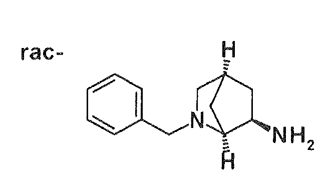 | 14 | 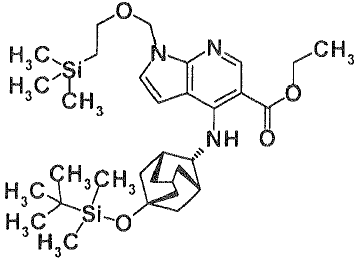 |
| 15 | 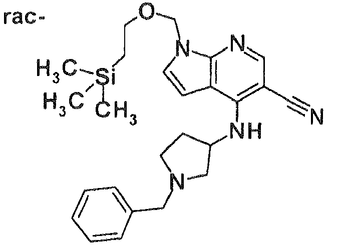 | 15-1 | 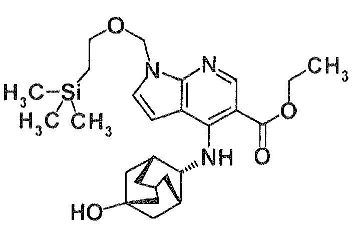 |
| 15-2 | 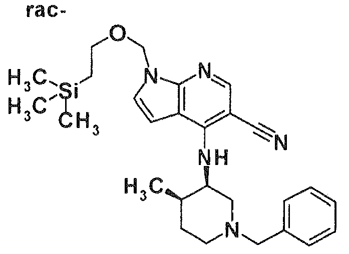 | 15-3 | 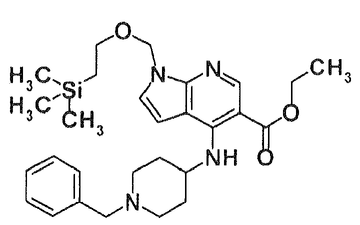 |
| 15-4 | 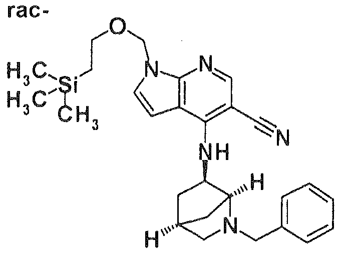 | 15-5 | 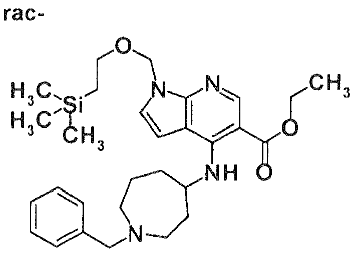 |

**[Table 10]**

| Pr | Strucure | Pr | Strucure |
|---|---|---|---|
| 15-6 | 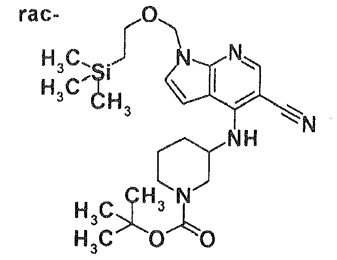 | 15-7 | 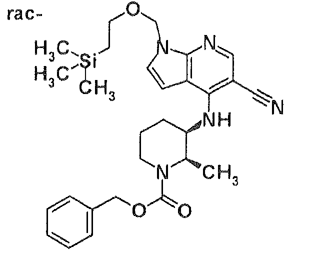 |
| 16 | 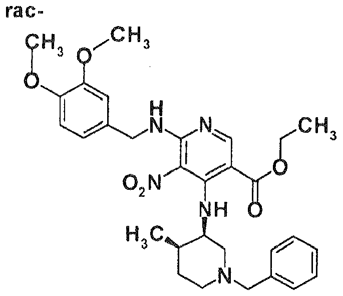 | 17 | 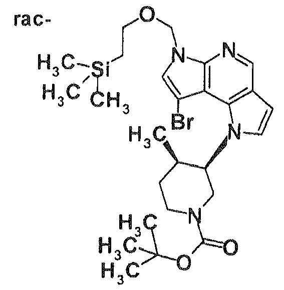 |
| 18 | 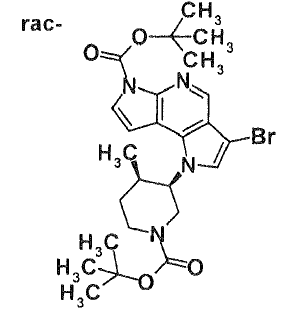 | 18-1 | 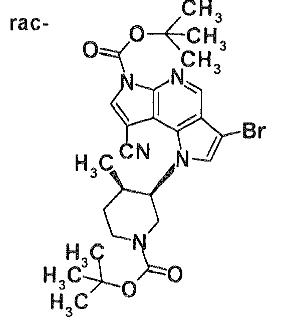 |
| 18-2 | 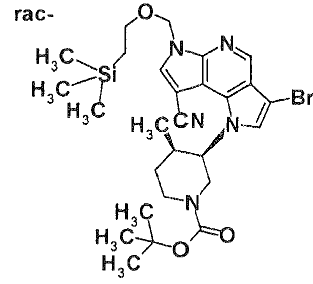 | 19 | 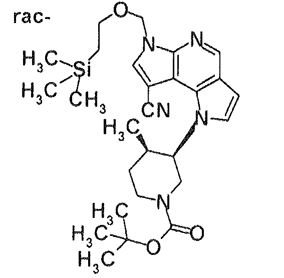 |

**[Table 11]**

| Pr | Strucure | Pr | Strucure |
|---|---|---|---|
| 20 | 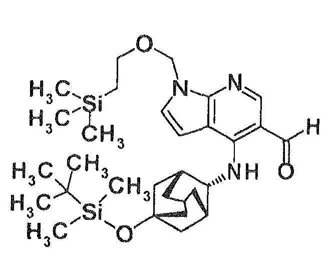 | 20-1 | 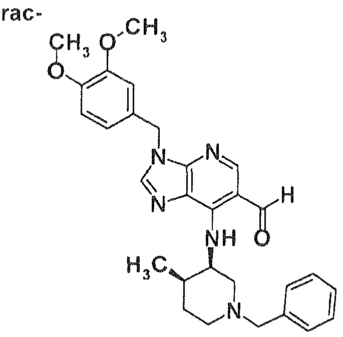 |
| 20-2 | 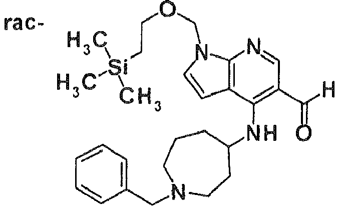 | 20-3 | 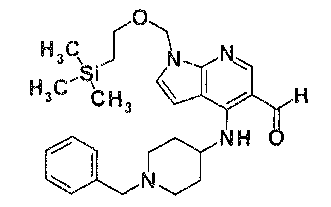 |
| 21 | 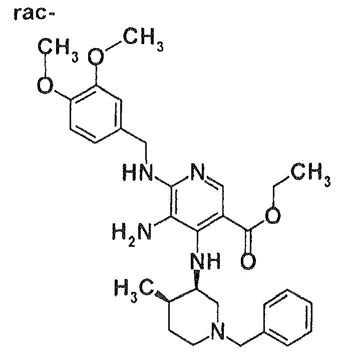 | 22 | 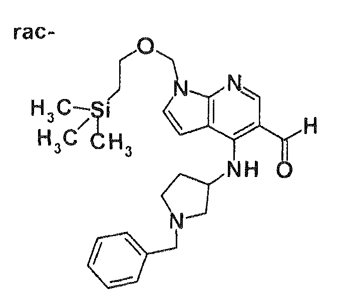 |
| 22-1 | 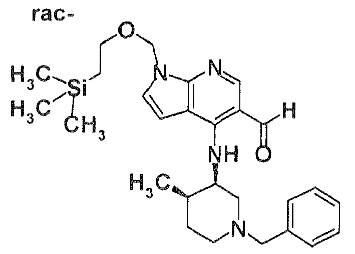 | 22-2 | 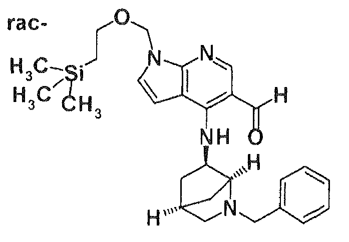 |

**[Table 12]**

| Pr | Strucure | Pr | Strucure |
|---|---|---|---|
| 22-3 | 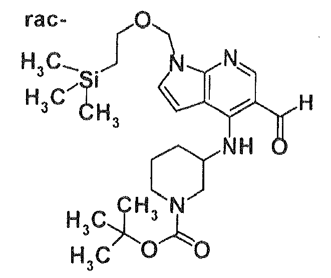 | 22-4 | 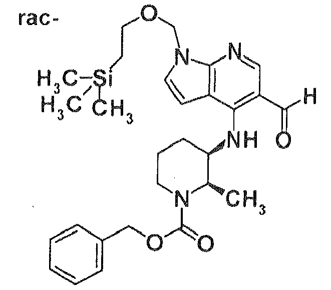 |
| 23 | 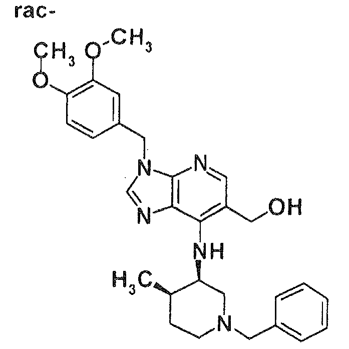 | 23-1 | 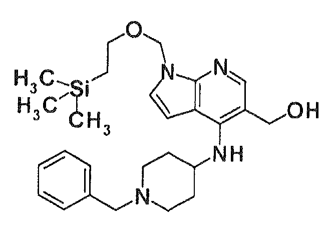 |
| 23-2 | 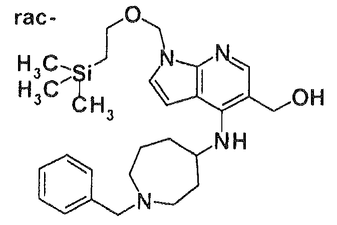 | 24 | 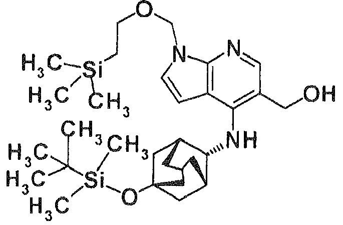 |
| 25 | 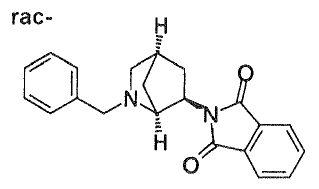 | 26 | 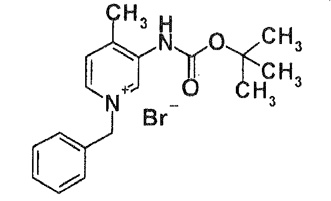 |

**[Table 13]**

| Pr | Strucure | Pr | Strucure |
|---|---|---|---|
| 27 | 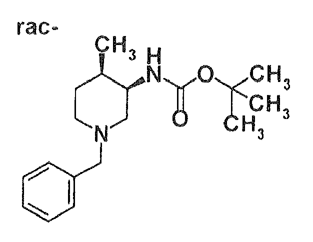 | 28 | 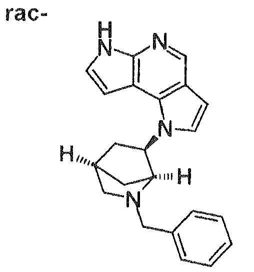 |
| 28-1 | 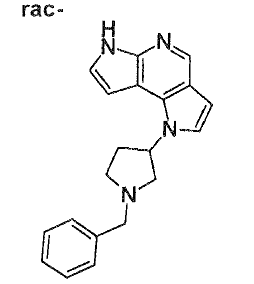 | 28-2 | 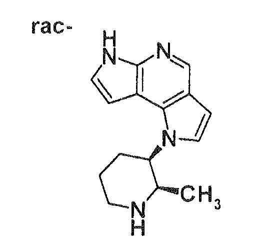 |
| 29 | 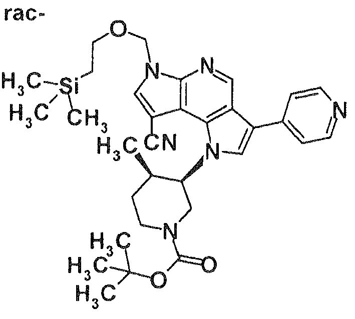 | 1-4 | 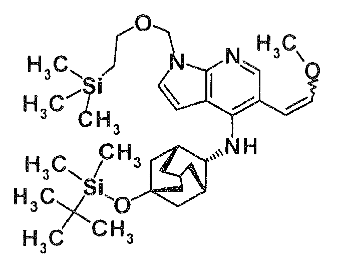 |

**[Table 14]**

| Pr | Data |
|---|---|
| 1 | ESI+ : 507 [M+H]+ |
| 1-1 | ESI+ : 528 [M+H]+ |
| 1-2 | ESI+ : 507 [M+H]+ |
| 1-3 | ESI+ : 493 [M+H]+ |
| 1-4 | ESI+ : 584 [M+H]+ |
| 2 | ESI+ : 371 [M+H]+ |
| 3 | ESI+ : 475 [M+H]+ |
| 3-1 | ESI+ : 461 [M+H]+ |
| 3-2 | ESI+ : 496 [M+H]+ |
| 3-3 | ESI+ : 475 [M+H)+ |
| 4 | ESI+ : 544 [M+H]+ |
| 5 | ESI+ : 355 [M+H]+ |
| 5-1 | ESI+ : 330 [M+Na]+ |
| 6 | ESI+ : 345 [M+H]+ |
| 6a | ESI+ : 345 [M+H]+ |
| 6b | ESI+ : 345 [M+H]+ |
| 6-1 | ESI+ : 331 [M+H]+ |
| 6-2 | ESI+ : 345 [M+H]+ |
| 6-3 | ESI+ : 241 [M+H]+ |
| 6-4 | ESI+ : 357 [M+H]+ |
| 7 | ESI+ : 485 [M+H]+ |
| 8 | ESI+ : 455 (M+H)+ |
| 8-1 | ESI+ : 480 [M+H]+ |
| 9 | ESI+ : 333, 335 [M+H]+ |
| 9-1 | ESI+ : 280 [M+H]+ |
| 9-1a | ESI+ : 280 [M+H]+ |
| 9-1b | ESI+ : 280 [M+H]+ |
| 10 | ESI+ : 333, 335 [M+H]+ |
| 10-1 | ESI+ : 358, 360 [M+H]+ |
| 11 | ESI+ : 227 [M+H]+ |
| 11-1 | ESI+ : 256 [M+H]+ |
| 11-2 | ESI+ : 255 [M+H]+ |
| 11-3 | ESI+ : 241 [M+H]+ |
| 11-4 | ESI+ : 255 [M+H]+ |
| 11-5 | ESI+ : 255 [M+H]+ |
| 11-6 | ESI+ : 255 [M+H]+ |
| 11-7 | ESI+ : 253 [M+H]+ |
| 11-8 | ESI+ : 385 [M+H]+ |
| 12 | ESI+ : 346 [M+H]+ |
| 13 | ESI+ : 203 [M+H]+ |
| 14 | ESI+ : 600 [M+H]+ |
| 15 | ESI+ : 448 [M+H]+ |

**[Table 15]**

| Pr | Data |
|---|---|
| 15-1 | ESI+ : 486 [M+H]+ |
| 15-2 | ESI+ : 476 [M+H]+ |
| 15-3 | ESI+ : 509 [M+H]+ |
| 15-4 | ESI+ : 474 [M+H]+ |
| 15-5 | ESI+ : 523 [M+H]+ |
| 15-6 | ESI+ : 494 [M+Na]+ |
| 15-7 | ESI+ : 520 [M+H]+ |
| 16 | ESI+ : 564 [M+H]+ |
| 17 | ESI+ : 563, 565 [M+H]+ |
| 18 | ESI+ : 533, 535 [M+H]+ |
| 18-1 | ESI+ : 580,582[M+Na]+ |
| 18-2 | ESI+ : 610, 612 [M+Na]+ |
| 19 | ESI+ : 510 [M+H]+ |
| 20 | ESI+ : 556 [M+H]+ |
| 20-1 | ESI+ : 500 [M+H]+ |
| 20-2 | ESI+ : 479 [M+H]+ |
| 20-3 | ESI+ : 465 [M+H]+ |
| 21 | ESI+ : 534 [M+H]+ |
| 22 | ESI+ : 451 [M+H]+ |
| 22-1 | ESI+ : 479 [M+H]+ |
| 22-2 | ESI+ : 477 [M+H]+ |
| 22-3 | ESI+ : 497 [M+Na]+ |
| 22-4 | ESI+ : 523 [M+H]+ |
| 23 | ESI+ : 502 [M+H]+ |
| 23-1 | ESI+ : 467 [M+H]+ |
| 23-2 | ESI+ : 481 [M+H]+ |
| 24 | ESI+ : 558 (M+H]+ |
| 25 | ESI+ : 333 [M+H]+ |
| 26 | ESI+ : 299 [M]+ |
| 27 | ESI+ : 305 [M+H]+ |
| 28 | ESI+ : 343 [M+H]+ |
| 28-1 | ESI+ : 317 [M+H]+ |
| 28-2 | ESI+ : 255 [M+H]+ |
| 29 | ESI+ : 587 [M+H]+ |

### Example 1

To (methoxymethyl)triphenylphosphonium chloride (315 mg) was added THF (1.0 mL), and sodium bis(trimethylsilyl)amide (1.07 M solution in THF, 1.01 mL) was added dropwise thereto under ice-cooling, followed by stirring for 40 minutes. To this reaction mixture was added dropwise a solution of 4-{[(1R,2R,3S,5s)-S-{[tert-butyl(dimethyl)silyl]oxy}adamantan-2-yl]amino}-1-{[2-(trimethylsilyl)ethoxy]methyl}-1H-pyrrolo[2,3-b]pyridine-5-carbaldehyde (465 mg) in THF (5.0 mL), followed by stirring at room temperature for 2 hours. To the reaction mixture was added a saturated aqueous ammonium chloride solution, and then the mixture was extracted with EtOAc and washed with brine. The organic layer was dried over Na₂SO₄ and then filtered, and the filtrate was concentrated under reduced pressure. The residue was purified by silica gel column chromatography (EtOAc/Hx = 10/90 to 20/80) to obtain a crude product of N-[(1R,2R,3S,5S)-5-{([tert-butyl(dimethyl)silyl]oxy}adamantan-2-yl]-5-(2-methoxyvinyl)-1-{[2-(trimethylsilyl)ethoxy]methyl}-1H-pyrrolo[2,3-b]pyridin-4-amine.
To this crude product (445 mg) were added MeOH (5.0 mL) and acetyl chloride (0.16 mL), followed by stirring at 80°C for 1 hour. To the reaction mixture was added water (0.5 mL), followed by stirring at 80°C for 30 minutes. After completion of the reaction, to the reaction mixture was added a saturated aqueous sodium hydrogen carbonate solution, and then the mixture was extracted with CHCl₃ and washed with brine. The organic layer was dried over Na₂SO₄ and then filtered, and the filtrate was concentrated under reduced pressure. To the residue were added CH₂Cl₂ (3.0 mL) and TFA (3.0 mL), followed by stirring at room temperature for 2 hours. The reaction mixture was concentrated under reduced pressure, to the residue were added CH₂Cl₂ (2.0 mL), MeOH (1.0 mL), ethylenediamine (0.2 mL), and a 1 M aqueous sodium hydroxide solution (3.0 mL), followed by stirring at room temperature for 1 hour. To the reaction mixture was added water, and then the mixture was extracted with CHCl₃. The organic layer was dried over Na₂SO₄ and then filtered, and the filtrate was concentrated under reduced pressure. The residue was washed with MeOH and diisopropyl ether to obtain (1S,3R,4R,5s)-4-(dipyrrolo[2,3-b:2',3'-d]pyridin-1(6H)-yl)adamantan-1-ol(145 mg) as a white solid.

### Example 2

1-Pyrrolidin-3-yl-1,6-dihydrodipyrrolo[2,3-b:2',3'-d]pyridine (69.2 mg), cyanoacetic acid (52.0 mg), 1-hydroxybenzotriazole (62.0 mg), N-(3-dimethylaminopropyl)-N'-ethylcarbodiimide hydrochloride (87.9 mg), Et3N (0.0853 mL), and DMF (2.5 mL) were added and stirred at 50°C for 30 minutes. After completion of the reaction, the reaction mixture was concentrated under reduced pressure and the residue was purified by silica gel column chromatography (MeOH/CHCl₃ = 0/100 to 10/90). Further, the obtained mixture was purified by basic silica gel column chromatography (MeOH/CHCl₃ = 0/100 to 4/96). The obtained solid was washed with diisopropyl ether and dried under reduced pressure to obtain 3-(3-dipyrrolo[2,3-b:2',3'-d]pyridin-1(6H)-ylpyrrolidin-1-yl)-3-oxopropanenitrile (65.4 mg).

In the same manner as the method of Example 2, the compounds of Examples 2-1 to 2-86 shown in Tables below were prepared.

### Example 3

In the same manner as the method of Preparation Example 19, the compound of Example 3 shown in Tables below was prepared.

### Example 4

In the same manner as the method of Preparation Example 15, the compounds of Examples 4 and 4-1 to 4-2 shown in Tables below were prepared.

### Example 5

To a solution of rac-1-[(3R,4R)-4-methylpiperidin-3-yl]-1,6-dihydrodipyrrolo[2,3-b:2',3'-d]pyridine (50 mg) and 4-nitrophenyl(cyanomethyl)carbamate (87 mg) in DMF (1.0 mL) was added Et₃N (0.082 mL), followed by stirring at 120°C for 30 minutes under microwave irradiation. After completion of the reaction, to the reaction mixture was added water, and the mixture was extracted with CHCl₃, and washed with a 1 N aqueous sodium hydroxide solution and brine. The organic layer was dried over MgSO₄ and then filtered, and the filtrate was concentrated under reduced pressure. The residue was purified by silica gel column chromatography (MeOH/CHCl₃ = 5/95), and the obtained solid was washed with isopropanol-diisopropyl ether and then collected by filtration to obtain rac-(3R,4R)-N-(cyanomethyl)-3-dipyrrolo[2,3 -b:2',3'-d]pyridin-1(6H)-yl-4-methylpiperidine-1-carboxamide (25 mg) as a white powder.

In the same manner as the method of Example 5, the compounds of Examples 5-1 to 5-11 shown in Tables below were prepared.

### Example 6

To a mixture of 1.00 mL of a solution of rac-1-[(3R,4R)-4-methylpiperidin-3-yl]-1,6-dihydrodipyrrolo[2,3-b:2',3'-d]pyridine (130 mg) in DMF (20 mL) and phenylmethanesulfonyl chloride (0.040 mmol) was added pyridine (0.5 mL), followed by stirring at room temperature overnight. To the reaction mixture were added MP-Carbonate (Biotage 800269) (50 mg) and PS-NCO (Biotage 800262) (50 mg), followed by stirring for 2 hours. The reaction mixture was filtered and the filtrate was concentrated. This concentrate was purified through collection by separation by means of liquid chromatography (LC) (aq. HCOOH/MeOH) equipped with an MS trigger (LC condition for collection by separation: SunFire column 5 µm 19*100 mm, MeOH/0.1% aq. HCOOH = 10/90 (0 min) - 10/90 (1 Min) - 95/5 (8 min) - 95/5 (12 min) - 10/90 (13 min)) to obtain rac-1-[(3R,4R)-1-(benzylsulfonyl)-4-methylpiperidin-3-yl]-1,6-dihydrodipyrrolo[2,3-b:2',3'-d]pyridine (3.2 mg).

In the same manner as the method of Example 6, the compounds of Examples 6-1 to 6-9 shown in Tables below were prepared.

### Example 7

To rac-1-[(3R,4R)-4-methylpiperidin-3-yl]-1,6-dihydropyrrolo[2,3-b:2',3'-d]pyridine (67 mg) was added dichloroethane (2 mL) at room temperature, and then diisopropylethylamine (95 µL) and trifluoroacetic acid anhydride (56 µL) were added thereto under ice-cooling. After stirring at room temperature for 1 hour, to the reaction mixture was added a saturated aqueous ammonium chloride solution, and the mixture was extracted with CH₂Cl₂. The organic layer was dried over Na₂SO₄ and then filtered, and the filtrate was concentrated under reduced pressure. The obtained residue was purified by silica gel column chromatography (CHCl₃/MeOH = 100/0 to 92/8) to obtain rac-1-[(3R,4R)-4-methyl-1-(trifluoroacetyl)piperidin-3-yl]-1,6-dihydrodipyrrolo[2,3-b:2',3'-d]pyridine (56 mg) as a white solid.

### Example 8

In the same manner as the method of Preparation Example 6, the compounds of Examples 8 and 8-1 to 8-8 shown in Tables below were prepared.

### Example 9

To a solution of rac-1-[(3R,4R)-4-methylpiperidin-3-yl]-1,6-dihydrodipyrrolo[2,3-b:2',3'-d]pyridine (100 mg) in DMF (4 mL) were added Et₃N (200 µl) and 2,6-dimethylmorpholine-4-sulfonyl chloride (100 mg), followed by stirring at room temperature for 2 hours. To the reaction mixture was added water, followed by extraction with EtOAc. The extract was washed with brine, dried over MgSO₄ and then filtered, and the filtrate was concentrated under reduced pressure. The obtained residue was purified by silica gel column chromatography (CHCl₃/MeOH = 100/0 to 95/5) to obtain rac-1-{(3R,4R)-1-[(2,6-dimethylmorpholine-4-yl)sulfonyl]-4-methylpiperidin-3-yl}-1,6-dihydrodipyrrolo[2,3-b:2',3'-d]pyridine (131 mg) as a white solid.

In the same manner as the method of Example 9, the compounds of Examples 9-1 to 9-4 shown in Tables below were prepared.

For the Example Compounds, the structures are shown in Tables 16 to 27 and Tables 43 to 47, and the physicochemical data are shown in Tables 28 to 32 and Tables 48 to 52.

**[Table 16]**

| Ex | Structure | Ex | Structure |
|---|---|---|---|
| 1 | 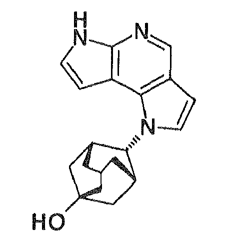 | 2 | 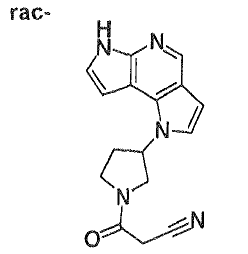 |
| 2-1 | 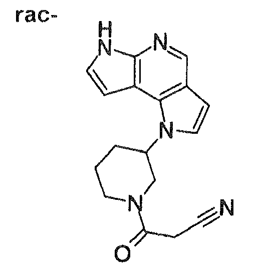 | 2-2 | 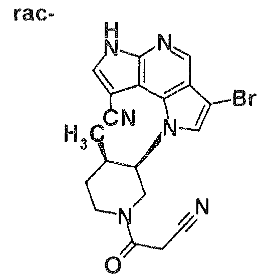 |
| 2-3 | 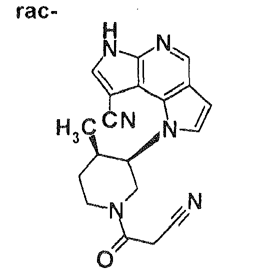 | 2-4 | 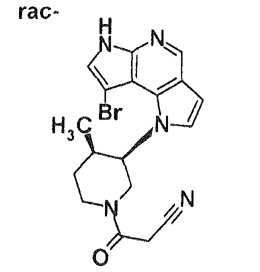 |
| 2-5 | 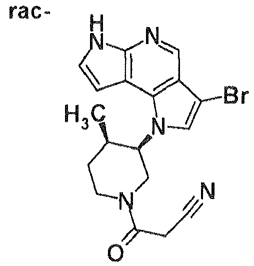 | 2-6 | 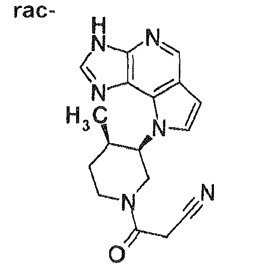 |

**[Table 17]**

| Ex | Structure | Ex | Structure |
|---|---|---|---|
| 2-7 | 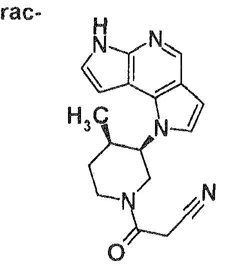 | 2-8 | 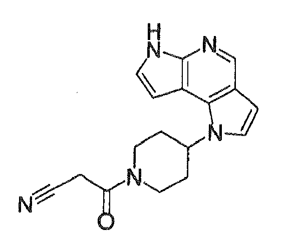 |
| 2-9 | 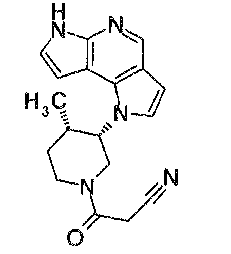 | 2-10 | 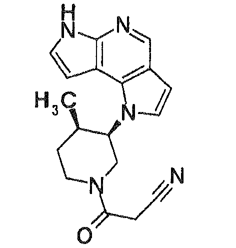 |
| 2-11 | 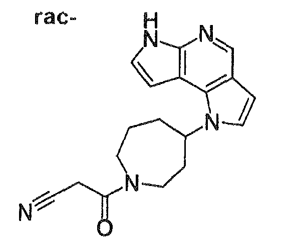 | 2-12 | 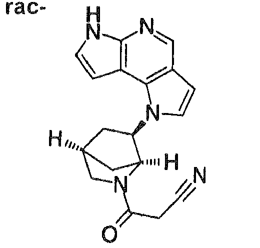 |
| 2-13 | 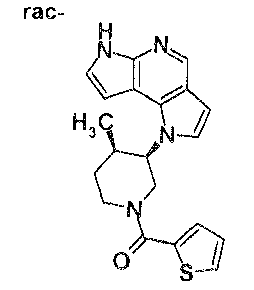 | 2-14 | 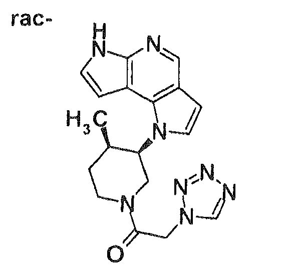 |

**[Table 18]**

| Ex | Structure | Ex | Structure |
|---|---|---|---|
| 2-15 | 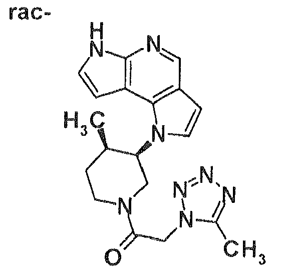 | 2-16 | 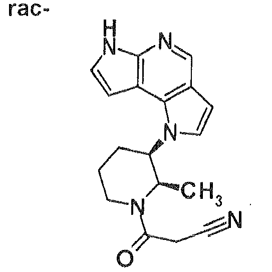 |
| 2-17 | 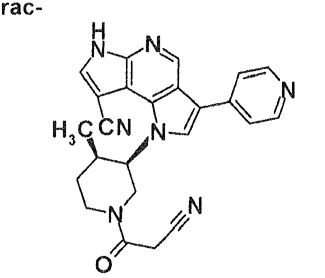 | 2-18 | 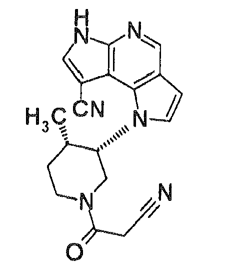 |
| 2-19 | 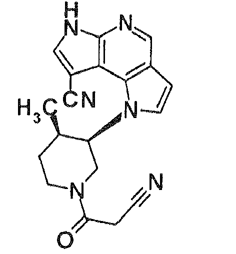 | 2-20 | 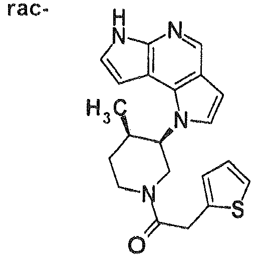 |
| 2-21 | 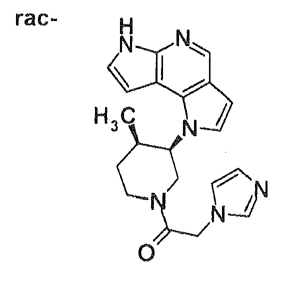 | 2-22 | 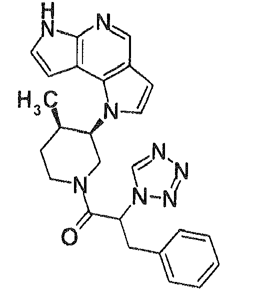 |
| | | | diastereomeric mixture |

**[Table 19]**

| Ex | Structure | Ex | Structure |
|---|---|---|---|
| 2-23 | 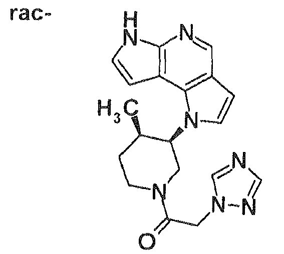 | 2-24 | 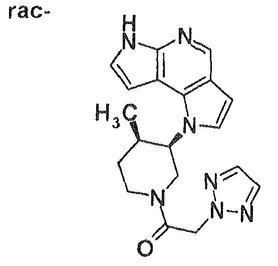 |
| 2-25 | 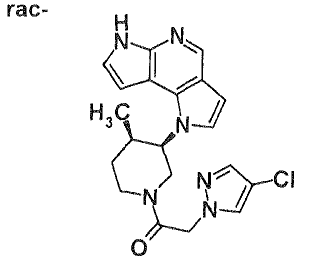 | 2-26 | 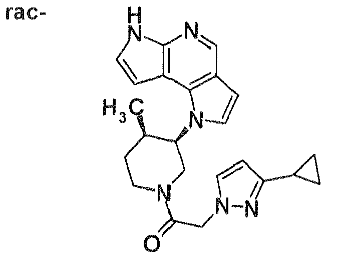 |
| 2-27 | 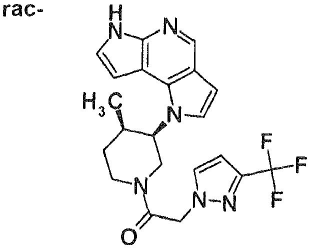 | 2-28 | 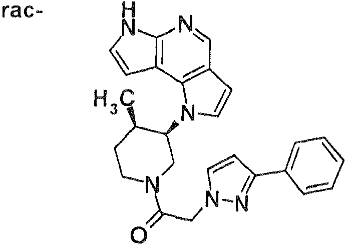 |
| 2-29 | 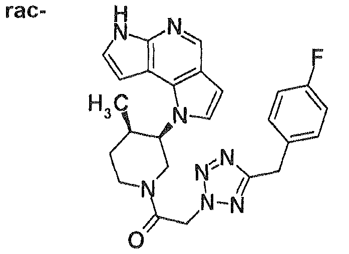 | 2-30 | 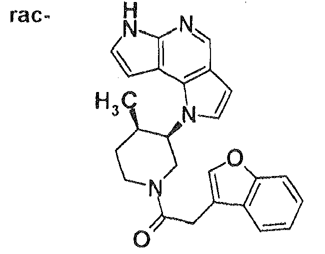 |

**[Table 20]**

| Ex | Structure | Ex | Structure |
|---|---|---|---|
| 2-31 | 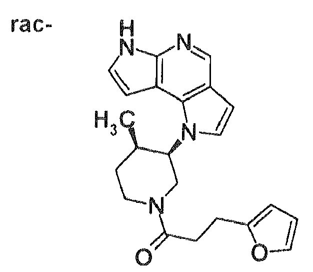 | 2-32 | 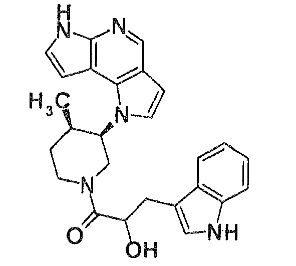 |
| | | | diastereomeric mixture |
| 2-33 | 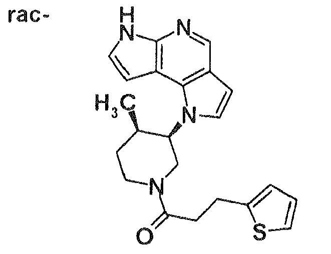 | 2-34 | 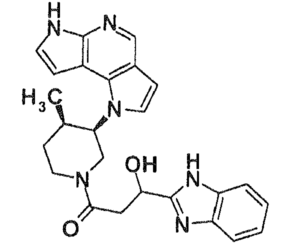 |
| | | | diastereomeric mixture |
| 2-35 | 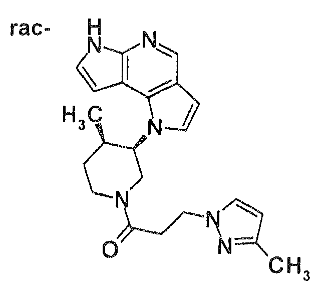 | 2-36 | 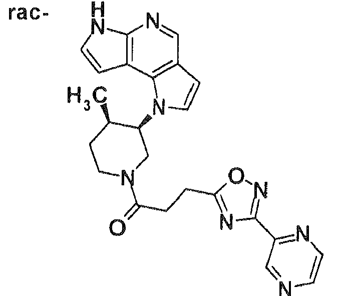 |
| 2-37 | 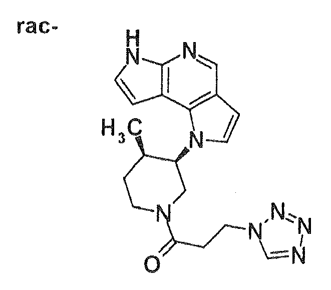 | 2-38 | 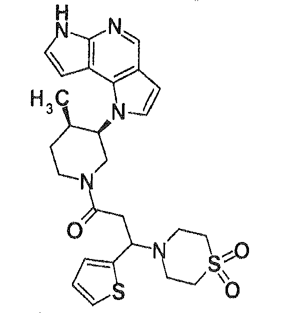 |
| | | | diastereomeric mixture |

**[Table 21]**

| Ex | Structure | Ex | Structure |
|---|---|---|---|
| 2-39 | 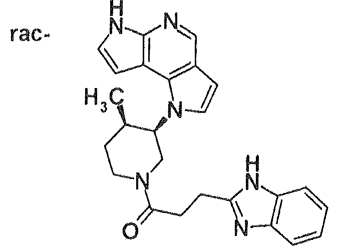 | 2-40 | 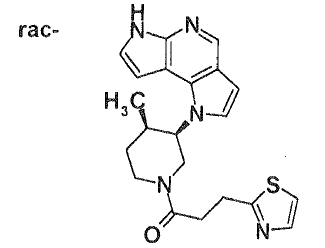 |
| 2-41 | 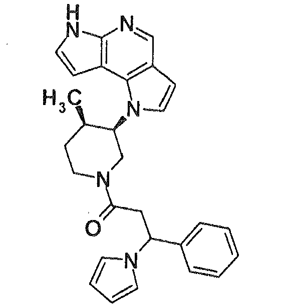 | 2-42 | 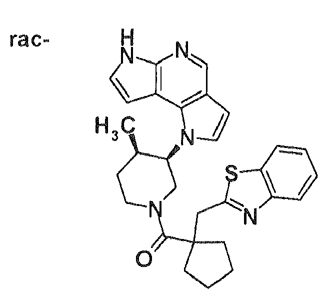 |
| | diastereomeric mixture | | |
| 2-43 | 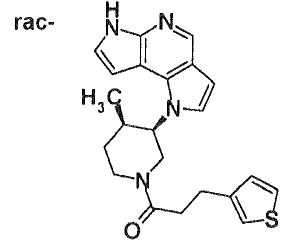 | 2-44 | 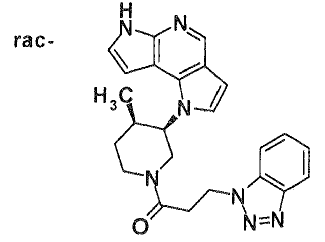 |
| 2-45 | 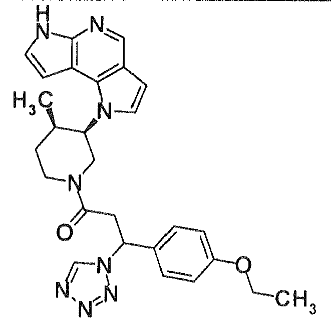 | 2-46 | 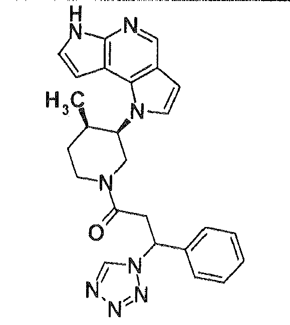 |
| | diastereomeric mixture | | diastereomeric mixture |

**[Table 22]**

| Ex | Structure | Ex | Structure |
|---|---|---|---|
| 2-47 | 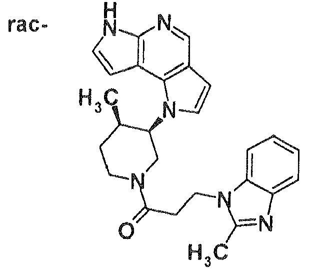 | 2-48 | 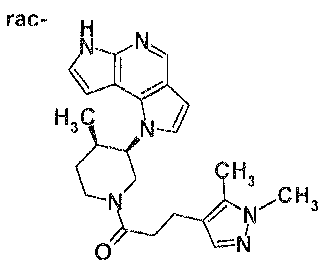 |
| 2-49 | 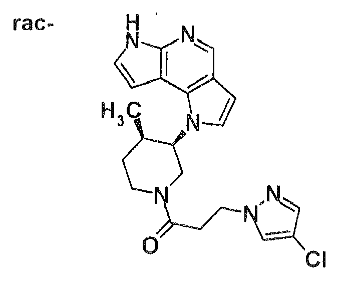 | 2-50 | 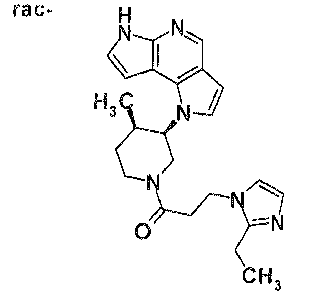 |
| 2-51 | 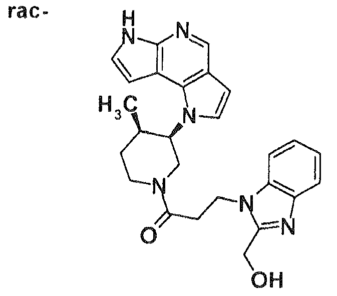 | 2-52 | 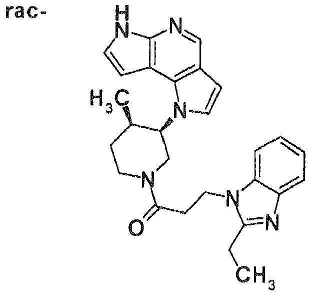 |
| 2-53 | 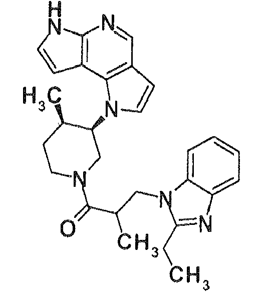 | 2-54 | 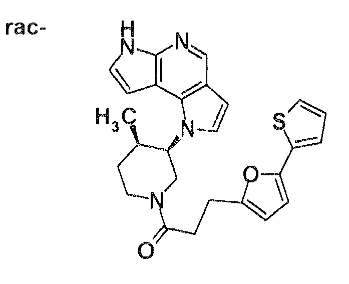 |
| | diastereomeric mixture | | |

**[Table 23]**

| Ex | Structure | Ex | Structure |
|---|---|---|---|
| 2-55 | 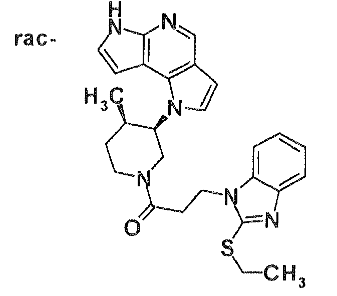 | 2-56 | 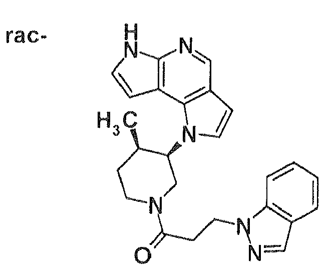 |
| 2-57 | 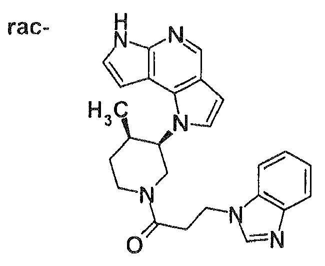 | 2-58 | 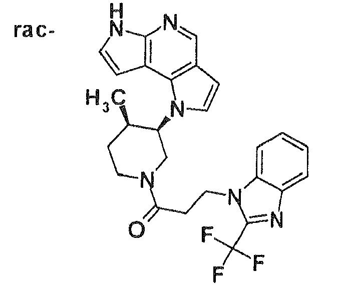 |
| 2-59 | 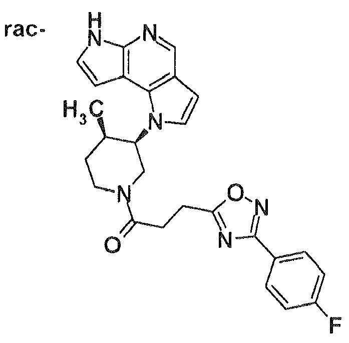 | 2-60 | 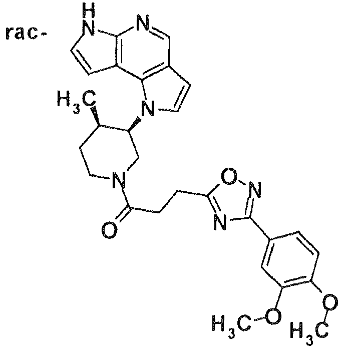 |
| 2-61 | 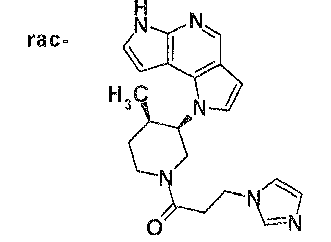 | 2-62 | 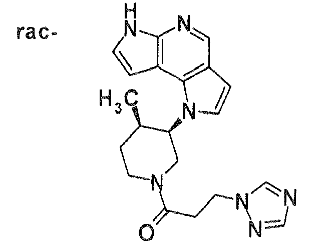 |

**[Table 24]**

| Ex | Structure | Ex | Structure |
|---|---|---|---|
| 2-63 | 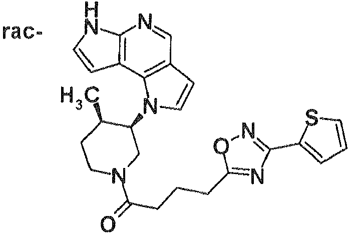 | 2-64 | 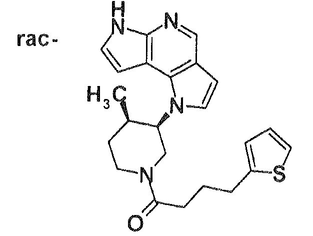 |
| 2-65 | 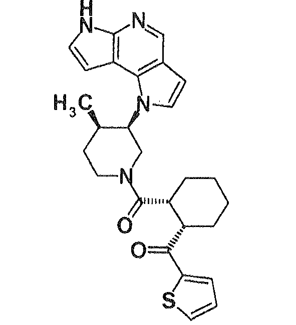 | 2-66 | 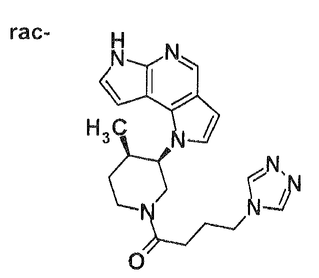 |
| | diastereomeric mixture | | |
| 2-67 | 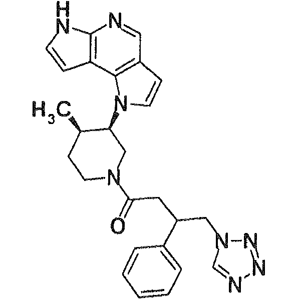 | 2-68 | 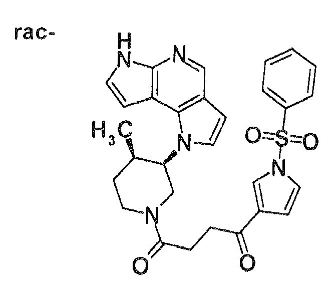 |
| | diastereomeric mixture | | |
| 2-69 | 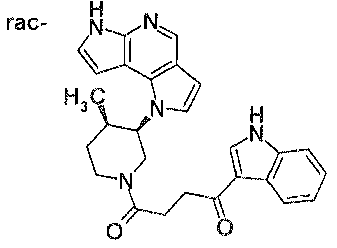 | 2-70 | 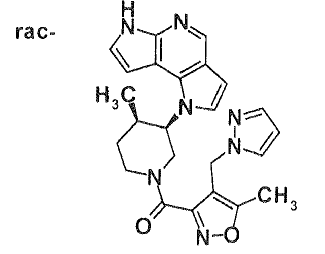 |

**[Table 25]**

| Ex | Structure | Ex | Structure |
|---|---|---|---|
| 2-71 | 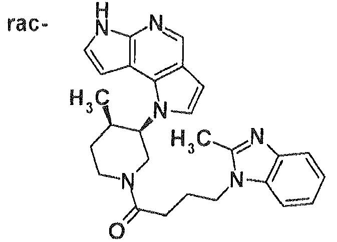 | 3 | 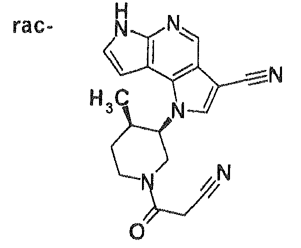 |
| 4 | 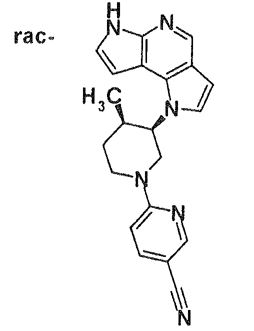 | 4-1 | 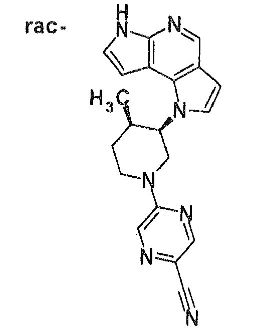 |
| 4-2 | 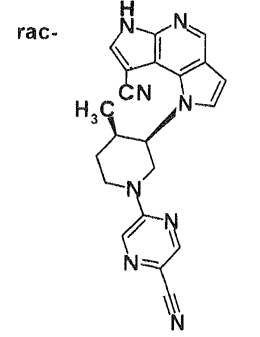 | 5 | 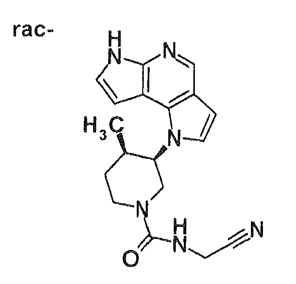 |
| 5-1 | 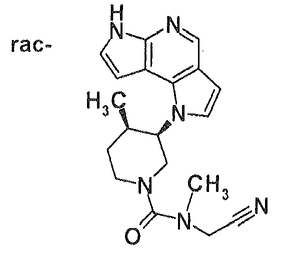 | 5-2 | 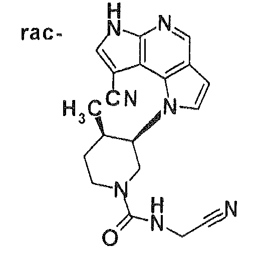 |

**[Table 26]**

| Ex | Structure | Ex | Structure |
|---|---|---|---|
| 5-3 | 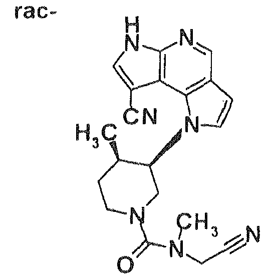 | 5-4 | 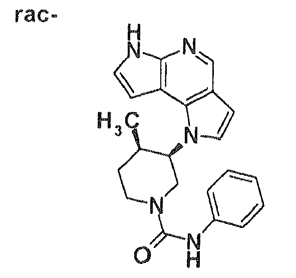 |
| 5-5 | 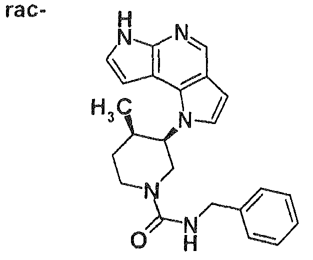 | 5-6 | 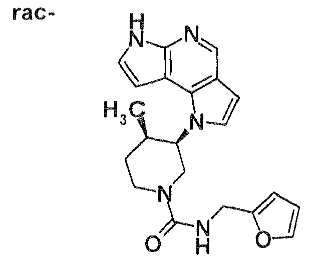 |
| 5-7 | 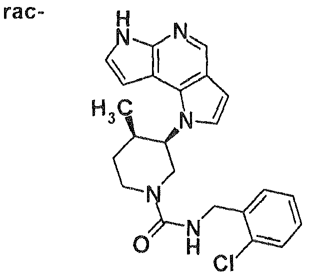 | 5-8 | 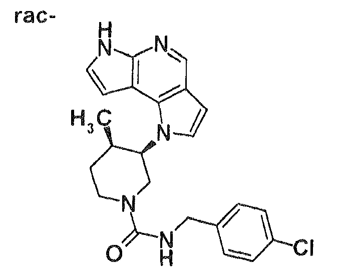 |
| 5-9 | 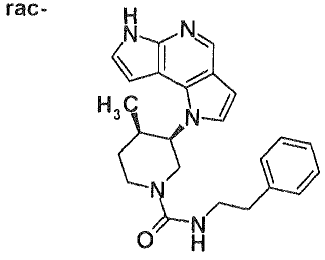 | 5-10 | 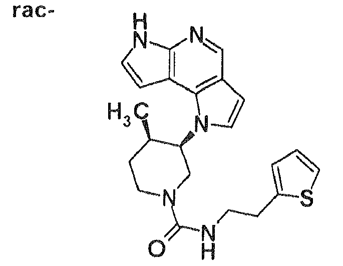 |

**[Table 27]**

| Ex | Structure | Ex | Structure |
|---|---|---|---|
| 6 | 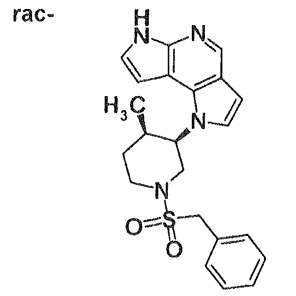 | 6-1 | 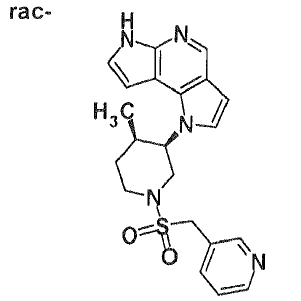 |
| 6-2 | 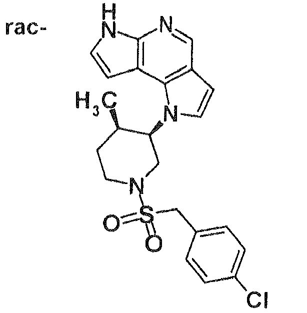 | 6-3 | 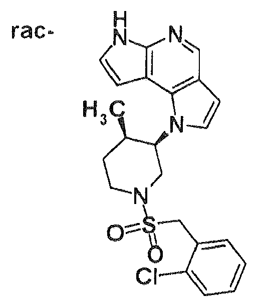 |
| 6-4 | 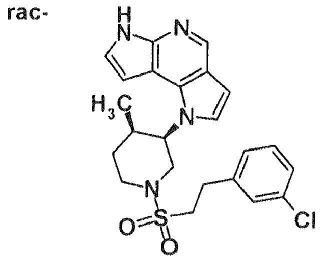 | 6-5 | 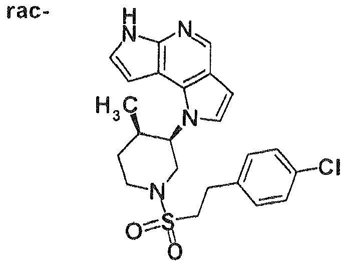 |
| 6-6 | 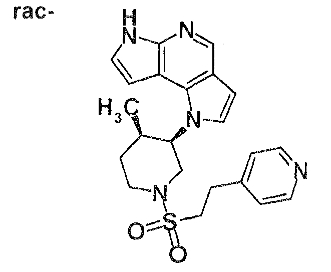 | | |

**[Table 28]**

| Ex | DATA |
|---|---|
| 1 | NMR-DMSO-d₆: 1.52-1.60 (2H, m), 1.64-1.71 (2H, m), 1.77-1.92 (4H, m), 1.98-2.07 (2H, m), 21.17-2.24 (1H, m), 2.69-2.77 (2H, m), 4.51-4.67 (2H, m), 6.47-6.51 (1H, m), 6.61-6.65 (1H, m), 7.32-7.36 (1H, m), 7.52-7.56 (1H, m), 8.47 (1H, s), 11.64 (1H, brs) ESI+ : 308 [M+H]+ |
| 2 | NMR-DMSO-d₆: 2.27-2.59 (2H, m), 3.50-3.80 (3H, m), 3.91-4.10 (3H, m), 5.44-5.58 (1H, m), 6.65-6.67 (1H, m), 6.77-6.83 (1H, m), 7.27-7.38 (2H, m), 8.46 (1H, s), 11.67 (1H, s) ESI+ : 316 [M+Na]+ |
| 2-1 | NMR-DMSO-d₆: 1.66-1.91 (2H, m), 1.99-2.20 (2H, m), 2.71-3.07 (1H, m), 3.12-3.54 (1H, m), 3.71-3.92 (1H, m), 4.00-4.15 (2H, m), 4.41-4.87 (2H, m), 6.67-6.69 (1H, m), 6.76-6.93 (1H, m), 7.36-7.42 (2H, m), 8.467-8.47 (1H, m), 11.68-11.69 (1H, m) ESI+ : 308 [M+H]+ |
| 2-2 | NMR-DMSO-d₆ : 0.60-0.86 (3H, m), 1.36-2.23 (2H, m), 2.88-4.40 (7H, m), 5.28-5.80 (1H, m), 6.62-7.67 (1H, m), 8.43-8.54 (2H, m), 13.07 (1H, brs) ESI- : 423, 425 [M-H]- |
| 2-3 | NMR-DMSO-d₆ : 0.60-0.78 (3H, m), 1.53-1.75 (1H, m), 1.92-2.14 (1H, m), 3.25-4.36 (7H, m), 5.28-5.43 (1H, m), 6.77-6.82 (1H, m), 7.37-7.45 (1H, m), 8.35-8.37 (1H, m), 8.63-8.66 (1H, m), 12.90 (1H, brs) ESI+ : 347 [M+H]+ |
| 2-4 | NMR-DMSO-d₆ : 0.60-0.75 (3H, m), 1.57-1.71 (1H, m), 1.86-2.01 (1H, m), 3.25-4.29 (7H, m), 5.71-5.81 (1H, m), 6.71-6.78 (1H, m), 7.23-7.36 (1H, m), 7.58 (1H, s), 8.51-8.55 (1H, m), 12.17 (1H, brs) ESI+ : 400, 402 [M+H]+ |
| 2-5 | NMR-DMSO-d6 : 0.55-0.75 (3H, m), 1.58-1.75 (1H, m), 1.81-1.96 (1H, m), 3.20-4.38 (7H, m), 4.97-5.10 (1H, m), 6.75-6.86 (1H, m), 7.39-7.51 (2H, m), 8.30-8.38 (1H, m), 11.85 (1H, brs) ESI+ : 400,402 [M+H]+ |
| 2-6 | NMR-DMSO-d₆ : 0.62 (3H, d, J = 5.6 Hz), 1.65-1.78 (2H, m), 2.42-2.48 (1H, m), 3.34-3.37 (1H, m), 3.48-3.85 (2H, m), 3.98-4.17 (1H, m), 4.29-4.36 (2H, m), 5.62-5.70 (1H, m), 6.70-6.76 (1H, m), 7.25-7.39 (1H, m), 8.22-8.24 (1H, m), 8.56-8.59 (1H, m), 13.05 (1H, s) ESI+: 323 [M+H]+ |
| 2-7 | NMR-DMSO-d₆ : 0.57-0.71 (3H, m), 1.59-1.76 (1H, m), 1.81-1.93 (1H, m), 3.20-4.36 (7H, m), 4.94-5.10 (1H, m), 6.62-6.69 (1H, m), 6.74-6.79 (1H, m), 7.17-7.31 (1H, m), 7.32-7.36 (1H, m), 8.44-8.49 (1H, m), 11.65 (1H, brs) ESI+ : 322 [M+H]+ |
| 2-8 | NMR-DMSO-d₆ : 1.82-1.92 (1H, m), 1.98-2.11 (3H, m), 2.97-3.05 (1H, m), 3.40-3.51 (1H, m), 4.12 (2H, s), 4.51-4.58 (1H, m), 4.87-4.96 (1H, m), 6.63 (1H, d, J = 3.6 Hz), 6.83 (1H, dd, J = 2.0, 3.6 Hz), 7.34-7.36 (3H, m), 8.44 (1H, s), 11.64 (1H, s) ESI+ : 308 [M+H]+ |

**[Table 29]**

| Ex | DATA |
|---|---|
| 2-9 | NMR-DMSO-d₆ : 0.57-0.71 (3H, m), 1.59-1.76 (1H, m), 1.81-1.93 (1H, m), 3.20-4.36 (7H, m), 4.94-5.10 (1H, m), 6.62-6.69 (1H, m), 6.74-6.79 (1H, m), 7.17-7.31 (1H, m), 7.32-7.36 (1H, m), 8.44-8.49 (1H, m), 11.65 (1H, brs) ESI+ : 322 [M+H]+ mp.292-300°C(decomposition) [α]_{D}²⁵ -52.3 (c 0.723, 0,1M HCl) |
| 2-10 | NMR-DMSO-d₆ : 0.57-0.71 (3H, m), 1.59-1.76 (1H, m), 1.81-1.93 (1H, m), 3.20-4.36 (7H, m), 4.94-5.10 (1H, m), 6.62-6.69 (1H, m), 6.74-6.79 (1H, m), 7.17-7.31 (1H, m), 7.32-7.36 (1H, m), 8.44-8.49 (1H, m), 11.65 (1H, brs) ESI+ : 322 [M+H]+ mp.295-300°C(decomposition) [α]_{D}²⁵ +50.1 (c 0.733, 0.1M HCl) |
| 2-11 | NMR-DMSO-d₆ : 1.90-2.11 (5H, m), 2.17-2.24 (1H, m), 3.42-3.72 (4H, m), 4.02-4.21 (2H, m), 4.75-4.82 (1H, m), 6.61-6.63 (1H, m), 6.76-6.79 (1H, m), 7.28-7.39 (2H, m), 8.43 (1H, s), 11.62 (1H, s) ESI+ : 322 [M+H]+ |
| 2-12 | NMR-DMSO-d₆ : 1.64-1.76 (1H, m), 1.89-2.17 (2H, m), 2.26-2.46 (1H, m), 2.75-2.83 (1H, m), 3.13-3.22 (1H, m), 3.28-343 (1H, m), 3.87-4.11 (2H, m), 4.52-4.61 (1H, m), 4.89-5.10 (1H, m), 6.63-6.65 (1H, m), 6.85-7.00 (1H, m), 7.34-7.45 (2H, m), 8.44-8.45 (1H, m), 11.61-11.65 (1H, m) ESI+ : 320 [M+H]+ |
| 2-13 | NMR-DMSO-d₆ : 0.67 (3H, d, J = 6.5 Hz), 1.70-1.81 (1H, m), 1.87-2.00 (1H, m), 2.42-2.60 (1H, m), 3.54-3.70 (1H, m), 3.95-4.10 (2H, m), 4.29 (1H, dd, J = 3.6, 13.4 Hz), 5.04-5.12 (1H, m), 6.66 (1H, d, J= 3.3 Hz), 6.81 (1H, dd, J= 1.8, 3.3 Hz), 7.05 (1H, brs), 7.20-7.43 (3H, m), 7.66-7.76 (1H, m), 8.46 (1H, s), 11.66 (1H, brs) ESI+ : 365 [M+H]+ |
| 2-14 | NMR-DMSO-d₆ : 0.61-0.75 (3H, m), 1.63-2.02 (2H, m), 2.43-2.57 (1H, m), 3.48-4.06 (33H, m), 4.10-4.25 (1H, m), 4.97-5.25 (1H, m), 5.64-6.00 (2H, m), 6.62-6.83 (2H, m), 7.25-7.43 (2H, m), 8.43-8.53 (1H, m), 9.26-9.34 (1H, m), 11.60-11.73 (1H, m) ESI+ : 365 [M+H]+ |
| 2-15 | NMR-DMSO-d₆ : 0.60-0.75 (3H, m), 1.65-1.98 (2H, m), 2.35-2.60 (4H, m), 3.48-4.30 (4H, m), 4.98-5.95 (3H, m), 6.61-6.85 (2H, m), 7.24-7.40 (2H, m), 8.42-8.53 (1H, m), 11.59-11.73 (1H, m) ESI+ : 379 [M+H]+ |
| 2-16 | NMR-DMSO-d₆ : 0.70-0.85 (3H, m), 1.01-1.09 (1H, m), 1.63-2.12 (3H, m), 3.12-3.64 (2H, m), 3.83-4.25 (2H, m), 4.32-5.33 (2H, m), 6.60-6.73 (2H, m), 7.30-7.45 (2H, m),8.47 (1H, s), 11.59-11.77 (1H, m) ESI+ : 322 [M+H]+ |
| 2-17 | NMR-DMSO-d₆ : 0.71-0.86 (3H, m), 0.93-4.40 (9H, m), 5.32-5.81 (1H, m), 7.76-7.82 (2H, m), 7.93-8.03 (1H, m), 8.47 (1H, s), 8.58-8.65 (2H, m), 9.04-9.09 (1H, m), 13.07 (1H, brs) ESI- : 422 [M-H]- |

**[Table 30]**

| Ex | DATA |
|---|---|
| 2-18 | NMR-DMSO-d₆ : 0.60-0.78 (3H, m), 1.53-1.75 (1H, m), 1.92-2.14 (1H, m), 3.25-4.36 (7H, m), 5.28-5.43 (1H, m), 6.77-6.82 (1H, m), 7.37-7.45 (1H, m), 8.35-8.37 (1H, m), 8.63-8.66 (1H, m), 12.90 (1H, brs) ESI+ : 347 [M+H]+ mp.286-290°C(decomposition) [α]_{D}²⁵ -8.27 (c 0.517, 0.1M HCl) |
| 2-19 | NMR-DMSO-d₆ : 0.60-0.78 (3H, m), 1.53-1.75 (1H, m), 1.92-2.14 (1H, m), 3.25-4.36 (7H, m), 5.28-5.43 (1H, m), 6.77-6.82 (1H, m), 7.37-7.45 (1H, m), 8.35-8.37 (1H, m), 8.63-8.66 (1H, m), 12.90 (1H, brs) ESI+ : 347 [M+H]+ mp.288-290°C(decomposition) [α]_{D}²⁵ +5.95 (c 0.507, 0.1M HCl) |
| 2-20 | ESI+ : 379 [M+H]+ |
| 2-21 | ESI+ : 363 [M+H]+ |
| 2-22 | ESI+ : 455 [M+H]+ |
| 2-23 | ESI+ : 364 [M+H]+ |
| 2-24 | ESI+ : 364 [M+H]+ |
| 2-25 | ESI+ : 397 [M+H]+ |
| 2-26 | ESI+ : 403 [M+H]+ |
| 2-27 | ESI+ : 431 [M+H]+ |
| 2-28 | ESI+ : 439 [M+H]+ |
| 2-29 | ESI+ : 473 [M+H]+ |
| 2-30 | ESI+ : 413 [M+H)+ |
| 2-31 | ESI+ : 377 [M+H]+ |
| 2-32 | ESI+ : 442 [M+H]+ |
| 2-33 | ESI+ : 393 [M+H]+ |
| 2-34 | ESI+ : 443 [M+H]+ |
| 2-35 | ESI+ : 391 [M+H]+ |
| 2-36 | ESI+ : 457 [M+H]+ |
| 2-37 | ESI+ : 379 [M+H]+ |
| 2-38 | ESI+ : 526 [M+H]+ |
| 2-39 | ESI+ : 427 [M+H]+ |
| 2-40 | ESI+ : 394 [M+H]+ |
| 2-41 | ESI+ : 452 [M+H]+ |
| 2-42 | ESI+ : 498 [M+H]+ |
| 2-43 | ESI+ : 393 [M+H]+ |
| 2-44 | ESI+ : 428 [M+H]+ |
| 2-45 | ESI+ : 499 [M+H]+ |
| 2-46 | ESI+ : 455 [M+H]+ |
| 2-47 | ESI+ : 441 [M+H]+ |
| 2-48 | ESI+ : 405 [M+H]+ |
| 2-49 | ESI+ : 411 [M+H]+ |
| 2-50 | ESI+ : 405 [M+H]+ |

**[Table 31]**

| Ex | DATA |
|---|---|
| 2-51 | ESI+ : 457 [M+H]+ |
| 2-52 | ESI+ : 455 [M+H]+ |
| 2-53 | ESI+ : 469 [M+H]+ |
| 2-54 | ESI+ : 459 [M+H]+ |
| 2-55 | ESI+ : 487 [M+H]+ |
| 2-56 | ESI+ : 427 [M+H]+ |
| 2-57 | ESI+ : 427 [M+H]+ |
| 2-58 | ESI+ : 495 [M+H]+ |
| 2-59 | ESI+ : 473 [M+H]+ |
| 2-60 | ESI+ : 515 [M+H]+ |
| 2-61 | ESI+ : 377 [M+H]+ |
| 2-62 | ESI+ : 378 [M+H]+ |
| 2-63 | ESI+ : 475 [M+H]+ |
| 2-64 | ESI+ : 407 [M+H]+ |
| 2-65 | ESI+ : 475 [M+H]+ |
| 2-66 | ESI+ : 392 [M+H]+ |
| 2-67 | ESI+ : 469 [M+H]+ |
| 2-68 | ES1+ : 544 [M+H]+ |
| 2-69 | ESI+ : 454 [M+H]+ |
| 2-70 | ESI+ : 444 [M+H]+ |
| 2-71 | ESI+ : 455 [M+H]+ |
| 3 | NMR-DMSO-d₆ : 0.57-0.70 (3H, m), 1.60-1.76 (1H, m), 1.80-1.97 (1H, m), 3.25-4.38 (7H, m), 5.06-5.20 (1H, m), 6.84-6.94 (1H, m), 7.50-7.54 (1H, m), 8.20-8.25 (1H, m), 8.52-8.63 (1H, m), 12.04 (1H, brs) ESI+ : 369 [M+Na]+ |
| 4 | NMR-DMSO-d₆ : 0.69 (3H, d, J = 6.9 Hz), 1.66-1.78 (1H, m), 1.86-1.97 (1H, m), 2.46-2.58 (1H, m), 3.63-3.72 (1H, m), 4.01-4.17 (2H, m), 4.44 (1H, dd, J = 6.6, 13.5 Hz), 5.02-5.09 (1H, m), 6.59 (1H, d, J = 3.3 Hz), 6.76 (1H, dd, J = 1.9, 3.3 Hz), 7.02 (1H, d, J = 9.1 Hz), 7.21 (1H, d, J = 3.3 Hz), 7.32 (1H, dd, J = 3.3, 3.3 Hz), 7.84 (1H, dd, J = 2.3, 9.1 Hz), 8.45 (1H, s), 8.46-8.47 (1H, m), 11.64 (1H, s) ESI+ : 357 [M+H]+ |
| 4-1 | NMR-DMSO-d₆ : 0.69 (3H, d, J = 6.9 Hz), 170-1.82 (1H, m), 1.92-2.03 (1H, m), 2.48-2.58 (1H, m), 3.76-3.85 (1H, m), 4.03-4.13 (1H, m), 4.19 (1H, dd, J = 3.8, 13.6 Hz), 4.48 (1H, dd, J = 6.6,13.6 Hz), 5.08-5.15 (1H, m), 6.59 (1H, d, J = 3.3 Hz), 6.79 (1H, dd, J = 1.8, 3.3 Hz), 7.22 (1H, d, J = 3.3 Hz), 7.33 (1H, dd, J = 3.3, 3.3 Hz), 8.45 (1H, s), 8.47-8.49 (1H, m), 8.52 (1H, d, J= 1.4Hz), 11.63(1H, s) ESI+ : 358 [M+H]+ |
| 4-2 | NMR-DMSO-d₆ : 0.77 (3H, d, J = 6.8 Hz), 1.67-1.76 (1H, m), 2.08-2.19 (1H, m), 2.49-2.58 (1H, m), 3.80-3.89 (1H, m), 3.98-4.06 (1H, m), 4.35 (1H, dd, J = 4.0, 13.2 Hz), 4.44 (1H, dd, J = 8.0, 13.2 Hz), 5.40-5.46 (1H, m), 6.77 (1H, d, J = 3.2 Hz), 7.43 (1H, d, J = 3.2 Hz), 8.35 (1H, s), 8.53-8.57 (2H, m), 8.65 (1H, s), 12.90 (1H, brs) ESI+ : 405 [M+Na]+ |

**[Table 32]**

| Ex | DATA |
|---|---|
| 5 | NMR-DMSO-d₆: 0.62 (3H, d, J = 6.9 Hz), 155-1.67 (1H, m), 1.75-1.87 (1H, m), 2.36-2.47 (1H, m), 3.36-3.45 (1H, m), 3.57-3.69 (1H, m), 3.76 (1H, dd, J = 3.6, 13.5 Hz), 3.98-4.07 (3H, m), 4.89-4.96 (1H, m), 6.63 (1H, d, J = 3.3 Hz), 6.74 (1H, dd, J = 1.9, 3.3 Hz), 7.24 (1H, d, J = 3.3 Hz), 7.33 (1H, dd, J = 3.3, 3.3 Hz), 7.34-7.40 (1H, m), 8.49 (1H, s), 11.63 (1H, s) ESI+: 337 [M+H]+ |
| 5-1 | NMR-DMSO-d₆: 0.65 (3H, d, J = 6.9 Hz),163-1.76 (1H, m), 1.80-1.92 (1H, m), 2.37-2.50 (1H, m), 2.82 (3H, s), 3.20-3.30 (1H, m), 3.50-3.60 (1H, m), 3.67 (1H, dd, J = 3.6, 13.3 Hz), 3.83 (1H, dd, J = 6.8, 13.3 Hz), 4.09 (2H, s), 4.97-5.04 (1H, m), 6.64 (1H, d, J = 3.2 Hz), 6.74 (1H, dd, J = 1.9, 3.3 Hz), 7.30 (1H, d, J = 3.3 Hz), 7.33 (1H, dd, J = 3.3, 3.3 Hz), 8.46 (1H, s), 11.65 (1H, s) ESI+: 351 [M+H]+ |
| 5-2 | NMR-DMSO-d₆: 0.68 (3H, d, J = 7.0 Hz), 150-1.60 (1H, m), 2.00-2.11 (1H, m), 2.37-2.62 (1H, m), 324-3.60 (2H, m), 3.87 (1H, dd, J = 3.8, 13.0 Hz), 3.99 (1H, dd, J = 8.1, 13.0 Hz), 4.00-4.10 (2H, m), 5.25-5.31 (1H, m), 6.79 (1H, d, J = 2.9 Hz), 7.34-7.44 (2H, m), 8.35 (1H, s), 8.65 (1H,s), 12.90 (1H, bs) ESI+: 362 [M+H]+ |
| 5-3 | NMR-DMSO-d₆: 0.70 (3H, d, J = 7.0 Hz), 155-1.71 (1H, m), 2.05-2.18 (1H, m), 2.38-2.58 (1H, m), 2.86 (3H, s), 328-3.46 (2H, m), 3.71 (1H, dd, J = 4.0,13.1 Hz), 3.86 (1H, dd, J = 8.1, 13.1 Hz), 4.10 (2H, s), 5.37-5.44 (1H, m), 6.79 (1H, d, J = 3.4 Hz), 7.45 (1H, d, J = 3.4 Hz), 8.36 (1H, s), 8.65 (1H, s), 12.90 (1H, bs) ESI+: 398 [M+Na]+ |
| 5-4 | ESI+: 374 [M+H]+ |
| 5-5 | ESI+: 388 [M+H]+ |
| 5-6 | ESI+: 378 [M+H]+ |
| 5-7 | ESI+: 422 [M+H]+ |
| 5-8 | ESI+: 422 [M+H]+ |
| 5-9 | ESI+: 402 [M+H]+ |
| 5-10 | ESI+: 408 [M+H]+ |
| 6 | ESI+: 409 [M+H]+ |
| 6-1 | ESI+: 410 [M+H]+ |
| 6-2 | ESI+: 443 [M+Hl+ |
| 6-3 | ESI+: 443 [M+H]+ |
| 6-4 | ESI+: 457 [M+H]+ |
| 6-5 | ESI+: 457 [M+H]+ |
| 6-6 | ESI+: 424 [M+H]+ |

**[Table 33]**

| Pr | Structure | Pr | Structure |
|---|---|---|---|
| 1-5 | 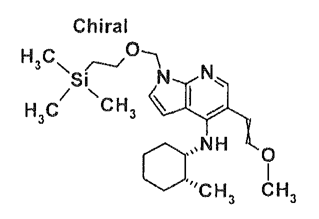 | 1-6 | 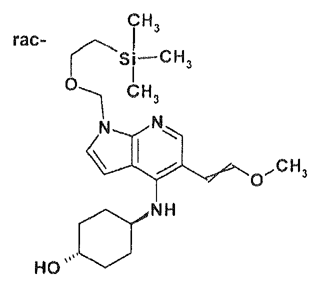 |
| 1-7 | 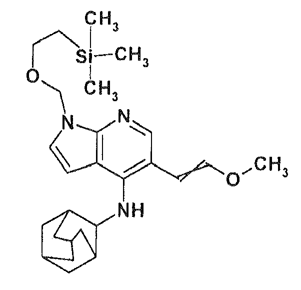 | 1-8 | 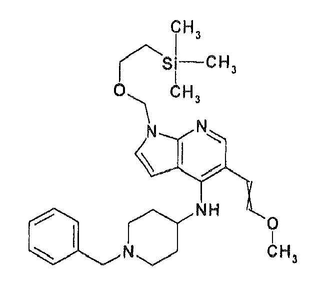 |
| 2-1 | 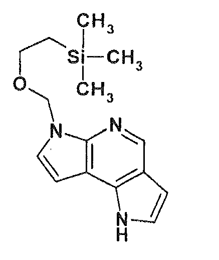 | 3-4 | 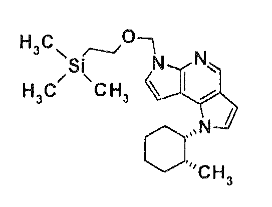 |
| 3-5 | 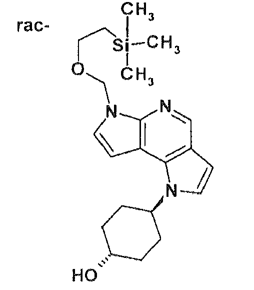 | 3-6 | 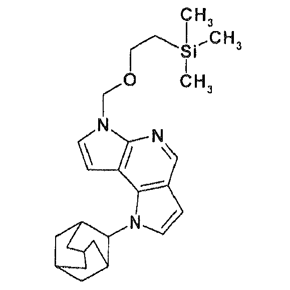 |

**[Table 34]**

| Pr | Structure | Pr | Structure |
|---|---|---|---|
| 3-7 | 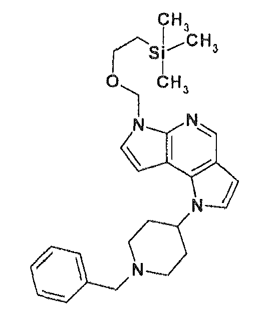 | 5-2 | 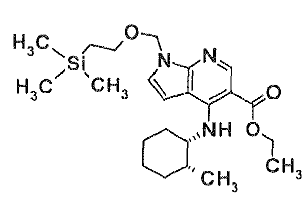 |
| 5-3 | 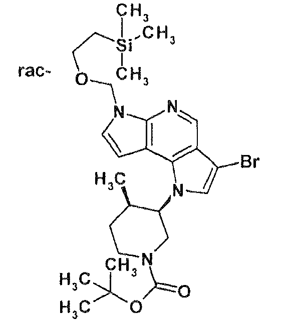 | 6-5 | 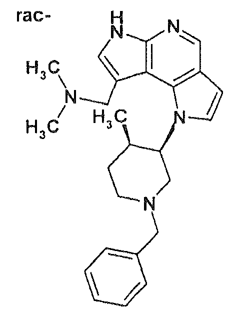 |
| 6-6 | 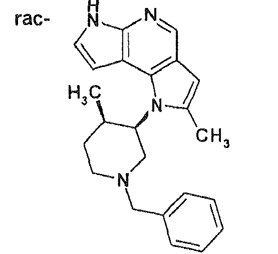 | 6-7 | 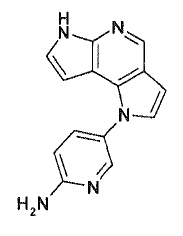 |
| 6-8 | 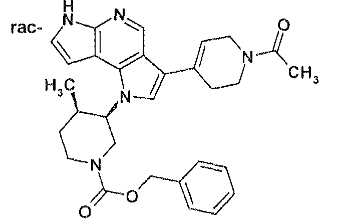 | 6-9 | 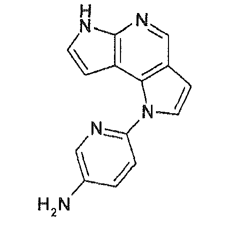 |

**[Table 35]**

| Pr | Structure | Pr | Structure |
|---|---|---|---|
| 11-9 | 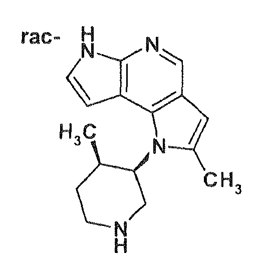 | 11-10 | 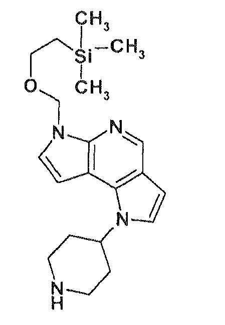 |
| 15-8 | 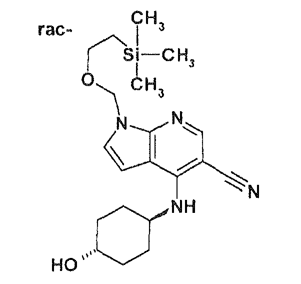 | 15-9 | 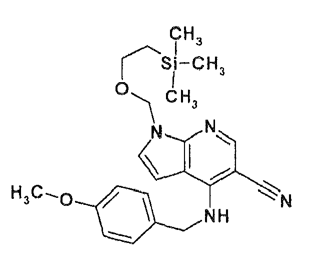 |
| 15-10 | 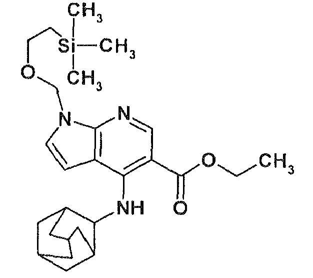 | 15-11 | 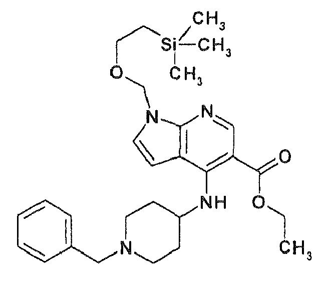 |

**[Table 36]**

| Pr | Structure | Pr | Structure |
|---|---|---|---|
| 15-12 | 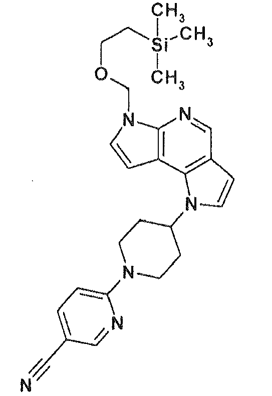 | 20-4 | 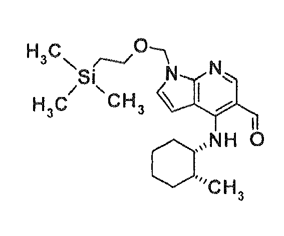 |
| 20-5 | 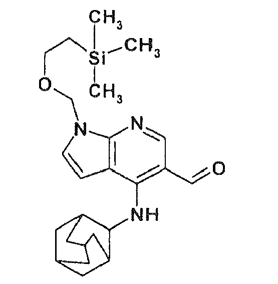 | 20-6 | 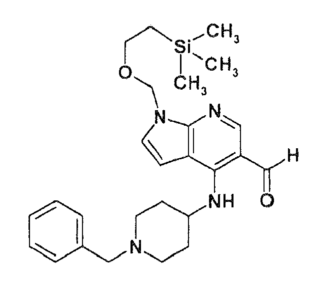 |
| 21-1 | 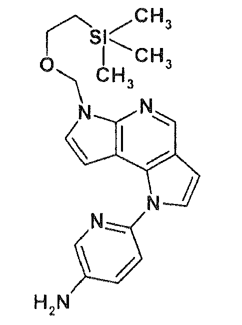 | 22-5 | 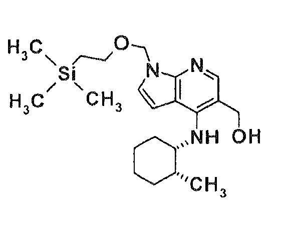 |

**[Table 37]**

| Pr | Structure | Pr | Structure |
|---|---|---|---|
| 22-6 | 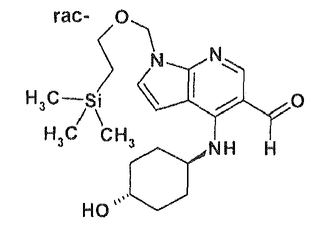 | 22-7 | 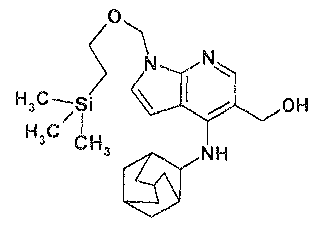 |
| 22-8 | 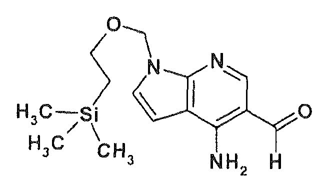 | 22-9 | 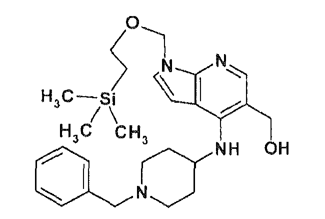 |
| 44 | 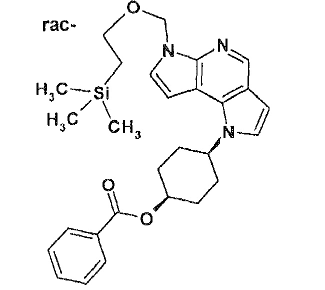 | 29-1 | 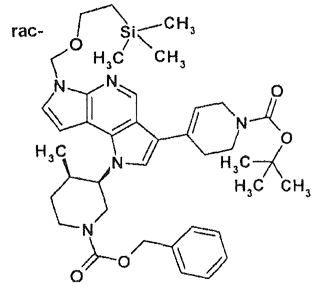 |
| 30 | 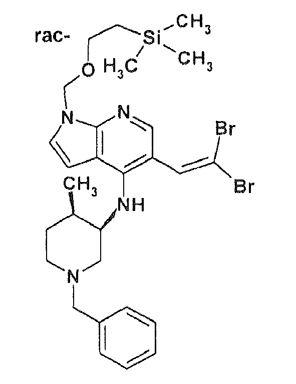 | 10-2 | 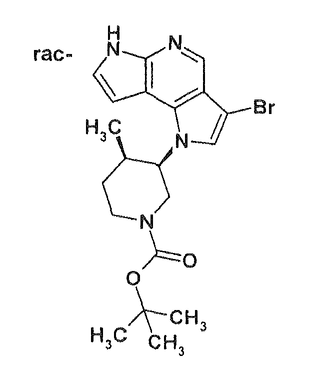 |

**[Table 38]**

| Pr | Structure | Pr | Structure |
|---|---|---|---|
| 32 | 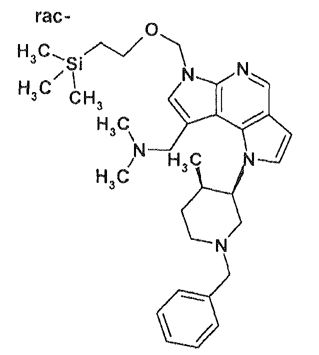 | 33 | 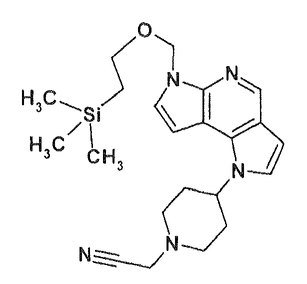 |
| 34 | 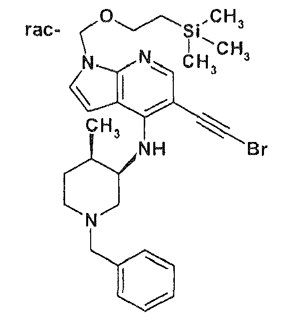 | 35 | 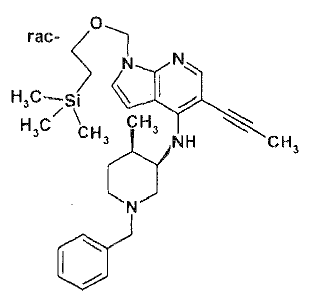 |
| 36 | 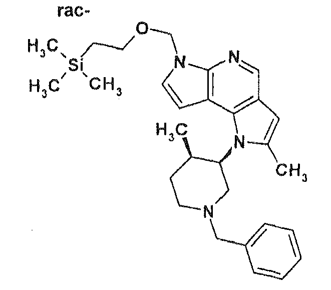 | 37 | 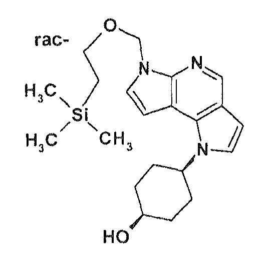 |
| 38 | 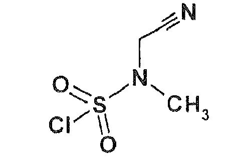 | 38-1 | 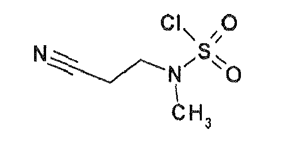 |

**[Table 39]**

| Pr | Structure | Pr | Structure |
|---|---|---|---|
| 39 | 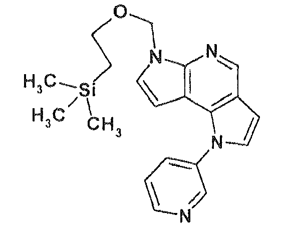 | 39-1 | 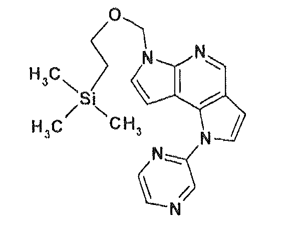 |
| 39-2 | 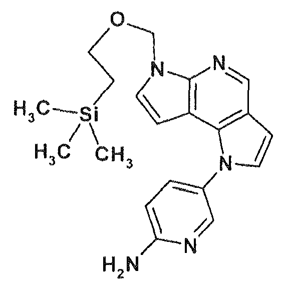 | 39-3 | 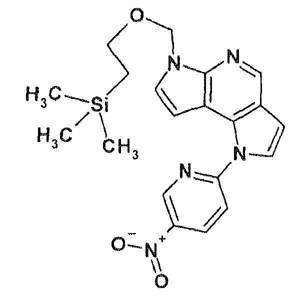 |
| 40 | 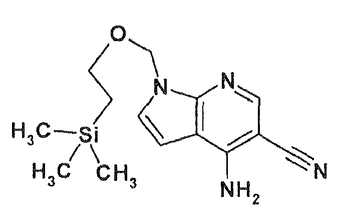 | 41 | 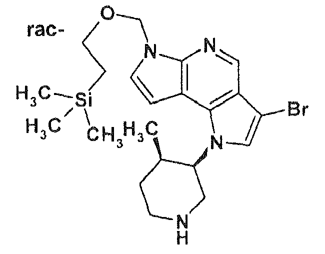 |
| 41-1 | 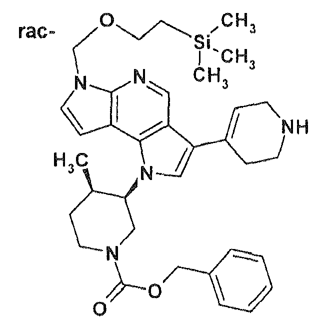 | 42 | 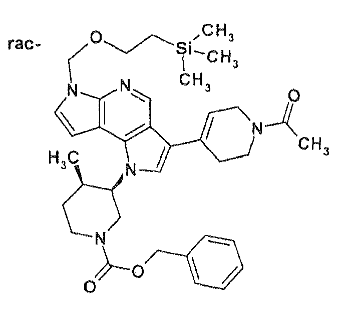 |

**[Table 40]**

| Pr | Structure | Pr | Structure |
|---|---|---|---|
| 43 | 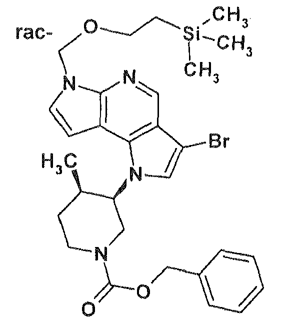 | 31 | 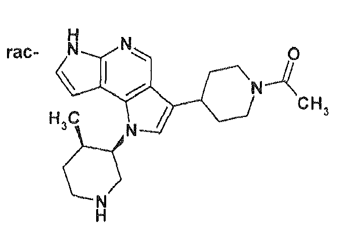 |
| 11-11 | 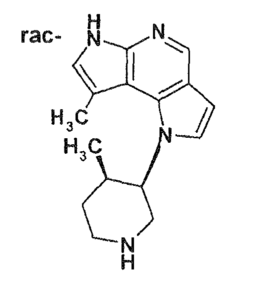 | 44-1 | 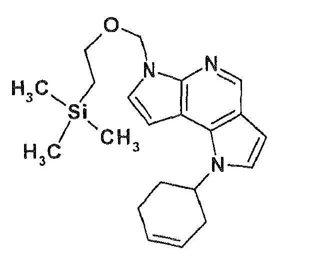 |
| 3-8 | 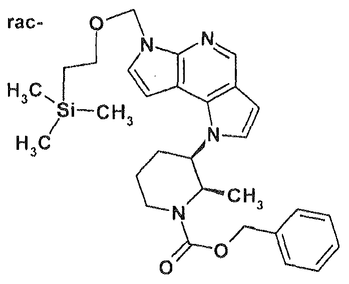 | | |

**[Table 41]**

| Pr | Data |
|---|---|
| 1-5 | ESI+ : 416 [M+H]+ |
| 1-6 | ESI+ : 418 [M+H]+ |
| 1-7 | ESI+ : 454 [M+H]+ |
| 1-8 | ESI+ : 493 [M+H]+ |
| 2-1 | APCI + : 288 [M+H]+ |
| 3-4 | ESI+ : 384 [M+H]+ |
| 3-5 | ESI+ : 386 [M+H]+ |
| 3-6 | ESI+ : 422 [M+H]+ |
| 3-7 | ESI+ : 461 [M+H]+ |
| 5-2 | ESI+: 432 [M+H]+ |
| 5-3 | ESI+ : 565, 563 [M+H]+ |
| 6-5 | ESI+ : 402 [M+H]+ |
| 6-6 | ESI+ : 359 [M+H]+ |
| 6-7 | APCI+: 250 [M+H]+ |
| 6-8 | ESI+:512 [M+H]+ |
| 6-9 | APCI+:250 [M+H]+ |
| 10-2 | ESI+ : 435, 433 [M+H]+ |
| 11-9 | ESI+: 269 [M+H]+ |
| 11-10 | ESI+: 371 [M+H]+ |
| 15-8 | ESI+: 387 [M+H]+ |
| 15-9 | APCI+: 409 [M+H]+ |
| 15-10 | ESI+: 470 [M+H]+ |
| 15-11 | ESI+ : 509 [M+H]+ |
| 15-12 | ESI+: 473 [M+H]+ |
| 20-4 | ESI+: 388 [M+H]+ |
| 20-5 | ESI+: 426 [M+H]+ |
| 20-6 | ESI+: 465 [M+H]+ |
| 21-1 | APCI+: 380 [M+H]+ |
| 22-5 | ESI+: 390 [M+H]+ |
| 22-6 | ESI+: 390 [M+H]+ |
| 22-7 | ESI+: 428 [M+H]+ |
| 22-8 | APCI+: 292 [M+H]+ |
| 22-9 | ESI+ : 467 [M+H]+ |
| 44 | ESI+: 490 [M+H]+ |
| 29-1 | ESI+: 700 [M+H]+ |
| 30 | ESI+ : 633, 635 [M+H]+ |
| 31 | ESI+ : 380 [M+H]+ |
| 32 | ESI+ : 532 [M+H]+ |
| 33 | ESI+ : 410 [M+H]+ |

**[Table 42]**

| Pr | Data |
|---|---|
| 34 | ESI+ : 553, 555 [M+H]+ |
| 35 | ESI+ : 489 [M+H]+ |
| 36 | ESI+ : 489 [M+H]+ |
| 37 | ESI+ : 386 [M+H]+ |
| 38 | NMR-CDCl₃: 3.17 (3H, s), 4.29 (2H, s) |
| 38-1 | NMR-CDCl₃: 2.78 (2H, t, J = 6.8 Hz), 3.15 (3H, s), 3.56 (2H, t, J = 6.8 Hz) |
| 39 | APCI+: 365 [M+H]+ |
| 39-1 | APCI+: 366 [M+H]+ |
| 39-2 | APCI+ : 380 [M+H]+ |
| 39-3 | APCI+: 410 [M+H]+ |
| 40 | APCI+: 289 [M+H]+ |
| 41 | ESI+: 465, 463 [M+H]+ |
| 41-1 | ESI+: 600 [M+H]+ |
| 42 | ESI+: 642 [M+H]+ |
| 43 | ESI+: 599, 597 [M+H]+ |
| 11-11 | ESI+: 269 [M+H]+ |
| 44-1 | ESI+: 368 [M+H]+ |
| 3-8 | ESI+: 519 [M+H]+ |

**[Table 43]**

| Ex | Structure | Ex | Structure |
|---|---|---|---|
| 2-72 | 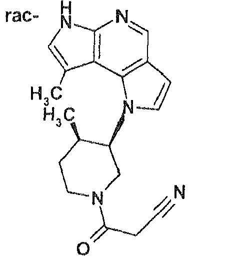 | 2-73 | 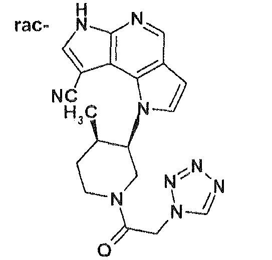 |
| 2-74 | 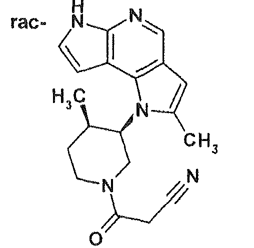 | 2-75 | 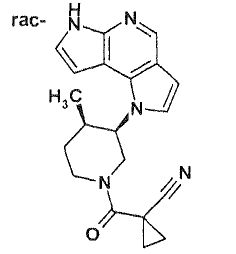 |
| 2-76 | 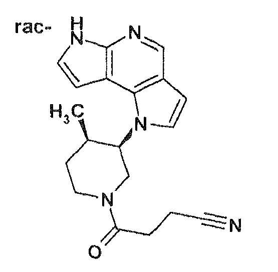 | 2-77 | 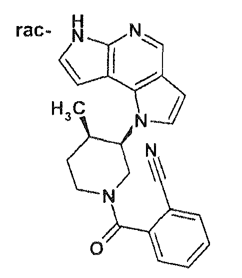 |
| 2-78 | 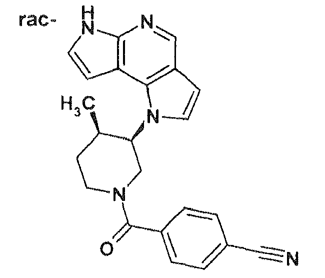 | 2-79 | 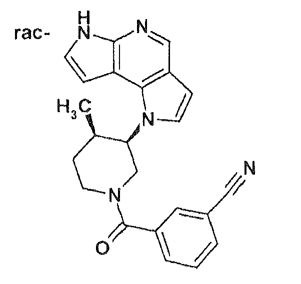 |

**[Table 44]**

| Ex | Structure | Ex | Structure |
|---|---|---|---|
| 2-80 | 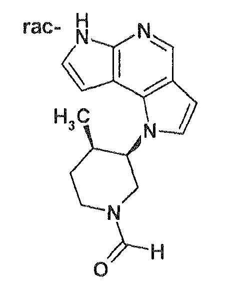 | 2-81 | 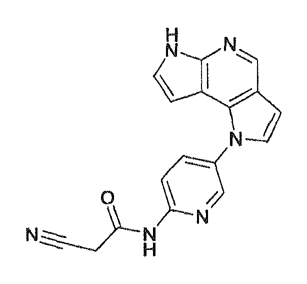 |
| 2-82 | 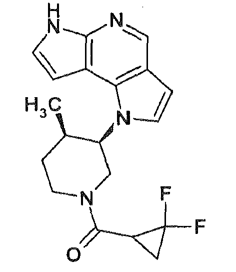 | 2-83 | 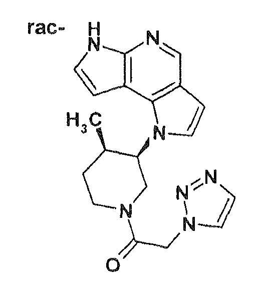 |
| | diastereo mixture | | |
| 2-84 | 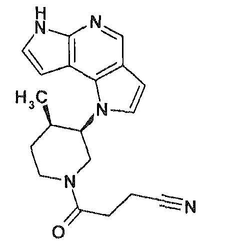 | 2-85 | 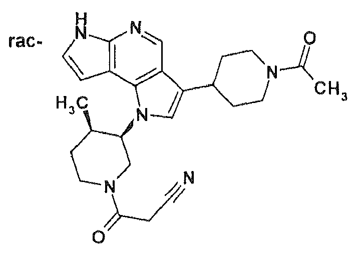 |
| 2-86 | 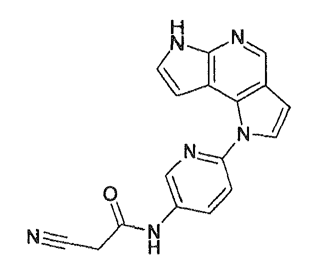 | 5-11 | 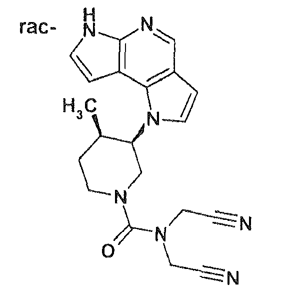 |

**[Table 45]**

| Ex | Structure | Ex | Structure |
|---|---|---|---|
| 6-7 | 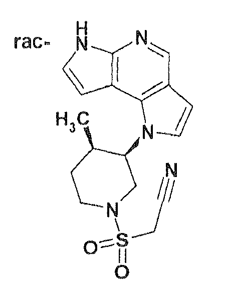 | 6-8 | 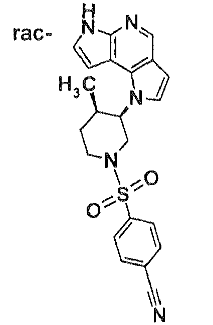 |
| 6-9 | 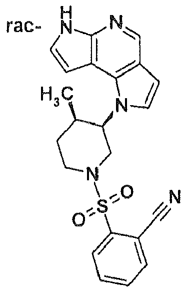 | 7 | 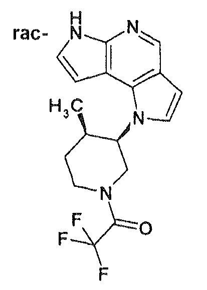 |
| 8 | 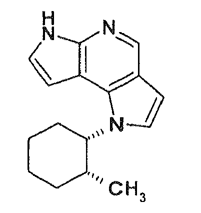 | 8-1 | 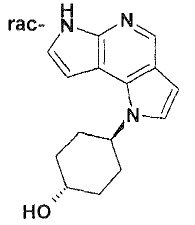 |
| 8-2 | 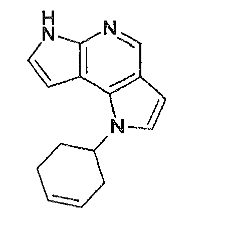 | 8-3 | 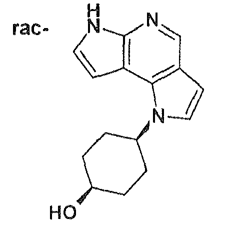 |

**[Table 46]**

| Ex | Structure | Ex | Structure |
|---|---|---|---|
| 8-4 | 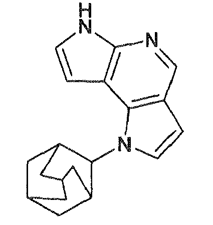 | 8-5 | 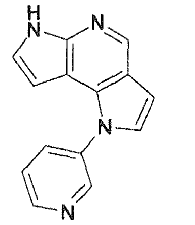 |
| 8-6 | 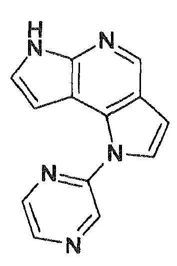 | 8-7 | 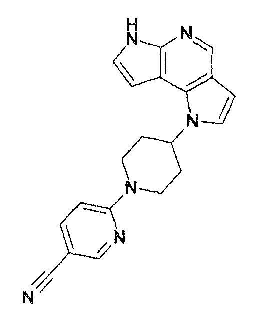 |
| 8-8 | 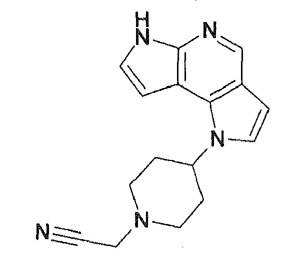 | 9 | 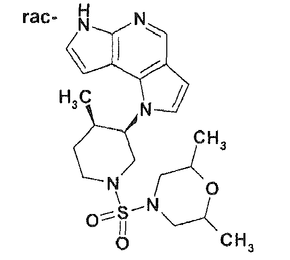 |
| 9-1 | 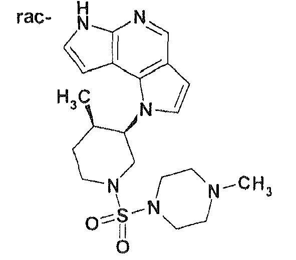 | 9-2 | 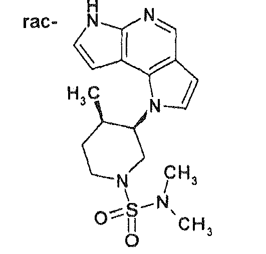 |

**[Table 47]**

| Ex | Structure | Ex | 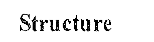 |
|---|---|---|---|
| 9-3 | 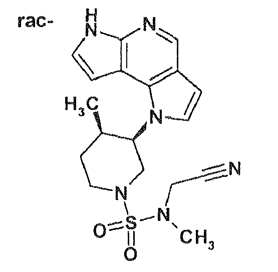 | 9-4 | 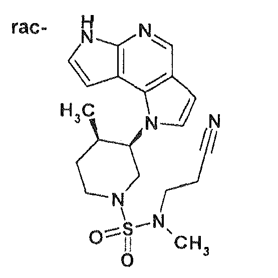 |

**[Table 48]**

| Ex | DATA |
|---|---|
| 2-72 | NMR-DMSO-d₆: 0.61-0.71 (3H, m), 1.61-1.91 (2H, m), 2.34 (3H, d, J = 3.6 Hz), 2.35-2.44 (1H, m), 3.37-3.43 (1H, m), 3.57-3.95 (3H, m), 4.00-4.31 (2H, m), 4.88-4.99 (1H, m), 6.71-7.74 (1H, m), 6.95-7.03 (1H, m), 7.31-7.34 (1H, m), 8.41-8.43 (1H, m), 11.60 (1H, s) ESI+ : 336 [M+H]+ |
| 2-73 | NMR-DMSO-d₆: 0.67-0.82 (3H, m), 1.57-1.86 (1H, m), 2.03-2.16 (1H, m), 2.44-2.59 (1H, m), 3.20-4.26 (4H, m), 5.25-5.95 (3H, m), 6.78-6.88 (1H, m), 7.42-7.50 (1H, m), 8.31-8.40 (1H, m), 8.61-8.69 (1H, m), 9.30 (1H, d, J = 3.6 Hz), 12.75-12.96 (1H, brs) ESI+ : 390 [M+H]+ |
| 2-74 | NMR-DMSO-d₆: 0.86-0.91 (3H, m), 1.60-1.68 (1H, m), 1.94-2.26 (1H, m), 2.51-2.53 (3H, m), 2.92-5.13 (8H, m), 6.36-6.37 (1H, m), 6.54-6.58 (1H, m), 7.32-7.36 (1H, m), 8.34-8.35 (1H, m), 11.56-11.62 (1H, m) ESI+ : 336 [M+H]+ |
| 2-75 | NMR-DMSO-d₆: 0.68 (3H, t, J = 6.8 Hz), 1.23-2.00 (7H, m), 3.69-4.27 (4H, m), 5.06 (1H, m), 6.66 (1H, d, J = 3.2 Hz), 6.80 (1H, m), 7.22 (1H, d, J = 3.2 Hz), 7.34 (1H, t, J = 2.8 Hz), 8.47 (1H, s), 11.65 (1H, s) ESI+: 348 [M+H]+ |
| 2-76 | NMR-DMSO-d₆: 0.66 (3H, m), 1.60-2.23 (3H, m), 2.41-3.01 (4H, m), 3.43-3.51 (1H, m), 3.65-4.19 (3H, m), 4.98 (1H, m), 6.64 (1H, m), 6.76 (1H, m), 7.22 (1H, m), 7.34 (1H, m), 8.46 (1H, m), 11.63 (1H, bs) ESI+ : 336 [M+H]+ |
| 2-77 | NMR-DMSO-d₆: 0.69 (3H, m), 1.65 (1H, m), 1.93 (1H, m), 3.49 (1H, m), 3.69 (1H, m), 3.87 (1H, m), 4.07 (1H, m), 4.27 (1H, m), 5.01 (1H, m), 6.61-6.80 (2H, m), 7.22-7.48 (4H, m), 7.67-8.01 (2H, m), 8.46 (1H, m), 11.64 (1H, m) APCI+: 384 [M+H]+ |
| 2-78 | NMR-DMSO- d₆: 0.69 (3H, m), 1.58-2.00 (3H, m), 3.52 (1H, m), 3.67-3.87 (1H, m), 4.20 (2H, m), 5.04 (1H, m), 6.67-6.85 (2H, m), 7.24-7.36 (3H, m), 7.61 (1H, m), 7.73 (1H, m), 7.99 (1H, m), 8.48 (1H, m), 11.66 (1H, m) ESI+ : 384 [M+H]+ |
| 2-79 | NMR-DMSO-d₆: 0.70 (3H, m), 1.64-1.96 (3H, m), 3.50 (1H, m), 3.80 (1H, m), 4.19 (2H, m), 5.04 (1H, m), 6.67-6.85 (2H, m), 7.27-7.43 (3H, m), 7.60-8.05 (3H, m), 8.47 (1H, s), 11.65 (1H, s) ESI+ : 384 [M+H]+ |
| 2-80 | NMR-DMSO-d₆: 0.66 (3H, t, J = 8.0 Hz), 1.58-1.73 (1H, m), 1.78-1.88 (1H, m), 2.45-2.51 (1H, m), 3.39-3.51 (1H, m), 3.63-3.71 (1H, m), 3.74-3.87 (1H, m), 3.96-4.12 (1H, m), 4.93-5.03 (1H, m), 6.65 (1H, dd, J = 12.8, 3.6 Hz), 6.77 (1H, m), 7.18-7.29 (1H, m), 7.32-7.35 (1H, m), 8.00-8.16 (1H, m), 8.46 (1H, s), 11.64 (1H, s) ESI+ : 283 [M+H]+ |

**[Table 49]**

| Ex | DATA |
|---|---|
| 2-81 | NMR-DMSO-d₆: 4.06 (2H, s), 5.95 (1H, m), 6.88 (1H, d, J = 3.2 Hz), 7.25 (1H, t, J = 3.2 Hz), 7.50 (1H, d, J = 3.6 Hz), 8.15 (1H, dd, J = 8.4, 2.8 Hz), 8.24 (1H, m), 8.56 (1H, s), 8.64 (1H, d, J = 2.8 Hz), 11.13 (1H, s), 11.71 (1H, s) ESI+: 317 [M+H]+ |
| 2-82 | NMR-DMSO-d₆: 0.63-0.72 (3H, m), 1.62-2.02 (5H, m), 3.19-4.29 (5H, m), 4.91-5.12 (1H, m), 6.59-6.79 (2H, m), 7.12-7.36 (2H, m), 8.44-8.48 (1H, m), 11.61-11.67 (1H, m) ESI+: 359 [M+H]+ |
| 2-83 | NMR-DMSO-d₆: 0.61-0.71 (3H, m), 1.72 (1H, m), 1.87 (1H, m), 3.49-4.22 (5H, m), 4.96-5.82 (3H, m), 6.62-6.72 (1H, m), 6.76-6.81 (1H, m), 7.23-7.32 (1H, m), 7.33-7.36 (1H, m), 7.75 (1H, s), 7.83 (1H, s), 8.44-8.50 (1H, m), 11.63 (1H, s) ESI+ : 364 [M+H]+ |
| 2-84 | NMR-DMSO-d₆: 0.62-0.70 (3H, m), 1.63-1.74 (1H, m), 1.78-1.91 (1H, m), 2.17-2.23 (1H, m), 2.50-2.67 (2H, m), 2.76-2.99 (1H, m), 3.47 (1H, m), 3.66-3.93 (2H, m), 4.02, 4.16 (1H, m), 4.91-5.08 (1H, m), 6.61-6.67 (1H, m), 6.74-6.80 (1H, m), 7,17-7.28 (1H, m), 7.32-7.36 (1H, m), 8.45-8.49 (1H, m), 11.65 (1H, s) NMR-DMSO-d6: 0.62-0.70 (3H, m), 1.63-1.74 (1H, m), 1.78-1.91 (1H, m), 2.17-2.23 (1H, m), 2.45-3.00 (4H, m), 3.47 (1H, m), 3.66-3.93 (2H, m), 4.02, 4.16 (1H, m), 4.91-5.08 (1H, m), 6.61-6.67 (1H, m), 6.74-6.80 (1H, m), 7.17-7.28 (1H, m), 7.32-7.36 (1H, m), 8.45-8.49 (1H, m), 11.65 (1H, s) ESI+ : 336 [M+H]+ |
| 2-85 | NMR-DMSO-d₆: 0.61-0.73 (3H, m), 1.40-1.77 (3H, m), 1.79-4.34 (17H, m), 4.45-4.56 (1H, m), 4.83-5.05 (1H, m), 6.67-6.76 (1H, m), 6.89-7.02 (1H, m), 7.29-7.35 (1H, m), 8.51-8.57 (1H, m), 11.62 (1H, brs) ESI+: 447 [M+H]+ |
| 2-86 | NMR-DMSO-d₆: 4.02 (2H, s), 6.44 (1H, m), 6.88 (1H, d, J = 3.2 Hz), 7.27 (1H, t, J = 2.8 Hz), 7.66 (1H, d, J = 3.2 Hz), 7.76 (1H, d, J = 8.8 Hz), 8.25 (1H, dd, J = 8.8, 2.4 Hz), 8.55 (1H, s), 8.79 (1H, d, J = 2.4 Hz), 10.74 (1H, s), 11.67 (1H, s) ESI+ : 317 [M+H]+ |
| 5-11 | NMR-DMSO-d₆: 0.65 (3H, d, J = 7.2 Hz), 1.69-1.76 (1H, m), 1.88-1.95 (1H, m), 2.46 (1H, m), 3.34 (1H, m), 3.56-3.62 (1H, m), 3.75 (1H, dd, J = 12.8, 4.0 Hz), 3.87 (1H, dd, J = 12.8, 6.8 Hz), 4.28 (4H, s), 5.04 (1H, m), 6.64 (1H, d, J = 3.2 Hz), 6.75 (1H, m), 7.27 (1H, d, J = 3.6 Hz), 7.34 (1H, t, J = 3.2 Hz), 8.46 (1H, s), 11.64 (1H, s) ESI+: 376 [M+H]+ |
| 6-7 | NMR-DMSO-d₆: 0.61 (3H, t, J = 6.8 Hz), 1.75 (1H, m), 1.91 (1H, m), 2.40 (1H, m), 3.36 (1H, m), 3.68-3.75 (2H, m), 3.96 (1H, dd, J = 12.8, 5.6 Hz), 5.00 (2H, s), 5.13 (1H, m), 6.67 (1H, d, J = 3.6 Hz), 6.80 (1H, m), 7.35 (1H, t, J = 3.2 Hz), 7.43 (1H, d, J = 3.6 Hz), 8.46 (1H, s), 11.64 (1H, s) ESI+: 358 [M+H]+ |

**[Table 50]**

| Ex | DATA |
|---|---|
| 6-8 | NMR-DMSO-d₆: 0.51 (3H, d, J = 7 Hz), 1.73-1.81 (2H, m), 2.17-2.23 (1H, m), 2.75-2.80 (1H, m), 3.15 (1H, dd, J = 4, 12 Hz), 3.64-3.67 (1H, m), 3.85 (1H, dd, J = 4, 12 Hz), 5.08 (1H, m), 6.69 (1H, d, J = 4 Hz), 6.72 (1H, m), 7.32 (1H, m), 7.58 (1H, d, J = 4 Hz), 8.00 (2H, d, J = 9 Hz), 8.17 (2H, d, J = 9 Hz), 8.47 (1H, s), 11.64 (1H, s) ESI+: 420 [M+H]+ |
| 6-9 | NMR-DMSO-d₆: 0.55 (3H, d, J = 7 Hz), 1.76-1.83 (2H, m), 2.24-2.29 (1H, m), 2.97-3.02 (1H, m), 3.29-3.39 (1H, m), 3.72-3.75 (1H, m), 3.93 (1H, dd, J = 4, 12 Hz), 5.11 (1H, m), 6.68 (1H, d, J = 4 Hz), 6.72 (1H, m), 7.33 (1H, m), 7.53 (1H, d, J = 4 Hz), 7.91-7.99 (2H, m), 8.07 (1H, d, J = 8 Hz), 8.21 (1H, d, J = 8 Hz), 8.47 (1H, s), 11.64 (1H, s) ESI+: 420 [M+H]+ |
| 7 | NMR-DMSO-d₆: 0.66-0.71 (3H, m), 1.59-1.81 (1H, m), 1.97-2.09 (1H, m), 2.48-2.58 (1H, m), 3.62-4.26 (4H, m), 4.97-5.16 (1H, m), 6.65-6.68 (1H, m), 6.75-6.85 (1H, m), 7.15-7.25 (1H, m), 7.29-7.37 (1H, m), 8.43-8.48 (1H, m), 11.56-11.69 (1H, m) ESI+: 351 [M+H]+ |
| 8 | NMR-DMSO-d₆: 0.63 (3H, d, J = 4.0 Hz), 1.48-1.69 (3H, m), 1.84-1.95 (3H, m), 2.23-2.34 (1H, m), 2.42-2.48 (2H, m), 4.76-4.80 (1H, m), 6.59 (1H, d, J = 3.2 Hz), 6.61-6.63 (1H, m), 7.29 (1H, d, J = 3.2 Hz), 7.33-7.35 (1H, m), 8.44 (1H, s), 11.61 (1H, s) ESI+: 254 [M+H]+ |
| 8-1 | NMR-DMSO-d₆: 1.50-1.60 (2H, m), 1.81-1.90 (2H, m), 1.99-2.08 (4H, m), 3.57-3.62 (1H, m), 4.53-4.59 (1H, m), 4.72 (1H, d, J = 4 Hz), 6.60 (1H, d, J = 4 Hz), 6.67 (1H, m), 7.34 (2H, m), 8.43 (1H, s), 11.67 (1H, brs) ESI+: 256 [M+H]+ |
| 8-2 | NMR-DMSO-d₆: 2.06-2.20 (3H, m), 2.33-2.40 (1H, m), 2.52-2.58 (2H, m), 4.83-4.91 (1H, m), 5.79-5.85 (2H, m), 6.61 (1H, m), 6.63 (1H, d, J = 4 Hz), 7.33 (1H, m), 7.36 (1H, d, J = 4 Hz), 8.45 (1H, s), 11.62 (1H, s) ESI+: 238 [M+H]+ |
| 8-3 | NMR-DMSO-d₆: 1.75-1.87 (6H, m), 2.15-2.24 (2H, m), 3.96 (1H, m), 4.53-4.59 (2H, m), 6.60 (1H, d, J = 3 Hz), 6.75 (1H, m), 7.33 (2H, m), 8.44 (1H, s), 11.59 (1H, s) ESI+: 256 [M+H]+ |
| 8-4 | NMR-DMSO-d₆: 1.68-1.71 (2H, m), 1.89 (2H, m), 1.91-2.05 (6H, m), 2.15-2.17 (2H, m), 2.53 (2H, m), 4.80 (1H, s), 6.48 (1H, m), 6.63 (1H, d, J = 4 Hz), 7.34 (1H, m), 7.58 (1H, d, J = 4 Hz), 8.47 (1H, s), 11.62 (1H, s) ESI+: 292 [M+H]+ |

**[Table 51]**

| Ex | DATA |
|---|---|
| 8-5 | NMR-DMSO-d₆: 5.96 (1H, dd, J = 1.6, 1.2 Hz), 6.91 (1H, d, J = 3.6 Hz), 7.27 (1H, t, J = 2.8 Hz), 7.54 (1H, d, J = 3.6 Hz), 7.68 (1H, dd, J = 8.0, 4.8 Hz), 8.13 (1H, dq, J = 8.4, 1.2 Hz), 8.58 (1H, s), 8.72 (1H, dd, J = 4.8, 1.2 Hz), 8.91 (1H, d, J = 2.8 Hz), 11.74 (1H, s) ESI+ : 235 [M+H]+ |
| 8-6 | NMR-DMSO-d₆: 6.58 (1H, m), 6.99 (1H, d, J = 3.6 Hz), 7.32 (1H, t, J = 2.8 Hz), 7.86 (1H, d, J = 3.6 Hz), 8.58 (1H, s), 8.70 (1H, d, J = 2.8 Hz), 8.76 (1H, dd, J = 2.8, 1.6 Hz), 9.15 (1H, d, J = 1.6 Hz), 11.74 (1H, s) ESI+ : 236 [M+H]+ |
| 8-7 | NMR-DMSO-d₆: 1.90-2.00 (2H, m), 2.14-2.17 (2H, m), 3.29-3.37 (2H, m), 4.67-4.71 (2H, m), 4.95-5.03 (1H, m), 6.61 (1H, d, J = 4 Hz), 6.82 (1H, m), 7.05 (1H, d, J = 9 Hz), 7.35-7.37 (2H, m), 7.88 (1H, dd, J = 2, 9 Hz), 8.44 (1H, s), 8.52 (1H, m), 11.63 (1H, s) ESI+ : 343 [M+H]+ |
| 8-8 | NMR-DMSO-d₆: 2.03-2.12 (4H, m), 2.54-2.59 (2H, m), 2.98-3.01 (2H, m), 3.32 (2H, s), 4.56-4.64 (1H, m), 6.63 (1H, d, J = 4 Hz), 6.71 (1H, m), 7.34 (1H, m), 7.40 (1H, d, J = 4 Hz), 8.45 (1H, s), 11.63 (1H, s) ESI+ : 280 [M+H]+ |
| 9 | NMR-DMSO-d₆: 0.63 (3H, t, J = 7.6 Hz), 1.07 (6H, dd, J = 6.4, 2.0 Hz), 1.70-1.86 (2H, m), 2.35 (1H, m), 3.14-3.66 (10H, m), 3.85 (1H, m), 5.07 (1H, m), 6.65 (1H, d, J = 3.2 Hz), 6.76 (1H, m), 7.34 (1H, t, J = 3.2 Hz), 7.51 (1H, d, J = 3.6 Hz), 8.46 (1H, s), 11.64 (1H, s) ESI+: 432 [M+H]+ |
| 9-1 | NMR-DMSO-d₆: 0.61 (3H, d, J = 6.8 Hz), 1.67-1.87 (2H, m), 2.15 (3H, s), 2.27-2.38 (5H, m), 3.14-3.22 (5H, m), 3.53 (1H, dd, J = 12.4, 3.6 Hz), 3.60 (1H, m), 3.84 (1H, dd, J = 12.4, 5.2 Hz), 5.07 (1H, m), 6.65 (1H, d, J = 3.2 Hz), 6.76 (1H, m), 7.34 (1H, t, J = 3.2 Hz), 7.51 (1H, d, J = 3.2 Hz), 8.46 (1H, s), 11.63 (1H, s) ESI+ : 417 [M+H]+ |
| 9-2 | NMR-DMSO-d₆: 0.61 (3H, t, J = 6.8 Hz), 1.69-1.87 (2H, m), 2.34 (1H, m), 2.80 (6H, s), 3.15-3.21 (1H, m), 3.53-3.61 (2H, m), 3.84 (1H, dd, J = 12.4, 5.2 Hz), 5.07 (1H, m), 6.65 (1H, d, J = 3.2 Hz), 6.78 (1H, m), 7.34 (1H, t, J = 3.2 Hz), 7.53 (1H, d, J = 3.2 Hz), 8.46 (1H, s), 11.63 (1H, s) ESI+ : 362 [M+H]+ |
| 9-3 | NMR-DMSO-d₆: 0.61 (3H, d, J = 6.8 Hz), 1.73 (1H, m), 1.90 (1H, m), 2.38 (1H, m), 2.91 (3H, s), 3.23 (1H, m), 3.53-3.62 (2H, m), 3.86 (1H, dd, J = 12.4, 6.0 Hz), 4.41 (2H, s), 5.10 (1H, m), 6.66 (1H, d, J = 3.2 Hz), 6.77 (1H, m), 7.34 (1H, t, J = 3.2 Hz), 7.47 (1H, d, J = 3.2 Hz), 8.46 (1H, s), 11.64 (1H, s) ESI+ : 387 [M+H]+ |

**[Table 52]**

| Ex | DATA |
|---|---|
| 9-4 | NMR-DMSO-d₆: 0.61 (3H, d, J = 7.2 Hz), 1.68-1.77 (1H, m), 1.83-1.89 (1H, m), 2.35 (1H, m), 2.79 (2H, t, J = 6.4 Hz), 2.86 (3H, s), 3.18 (1H, m), 3.44 (2H, m), 3.53-3.58 (2H, m), 3.83 (1H, dd, J = 12.0, 5.2 Hz), 5.09 (1H, m), 6.66 (1H, d, J = 3.2 Hz), 6.77 (1H, m), 7.34 (1H, t, J = 3.2 Hz), 7.50 (1H, d, J = 3.6 Hz), 8.46 (1H, s), 11.64 (1H, s) ESI+ : 401 [M+H]+ |

### Industrial Applicability

The compound of the formula (I) or a salt thereof has a JAK inhibitory action, and therefore can be used as an agent for preventing or treating diseases caused by undesirable cytokine signal transduction or diseases caused by abnormal cytokine signal transduction.

## Claims

1. A compound of the formula (I) or a salt thereof: 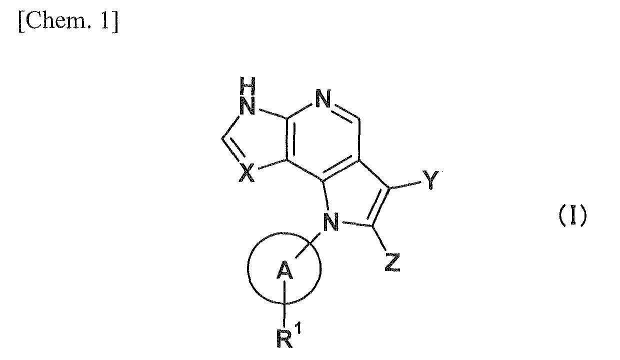 (wherein
A represents cycloalkyl which may be substituted, cycloalkenyl which may be substituted, or a nitrogen-containing hetero ring group which may be substituted,
X represents CR^{X} or N,
R^{X} represents H, OR^{XY1}, NR^{XY2}R^{XY3}, SR^{XY4}, halogen, cyano, or a lower alkyl, aryl, or hetero ring group,
Y represents H, OR^{XY1}, NR^{XY2}R^{XY3}, SR^{XY4}, halogen, cyano, lower alkyl which may be substituted, aryl which may be substituted, or a hetero ring group which may be substituted,
Z represents H or lower alkyl,
R^{XY1}, R^{XY2}, R^{XY3}, and R^{XY4} are the same as or different from each other and represent H or lower alkyl,
R¹ represents H, OH, -(CR¹¹R¹²)ₘ-R¹³, -SO₂-R¹⁴, or a hetero ring group which may be substituted,
R¹¹ and R¹² are the same as or different from each other and represent H, halogen, OH, lower alkyl which may be substituted, aryl which may be substituted, or a hetero ring group which may be substituted,
R¹¹ and R¹² are combined with each other to form oxo (=O), or
R¹¹ and R¹² may be combined with a carbon atom to which they are bonded to form cycloalkyl which may be substituted,
R¹³ represents H, halogen, cyano, -NR^{N1}R^{N2}, aryl which may be substituted, cycloalkyl which may be substituted, or a hetero ring group which may be substituted,
R^{N1} and R^{N2} are the same as or different from each other and represent H, lower alkyl which may be substituted, or aryl which may be substituted,
R¹⁴ represents NR^{N3}R^{N4}, or lower alkyl which may be substituted, or a hetero ring group which may be substituted,
R^{N3} and R^{N4} are the same as or different from each other and represent H, lower alkyl which may be substituted, or aryl which may be substituted, and
m represents 1, 2, 3, or 4).

2. The compound or a salt thereof as set forth in claim 1, wherein A is cycloalkyl which may be substituted, or a nitrogen-containing hetero ring group which may be substituted,
X is CR^{X} or N,
R^{X} is H, OR^{XY1}, NR^{XY2}R^{XY3}, SR^{XY4}, halogen, cyano, or a lower alkyl, aryl, or hetero ring group,
Y is H, OR^{XY1}, NR^{XY3}R^{XY3}, SR^{XY4}, halogen, cyano, or a lower alkyl, aryl, or hetero ring group,
R^{XY1}, R^{XY2}, R^{XY3} and R^{XY4} are the same as or different from each other and represent H or lower alkyl,
R¹ is H, OH,-(CR¹¹R¹²)ₘ-R¹³, -SO₂- (lower alkyl which may be substituted), or a hetero ring group which may be substituted,
R¹¹ and R¹² are the same as or different from each other and represent H, halogen, OH, lower alkyl which may be substituted, aryl which may be substituted, or a hetero ring group which may be substituted,
R¹¹ and R¹² are combined with each other to form oxo (=O),
R¹¹ and R¹² may be combined with a carbon atom to which they are bonded to form cycloalkyl,
R¹³ is H, halogen, cyano, -NR^{N1}R^{N2}, aryl which may be substituted, cycloalkyl which may be substituted, or a hetero ring group which may be substituted,
R^{N1} and R^{N2} are the same as or different from each other and represent H, lower alkyl which may be substituted, or aryl which may be substituted, and
m represents 1, 2, 3, or 4.

3. The compound or a salt thereof as set forth in claim 1, wherein A is 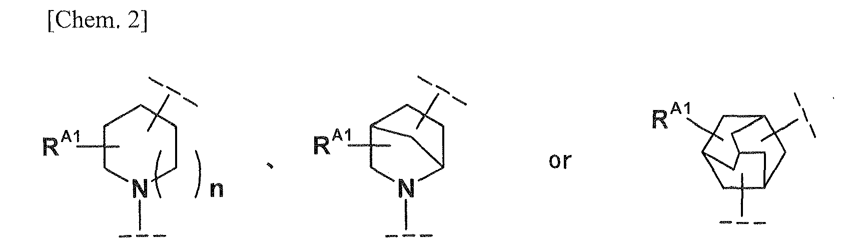 (wherein R^{A1} represents H or lower alkyl, and n represents 0, 1, or 2).

4. The compound or a salt thereof as set forth in claim 1, wherein A is 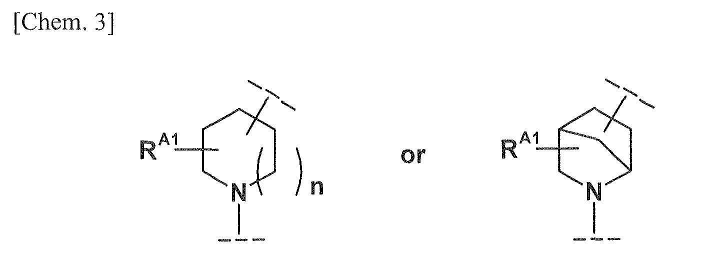 (wherein R^{A1} represents H or lower alkyl, and n represents 0, 1, or 2).

5. The compound or a salt thereof as set forth in claim 4, wherein R^{A1} is H or methyl.

6. The compound or a salt thereof as set forth in claim 5, wherein R¹ is -(CR¹¹R¹²)ₘ-R¹³, and R¹³ is cyano, -NR^{N1}R^{N2}, or a hetero ring group which may be substituted with lower alkyl.

7. The compound or a salt thereof as set forth in claim 6, wherein R¹ is -C(=O)-R¹³ or -C(=O)-CH₂-R¹³, R¹³ is cyano, -NR^{N1}R^{N2}, or 1H-tetrazol-1-yl which may be substituted with lower alkyl, and R^{N1} and R^{N2} are the same as or different from each other and represent H or lower alkyl which may be substituted with cyano.

8. The compound or a salt thereof as set forth in claim 5, wherein R¹ is 5-cyanopyridin-2-yl or 5-cyanopyrazin-2-yl.

9. The compound or a salt thereof as set forth in claim 7 or 8, wherein X is CR^{X}, R^{X} is H, bromo, or cyano, and Y is H, halogen, cyano, or a hetero ring group.

10. The compound or a salt thereof as set forth in claim 1, which is
rac-3-[(3R,4R)-3-(dipyrrolo[2,3-b:2',3'-d]pyridin-1 (6H)-yl)-4-methylpiperidin-1-yl]-3-oxopropanenitrile,
rac-(1S, 3R,4R,5S)-4-(dipyrrolo[2,3-b:2',3'-d]pyridin-1(6H)-yl)-adamantan-1-ol, rac-3-[(3R,4R)-3-(3-bromodipyrrolo[2,3-b:2',3'-d]pyridin-1 (6H)-yl)-4-methylpiperidin-1-yl] -3-oxopropanenitrile,
rac-1-[(3R,4R)-4-methyl-1-(1H-tetrazol-1-ylacetyl)piperidin-3-yl]-1,6-dihydrodipyrrolo[2,3-b:2',3'-d]pyridine,
rac-3-[(3R,4R)-3-(8-bromodipyrrolo[2,3-b:2',3'-d]pyridin-1(6H)-yl)-4-methylpiperidin-1-yl]-3-oxopropanenitrile,
rac-1-[(3R,4R)-1-(cyanoacetyl)-4-methylpiperidin-3-yl]-1,6-dihydrodipyrrolo[2,3-b:2',3'-d]pyridine-8-carbonitrile,
rac-1-[(3R,4R)-1-(cyanoacetyl)-4-methylpiperidin-3-yl]-1,6-dihydrodipyrrolo[2,3-b:2',3'-d]pyridine-3-carbonitrile,
rac-1-{(3R,4R)-4-methyl-1-[(5-methyl-1H-tetrazol-1-yl)acetyl]piperidin-3-yl}-1,6-dihydrodipyrrolo[2,3-b:2',3'-d]pyridine,
3-[(3S,4S)-3-dipyrrolo[2,3-b:2',3'-d]pyridin-1(6H)-yl-4-methylpiperidin-1-yl]-3-oxopropanenitrile,
3-[(3R,4R)-3-dipyrrolo[2,3-b:2',3'-d]pyridin-1 (6H)-yl-4-methylpiperidin-1-yl]-3-oxopropanenitrile,
(1S, 3R,4R,5S)-4-(dipyrrolo[2,3-b:2',3'-d]pyridin-1(6H)-yl)-adamantan-1-ol,
(1R,3S,4S,5R)-4-(dipyrrolo[2,3-b:2',3'-d]pyridin-1(6H)-yl)-adamantan-1-ol,
3-[(3S,4S)-3-(3-bromodipyrrolo[2,3-b:2',3'-d]pyridin-1(6H)-yl)-4-methylpiperidin-1-yl]-3-oxopropanenitrile,
3-[(3R,4R)-3-(3-bromodipyrrolo[2,3-b:2',3'-d]pyridin-1(6H)-yl)-4-methylpiperidin-1-yl]-3-oxopropanenitrile,
1-[(3S,4S)-4-methyl-1-(1H-tetrazol-1-ylacetyl)piperidin-3-yl]-1,6-dihydrodipyrrolo[2,3-b:2',3'-d]pyridine,
1-[(3R,4R)-4-methyl-1-(1H-tetrazol-1-ylacetyl)piperidin-3-yl]-1,6-dihydrodipyrrolo[2,3-b:2',3'-d]pyridine,
3-[(3S,4S)-3-(8-bromodipyrrolo[2,3-b:2',3'-d]pyridin-1(6H)-yl)-4-methylpiperidin-1-yl]-3-oxopropanenitrile,
3-[(3R,4R)-3-(8-bromodipyrrolo[2,3-b:2',3'-d]pyridin-1(6H)-yl)-4-methylpiperidin-1-yl]-3-oxopropanenitrile,
1-[(3S,4S)-1-(cyanoacetyl)-4-methylpiperidin-3-yl]-1,6-dihydrodipyrrolo[2,3-b:2',3'-d]pyridine-8-carbonitrile,
1-[(3R,4R)-1-(cyanoacetyl)-4-methylpiperidin-3-yl]-1,6-dihydrodipyrrolo[2,3-b:2',3'-d]pyridine-8-carbonitrile,
1-[(3S,4S)-1-(cyanoacetyl)-4-methylpiperidin-3-yl]-1,6-dihydrodipyrrolo[2,3-b:2',3'-d]pyridine-3-carbonitrile,
1-[(3R,4R)-1-(cyanoacetyl)-4-methylpiperidin-3-yl]-1,6-dihydrodipyrrolo[2,3-b:2',3'-d]pyridine-3-carbonitrile,
1- {(3S,4S)-4-methyl-1-[(5-methyl-1 H-tetrazol-1-yl)acetyl]piperidin-3-yl}-1,6-dihydrodipyrrolo[2,3-b:2',3'-d]pyridine, or
1-{(3R,4R)-4-methyl-1-[(5-methyl-1H-tetrazol-1-yl)acetyl]piperidin-3-yl}-1,6-dihydrodipyrrolo[2,3-b:2',3'-d]pyridine.

11. A pharmaceutical composition comprising the compound or a salt thereof as set forth in claim 1, and a pharmaceutically acceptable excipient.

12. A pharmaceutical composition for preventing or treating diseases caused by undesirable cytokine signal transduction or diseases caused by abnormal cytokine signal transduction, comprising the compound or a salt thereof as set forth in claim 1.

13. Use of the compound or a salt thereof as set forth in claim 1 for the manufacture of a pharmaceutical composition for preventing or treating diseases caused by undesirable cytokine signal transduction or diseases caused by abnormal cytokine signal transduction.

14. Use of the compound or a salt thereof as set forth in claim 1 for prevention or treatment of diseases caused by undesirable cytokine signal transduction or diseases caused by abnormal cytokine signal transduction.

15. A method for preventing or treating diseases caused by undesirable cytokine signal transduction or diseases caused by abnormal cytokine signal transduction, comprising administering to a patient an effective amount of the compound or a salt thereof as set forth in claim 1.

16. The compound or a salt thereof as set forth in claim 1 for prevention or treatment of diseases caused by undesirable cytokine signal transduction or diseases caused by abnormal cytokine signal transduction.
